# EUROPEAN PATENT APPLICATION

(11) **EP 2 524 967 A1**
(43) Date of publication of application: **21.11.2012**
(21) Application number: 11166816.6
(22) Date of filing: 19.05.2011
(51) Int. Cl.: C12Q 1/68

(54) **Methods and kits for diagnosing colorectal cancer**

(71) Applicant: Signature Diagnostics AG, 14473 Potsdam (DE)
(72) Inventor: Adams, Hans-Peter, 14478 Potsdam (DE); Hinzmann, Bernd, 13127 Berlin (DE); Mayr, Tobias, 10435 Berlin (DE); Rosenthal, André, 14194 Ludwigsfelde (DE)
(74) Representative: Hoppe, Georg Johannes

(57) **Abstract**

The invention pertains to a method for early detection and screening of colorectal cancer in human subjects based on RNA isolated from blood obtained from said subject. According to the invention, the abundance of at least 250 RNAs listed in tables 1 to 4 is measured. Using the invention, an accurate and noninvasive screening and diagnosis tool for colorectal cancer is provided with a sensitivity of at least 75 % and a specificity of 85 % that has high clinical utility and the potential for broad adoption.

## Description

The invention pertains to a method for diagnosing or detecting colorectal cancer in human subjects based on ribonucleic acid (RNA), in particular based on RNA from blood.

### Background

Colorectal cancer (CRC) is the second-leading cause of cancer-related deaths in the United States. Each year, approximately 150,000 people are diagnosed with CRC and almost 60,000 people die from the disease.

CRC arises from the mucosal forming the inner lining of colon and rectum. Like any other mucosa, it needs to be regenerated and proliferates at a high rate (about one third of all fecal matter are mucosa cells), and is thus susceptible to abnormal growth, i.e., neoplasia and/or dysplasia. In fact, abnormal colonic mucosal growth can be detected in about 40 % of all persons over the age of 55 years. The development of neoplasia into cancer is a will-established concept in the biomedical sciences; and is termed adenoma-carcinoma-sequence (ACS).

Pathologists classify abnormal mucosal growth into four categories with increasing severity: 1) Low-grade intraepithelial neoplasia (LIEN) or adenoma, which occurs in more than 30 %; 2) high-grade intraepithelial neoplasia (LIEN) or advanced adenoma, occurring in more than 2 %; 3) carcinoma in situ (CIS or pTis), where the cancerous growth is still confined to the mucosa; and 4) CRC, where the cancerous growth has invaded the submucosa. CRC is diagnosed with an incidence rate of about 1 % in persons over the age of 55 years with an average risk for the disease. The lifetime risk of developing CRC is estimated to be 1 in 18 persons (Cancer Statistics 2009: A Presentation From the American Cancer Society; 2009, American Cancer Society, Inc.).

After primary diagnosis of CRC, the spread/stage of the disease is classified according to the guidelines set forth by the "Union International Contre le Cancer" (UICC). UICC-stage 0 includes CIS only. UICC-stages I and II are comprised of the localized stages, whereas UICC-stage III describes CRC where tumor cells have metastasized into regional lymph nodes. The worst case is UICC-stage IV; it describes CRC which has metastasized into other organ(s), usually liver (∼75 %), peritoneum (∼22 %), and/or lung (∼21 %).

In 2008, the cancer registry in the state of Brandenburg/Germany documented 1591 patients with newly diagnosed CRC and stage information. They were staged into UICC-stage I: 22.6 %, UICC-stage II: 29.2 %, UICC-stage III: 28.9 %, and UICC-stage IV: 19.0 %. Relative five year survival-rates by UICC-stage were: I: 90.5%, II: 78.8%, III: 60.6%, and IV: 9.3%.

The U.S. National Institutes of Health (http://seer.cancer.gov) reported for the period 1999 to 2006 216,332 patients diagnosed with CRC with localized disease (UICC-stage I and II): 39 %, regional disease (UICC stage III): 37 % and distant disease (UICC-stage IV): 19 %. Relative five year survival-rates by stage were: localized (UICC-stages I and II): 90.4 %, regional (UICC stage III): 60.5 %, and distant (UICC-stage IV): 11.6 %. However, the statistics of the U.S. National Institutes of Health do not cover the U.S. population, while the data from the cancer registry in the state of Brandenburg/Germany do.

Current technologies to detect mucosal neoplasia (polyps/adenoma) and CRC can be categorized into three classes:
I) ***in-vitro* diagnostics** (IVDs,)-a specimen/sample (blood, stool, or urine) is taken from the test person and analyzed for one or more biomarkers as surrogate markers for colorectal neoplasia/cancer. Exemplary tests include guaic fecal occult blood test (gFOBT) or immunological fecal occult blood test (iFOBT), detection of tumor DNA-chains (deoxyribonucleic acid chains) in stool samples, detection of specific methylated tumor DNA-chains in stool samples, detection of specific free methylated DNA-chains in blood plasma, detection of elevated and/or flowered amounts of specific proteins in blood samples, or detection of elevated and/or lowered amounts of specific RNA-chains in blood samples;
II) **imaging methods without interventional capabilities** such as X-ray, double contrast barium enema (DCBE), video capsule endoscopy, or computed tomographic colonography;
III) **imaging methods with interventional capabilities** such as flexible sigmoidoscopy, colonoscopy, laparoscopy, or open surgery.

To obtain a definitive diagnosis of colorectal neoplasia/cancer, an invasive procedure is typically required. The procedure requires taking a sample of the visibly abnormal tissue growth (neoplasia/cancer) and having a person of skill in the art of pathology examine this sample, who will then decide (diagnose) whether this sample was taken from a neoplasia/cancer or not (Sternberg's Diagnostic Surgical Pathology (5th Edition). Mills SE, Carter D, Greenson JK, Reuter V, Stoler MH. Lippincott Williams & Wilkins (LWW), 2009).

In response to the high incidence and mortality rate of patients with CRC, the American Cancer Society issued the following statement: "There are significant updates to the guidelines for colorectal cancer screening. Two new tests are now recommended as options for colorectal cancer screening. They are stool DNA (sDNA) and computerized tomographic colonography (also known as "virtual colonoscopy"). For the first time, screening tests are grouped into categories based on performance characteristics: those that primarily detect cancer early and those that can also detect precancerous polyps. Tests that primarily detect cancer early are fecal (stool) tests, including guaiac-based and immunochemical-based fecal occult blood tests (gFOBT & FIT), and stool DNA tests (sDNA). Tests that detect both precancerous polyps and cancer include flexible sigmoidoscopy, colonoscopy, the double contrast barium enema, and computerized tomographic colonography (also known as virtual colonoscopy). It is the strong opinion of the expert panel that colon cancer prevention should be the primary goal of colorectal cancer screening. Exams that are designed to detect both early cancer and precancerous polyps should be encouraged if resources are available and patients are willing to undergo an invasive test." (Cancer Statistics 2009: A Presentation from the American Cancer Society; ©2009, American Cancer Society, Inc.). A review of current CRC screening in Europe can be found in: Zavoral M, Suchanek S, Zavada F, Dusek L, Muzik J. Seifert B, Fric P. Colorectal cancer screening in Europe. World J Gastroenterol. 2009 Dec 21; 15: 5907-15.

However, each of the tests for the detection of mucosal neoplasias (polyps/adenomas) and CRC has limitations.

For example, the imaging methods (with or without interventional capabilities) require preparation time for the test subject, specialized equipment, and specialized medical personnel. Therefore, colonoscopy and flexible sigmoidoscopy are used only in wealthy economies such as the U.S., Switzerland, and Germany as primary screening tools for early detection of CRC. Even in the U.K., France, Italy or Spain, IVDs, in most instances gFOBT, are used as a primary screening tool for colorectal cancer. Only patients with a positive IVD test result are referred to colonoscopy.

Recently, a screening program for CRC using gFOBT was initiated in the United Kingdom. All eligible persons were contacted via mail, and a test kit was delivered. Yet, just about 50 % of all contacted people complied. The willingness of patients to undergo gFOBT testing in Germany has dropped from 8.2 million tests in 2001 to 4.6 million tests in 2007 (Projekt wissenschaftliche Begleitung von Früherkennungs-Koloskopien in Deutschland, Berichtszeitraum 2008 - 6. Jahresbericht, im Auftrag des GKV-Spitzenverbands und der Kassenärztlichen Bundesvereinigung Version 1.1, Stand: 19. Februar 2010, Zentralinstitut für die kassenärztliche Versorgung in der Bundesrepublik Deutschland). In the U.S., about 24.01 % of all eligible patients underwent gFOBT-screening in 2000, in 2005 the rate dropped to 17.07 % (U.S. National Cancer Institute, http://progressreport.cancer.gov).

Thus, the clinical utility of all stool-based CRC-screening is limited because individuals in the CRC screening population are simply unwilling to take the test repeatedly, unless they have no other choice.

The U.S. National Institutes of Health reported that compliance with endoscopy (flexible sigmoidoscopy or colonoscopy) is dependent on the education and income of the population; by 2005 37.66 % of persons with less than high school education, 46.27 % of persons with high school education, and 57.52. % of persons with higher than high school education had ever had an endoscopy (not defined to CRC screening purposes).

Colonoscopy is an invasive procedure, which is not only inconvenient but may be associated with health risks. Approximately 3 % of the individuals over 55 years undergoing colonoscopy for screening purposes have heavy bleeding incidences. Additionally, in 2 of 1,000 individuals perforation of the colon occurs. Emergency operations must be performed to correct both heavy bleeding and perforation. As a result, 2 of 10,000 individuals who undergo colonoscopy will die from these complications. The relatively high rate of accidents in combination with the time consuming bowel cleaning procedure has led to a low adoption of colonoscopy as a screening tool even in those countries where colonoscopy is paid by the health insurances.

Thus, the overall clinical utility of all endoscopy-based CRC-screening is also limited, because it is only offered in a few countries, a high percentage of the CRC screening population is unwilling to take the test, and because of the complications associated with the test.

Therefore, the clinical utility of a test for detection of colorectal neoplasia depends not only on its performance characteristics, *i.e*., sensitivity and specificity, but on acceptance by the patients, the medical community, and, of course, the private or public health care system that has to pay for the test.

A blood test would have the highest chance of acceptance by patients, at least in Europe, the U.S., and Japan, In terms of the medical community, a blood test would also have the highest chance of acceptance, in particular if sensitivity and specificity are convincingly high, if there is no need for preparation time, if the blood need not be processed immediately but can be shipped to a central laboratory, if the test is accepted by local regulatory authorities, and if the test is commercially available. A high level of acceptance of a test can only be achieved if the test is endorsed by CRC screening guidelines and by the general health care system.

Although blood-based colon cancer screening has been attempted, each previously reported test is inherently limited in its respective specificity and sensitivity.

For example, Han et al., (Clin Cancer Res 2008; 14, 455-460; also: WO 2007/048074 A1) reported the discovery and validation of a five-gene expression (messenger RNA) signature for detection of colorectal carcinoma. Basically, the 37 candidate genes for the signature were selected from microarray data of 16 CRC cases and 15 controls. These 37 candidate genes were evaluated on a second set of 115 samples (58 CRC, 57 controls) using quantitative real-time PCR, validating 17 genes as differentially expressed. A further gene selection step using the PCR-results revealed the 5 gene signature, which was validated on a third set of 102 samples. The predictive power of these five genes, which was evaluated using a fourth set of 92 samples, correctly identifying 88 % (30 of 34) of CRC samples and only 64 % (27 of 42) of non-CRC samples. The intermediate zone contained 16 samples. The performance parameters are compiled into table A.

**Table A: Estimates and Exact Confidence Limits of GeneNews ColonSentry™ Test**

| Performance Parameter | N_{C} | N | Estimate | Exact Two-Sided 95 % CI-Limits | |
|---|---|---|---|---|---|
| | | | | Lower | Upper |
| Sensitivity | 30 | 34 | 0.88 | 0.725 | 0.967 |
| Specificity | 27 | 42 | 0.64 | 0.480 | 0.784 |
| Positive Predictive Value | 30 | 45 | 0.67 | 0.510 | 0.800 |
| Negative Predictive Value | 27 | 31 | 0.87 | 0.702 | 0.964 |
| Correct Classification Rate | 57 | 76 | 0.75 | 0.637 | 0.842 |

| | | | | | |
|---|---|---|---|---|---|
| N_{C} = Number of correctly classified cases; CI = Confidence interval; Exact confidence limits were computed using the proc FREQ of the statistics program SAS. | | | | | |

Provided that patients of the last validation set were a random selection of the screening population, applying the performance on a hypothetical set of 10,000 patients with an incidence of one percent and computing the performance parameters of this test yields the results shown in table B.

**Table B: Estimates and Exact Confidence Limits of GeneNews ColonSentry™ Test applied to a Hypothetical Set of 10000 Persons**

| Performance Parameter | N_{C} | N | Estimate | Exact Two-Sided 95 % CI-Limits | |
|---|---|---|---|---|---|
| | | | | Lower | Upper |
| Sensitivity | 73 | 100 | 0.73 | 0.632 | 0.814 |
| Specificity | 6979 | 9900 | 0.70 | 0.696 | 0.714 |
| Positive Predictive Value | 73 | 2994 | 0.02 | 0.019 | 0.031 |
| Negative Predictive Value | 6979 | 7006 | 1.00 | 0.994 | 0.997 |
| Correct Classification Rate | 7052 | 10000 | 0.71 | 0.696 | 0.714 |

| | | | | | |
|---|---|---|---|---|---|
| Nc = Number of correctly classified cases; CI = Confidence interval; Exact confidence limits were computed using the proc FREQ of the statistics program SAS. | | | | | |

However, based on the data provided by Han, 1,739 of 10,000 patients would have an "intermediate result". In clinical practice, this would mean that these 1,739 patients would have to undergo colonoscopy to clarify their state. However, in this computation, these 1,739 patients were regarded as having been predicted as low risk. The main disadvantage of the ColonSentry test is its relatively low sensitivity of 73 % and its low specificity of 70 %. Applied to a screening population of 1 million individuals this means that 2,700 individuals with undetected CRC will not be detected by the test. In addition, 300,000 individuals (30 %) are diagnosed as false positive, which need to be followed up by colonoscopy. The combination of a relatively high false negative rate of 27 % with a high false positive rate of 30 % reduces the clinical utility of this test and impedes acceptance by the medical community and the screening population itself.

Epigenomics AG, Germany, has a CE-marked test, Epi proColon^{®}, in the market that measures the methylation status of the Septin-9 gene and is based on detection of free somatic tumor DNA in blood serum.

**Table C: Estimates and Exact Confidence Limits of Epi proColon^{®} Test**

| Performance Parameter | N_{C} | N | Estimate | Exact Two-Sided 95% CI-Limits | |
|---|---|---|---|---|---|
| | | | | Lower | Upper |
| Sensitivity | 69 | 103 | 0.67 | 0.570 | 0.759 |
| Specificity | 135 | 154 | 0.88 | 0.814 | 0.924 |
| Positive Predictive Value | 69 | 88 | 0.78 | 0.684 | 0.865 |
| Negative Predictive Value | 135 | 169 | 0.80 | 0.730 | 0.856 |
| Correct Classification Rate | 204 | 257 | 0.79 | 0.739 | 0.842 |

| | | | | | |
|---|---|---|---|---|---|
| N_{C} = Number of correctly classified cases; CI = Confidence interval; Exact confidence limits were computed using the proc FREQ of the statistics program SAS. | | | | | |

The product performance figures of Epi proColon^{®} displayed in table C are cited from the companies' website (www.epigenomics.com). Table D shows the figures when the performance of the test is applied to a hypothetical screening population. Though the Epi proColon^{®} test performs better than GeneNews' test in some performance parameters its overall sensitivity for all four stages of CRC is only 67 %. This means that if 10,000 individuals are screened and the prevalence of CRC in the screening population is approximately 1 %, so that 33 individuals with CRC will be missed by the test. Applied to a screening population of 1 million individuals (3.7 % of the German screening population or 1.3% of the US screening population) 3,300 individuals with CRC will not be detected by the test. This high false negative rate limits significantly the clinical utility of the Epi proColon^{®} test. The false negative rate of patients with early stage CRC (UICC I and II) that will be missed is even higher.

**Table D: Estimates and Exact Confidence Limits of Epi proColon^{®} Test applied to a Hypothetical Set of 10,000 Persons**

| Performance Parameter | N_{C} | N | Estimate | Exact Two-Sided 95% CI-Limits | |
|---|---|---|---|---|---|
| | | | | Lower | Upper |
| Sensitivity | 67 | 100 | 0.67 | 0.569 | 0.761 |
| Specificity | 8679 | 9900 | 0.88 | 0.870 | 0.883 |
| Positive Predictive Value | 67 | 1288 | 0.05 | 0.041 | 0.066 |
| Negative Predictive Value | 8679 | 8712 | 1.00 | 0.995 | 0.997 |
| Correct Classification Rate | 8746 | 10000 | 0.87 | 0.868 | 0.881 |

| | | | | | |
|---|---|---|---|---|---|
| N_{C} = Number of correctly classified cases; CI = Confidence interval; Exact confidence limits were computed using the proc FREQ of the statistics program SAS. | | | | | |

Another blood-based test developed by OncoMethylome Science (Liege, Belgium) measures the methylation status of two Genes FOXE1 and SONE1. The sensitivity of this two-marker test for all four stages of CRC is 56 %, while the specificity is 91 % (ESMO meeting, Berlin, Germany, September 2009)

All three blood tests have a significant false negative rate and do not detect a significant number of patients with CRC. The ColonSentry test has a low specificity of 70 % and burdens colonoscopy facilities with a high number of false-positive test results.

Thus, there is a clear clinical need for an improved blood-based test for screening, detecting, or diagnosing colorectal cancer with high sensitivity and high specificity, which is minimally invasive so as to permit more widespread testing of the population to indicate the presence of colorectal cancer with high accuracy and therefore with a high clinical utility, and to ensure greater adherence to recommended protocols. Further, the identification of biomarkers, such as RNAs for use in such a minimally-invasive test would fulfill a longstanding need in the art.

### Brief description of the invention

The present invention provides methods and kits for diagnosing, detecting, and screening for colorectal cancer. Particularly, the invention provides for preparing RNA expression profiles of patient blood samples, the RNA expression profiles being indicative of the presence or absence of colorectal cancer. The invention further provides for evaluating the patient RNA expression profiles for the presence or absence of one or more RNA expression signatures that are indicative of colorectal cancer.

The inventors have surprisingly found that a sensitivity of at least 79 %, and a specificity of at least 89 %, is reached if and only if at least 250 RNAs are measured that are chosen from the RNAs listed in table 1. In other words, measuring 250 RNAs is necessary and sufficient for the detection of colon cancer in a human subject based on RNA from a blood sample obtained from said subject by measuring the abundance of at least 250 RNAs in the sample, that are chosen from the RNAs listed in table 1, and concluding based on the measured abundance whether the subject has colon cancer or not.

In one aspect, the invention provides a method for preparing RNA expression profiles that are indicative of the presence or absence of colorectal cancer. The RNA expression profiles are prepared from patient blood samples. The number of transcripts in the RNA expression profile may be selected so as to offer a convenient and cost effective means for screening samples for the presence or absence of colorectal cancer with high sensitivity and high specificity. Generally, the RNA expression profile includes the expression level or "abundance" of from 250 to about 1500 transcripts. In certain embodiments, the expression profile includes the RNA levels of 1000 transcripts or less, 800 transcripts or less, 500 transcripts or less.

In such embodiments, the profile may contain the expression level of at least 250 RNAs that are indicative of the presence or absence of colorectal cancer, and specifically, as selected from table 1, or may contain the expression level of at least 250, at least 500, at least 800, or of 1000 RNAs selected from table 1. Where larger profiles are desired, the profile may contain the expression level or abundance of at least about 1200 RNAs that are indicative of the presence or absence of colorectal cancer, and such RNAs may be selected from table 1. The identities and/or combinations of genes and/or transcripts that make up or are included in expression profiles are disclosed in table 1. In particular embodiments, the genes or transcripts include those listed in tables 2 to 4.

Such RNA expression profiles in accordance with this aspect may be evaluated for the presence or absence of an RNA expression signature indicative of colorectal cancer. Generally, the sequential addition of transcripts from table 1 to the expression profile provides for higher sensitivity and/or specificity for the detection of colorectal cancer. For example, the sensitivity of the methods provided herein may be at least 75 %, or at least 80 %, or at least 85 %, or at least 90 %. The specificity of the method may be at least 85 %, or at least 90 %, or at least 95%.

In a second aspect, the invention provides a method for detecting, diagnosing, or screening for colorectal cancer. In this aspect the method comprises preparing an RNA expression profile by measuring the abundance of at least 250, at least 500, or at least 800 RNAs in a patient blood sample, where the abundance of such RNAs are indicative of the presence or absence of colorectal cancer. The RNAs may be selected from the RNAs listed in table 1, and exemplary sets of such RNAs are disclosed in tables 2 to 4. The method further comprises evaluating the profile for the presence or absence of an RNA expression signature indicative of colorectal cancer, to thereby conclude whether the patient has or does not have colorectal cancer. The method generally provides a sensitivity for the detection of colorectal cancer of at least about 75 %, while providing a specificity of at least about 85 %.

In various embodiments, the method comprises determining the abundance of at least 250 RNAs, at least 500 RNAs, at least 800 RNAs, or of at least 1000 RNAs chosen from the RNAs listed in table 1, and as exemplified in tables 2 to 4, and classifying the sample as being indicative of colorectal cancer, or not being indicative of colorectal cancer.

In other aspects, the invention provides kits and custom arrays for preparing the gene expression profiles, and for determining the presence or absence of colorectal cancer.

### Detailed description of the invention

The invention provides methods and kits for screening, diagnosing, and detecting colorectal cancer in human patients (subjects). "Colorectal cancer" (CRC) refers to both colorectal adenoma and colorectal carcinoma.

A colorectal adenoma is characterized by atypical growth of epithelial cells in mucosal tissue, i.e. neoplasia. Hypercellularity with enlarged, hyperchromatic nuclei, varying degrees of nuclear stratification, nuclear polymorphisms, and loss of polarity are the histologically defining features. In colorectal adenoma, this abnormal growth is confined to the mucosa; a synonym of adenoma is intraepithelial neoplasia (IEN). If this atypical growth of epithelial cells extends/invades through the muscularis mucosae, the muscle layer under the mucosa, with destruction of the usual anatomical wall, the pathologist terms this atypical growth a colorectal carcinoma.

The distinction between high- (HIEN) and low-grade (LIEN) intraepithelial neoplasia refers to the extent of the defining features.

A patient with CRC is traditionally defined as having undergone surgery/resection of colon and/or rectum for CRC and whose resection specimen has undergone examination by a board certified pathologist, who has diagnosed a colorectal carcinoma as defined above. A patient with CRC may have undergone complete colonoscopy of colon and rectum during which the examinating physician has taken a sample of suspect tissue, which in turn has undergone examination by a board-certified pathologist, who has diagnosed a colorectal carcinoma as defined above. A synonym for a patient with CRC is "CRC-case" or simply "case."

A patient without CRC is traditionally a person that has undergone complete colonoscopy during which the examining physician, an endoscopist, has noted no abnormal tissue growth. A synonym for a patient without CRC is "non-CRC-case" or "control." This however does not exclude that this person has any other carcinoma.

A patient with HIEN is traditionally a person that has undergone surgery/resection of colon and/or rectum for suspected CRC and whose resection specimen has undergone examination by a board certified pathologist, who has diagnosed a high-grade intraepithelial neoplasia as defined above. Alternatively, a patient with HIEN may be a person that has undergone complete colonoscopy of colon and rectum during which the examinating physician has taken a sample of suspect tissue, which in turn has undergone examination by a board certified pathologist, who has diagnosed a high-grade intraepithelial neoplasia as defined above. A synonym for a person with HIEN is "HIEN-case" or "HIEN."

A patient with LIEN is traditionally a person that has undergone surgery/resection of colon and/or rectum for suspected CRC and whose resection specimen has undergone examination by a board certified pathologist, who has diagnosed a high-grade intraepithelial neoplasia. Alternatively, a patient with LIEN is a person that has undergone complete colonoscopy of colon and rectum during which the examinating physician has taken a sample of suspect tissue, which in turn has undergone examination by a board certified pathologist, who has diagnosed a low-grade intraepithelial neoplasia as defined above. A synonym for a person with LIEN "is LIEN-case" or "LIEN."

As disclosed herein, the present invention provides methods and kits for screening patient samples for those that are positive for CRC, that is, in the absence of colonoscopy and/or surgery or resection of colon or rectum with pathologist's examination.

The invention relates to the determination of the abundance of RNAs to detect a colorectal cancer in a human subject, wherein the determination of the abundance is based on RNA obtained (or isolated) from whole blood of the subject or from blood cells of the subject. For example, the sample may be obtained using PAXgene (QIAGEN) or an equivalent RNA isolation system. The measurement of the abundance of RNAs in the sample is preferably performed together, i.e. sequentially or preferably simultaneously. The blood sample preferably does not contain cancer cells. Preferably, the sample comprises or consists of white blood cells.

In various aspects, the invention involves preparing an RNA expression profile from a patient sample. The method may comprise isolating RNA from whole blood, and detecting the abundance or relative abundance of selected transcripts. As used herein, the terms RNA "abundance" and RNA "expression" are used interchangeably. The "RNAs" may be defined by reference to an expressed gene, or by reference to a transcript, or by reference to a particular oligonucleotide probe for detecting the RNA (or cDNA derived therefrom), each of which is listed in table 1 for 1000 RNAs that are indicative of the presence or absence of colorectal cancer. Specifically, table 1 lists such RNAs by probe ID, gene symbol, and nucleotide sequences, which are publicly accessible.

The number of transcripts in the RNA expression profile may be selected so as to offer a convenient and cost effective means for screening samples for the presence or absence of colorectal cancer with high sensitivity and high specificity. For example, the RNA expression profile may include the expression level or "abundance" of from 250 to about 1200 transcripts. In certain embodiments, the expression profile includes the RNA levels of 1000 transcripts or less, 800 transcripts or less, or 500 transcripts or less. Such profiles may be prepared, for example, using custom microarrays or multiplex gene expression assays as described in detail herein.

In such embodiments, the profile may contain the expression level of at least 250 RNAs that are indicative of the presence or absence of colorectal cancer, and specifically, as selected from table 1, or may contain the expression level of at least 500, 800, or 1000 RNAs selected from table 1. Where larger profiles are desired, the profile may contain the expression level or abundance of at least 1200 RNAs that are indicative of the presence or absence of colorectal cancer, and such RNAs may be selected from table 1. Such RNAs may be defined by gene, or by transcript ID, or by probe ID, as set forth in table 1.

The identities of genes and/or transcripts that make up, or are included in exemplary expression profiles are disclosed in tables 1 to 4. As shown herein, profiles selected from the RNAs of table 1 support the detection of colorectal cancer with high sensitivity and high specificity.

The present invention provides an in-vitro diagnostic test system (IVD) that is trained (as described further below) for the detection of a colorectal cancer. For example, in order to determine whether a patient has colorectal cancer, reference RNA abundance values for colon cancer positive and negative samples are determined. The RNAs can be quantitatively measured on an adequate set of training samples comprising cases and controls, and with adequate clinical information on carcinoma status, applying adequate quality control measures, and on an adequate set of test samples, for which the detection is yet to be made. With such quantitative values for the RNAs and the clinical data for the training samples, a classifier can be trained and applied to the test samples to calculate the probability of the presence or non-presence of the colorectal carcinoma.

Various classification schemes are known for classifying samples between two or more classes or groups, and these include, without limitation: Naive Bayes, Support Vector Machines, Nearest Neighbors, Decision Trees, Logistics, Articifial Neural Networks, and Rule-based schemes. In addition, the predictions from multiple models can be combined to generate an overall prediction. Thus, a classification algorithm or "class predictor" may be constructed to classify samples. The process for preparing a suitable class predictor is reviewed in R. Simon, Diagnostic and prognostic prediction using gene expression profiles in high-dimensional microarray data, British Journal of Cancer (2003) 89, 1599-1604, which review is hereby incorporated by reference.

In this context, the invention teaches an in-vitro diagnostic test system (IVD) that is trained in the detection of a colorectal cancer referred to above, comprising at least 250 RNAs, which can be quantitatively measured on an adequate set of training samples comprising cases and controls, with adequate clinical information on carcinoma status, applying adequate quality control measures, and on an adequate set of test samples, for which the detection yet has to be made. Given the quantitative values for the RNAs and the clinical data for the training samples, a classifier can be trained and applied to the test samples to calculate the probability of the presence or absence of the colorectal carcinoma.

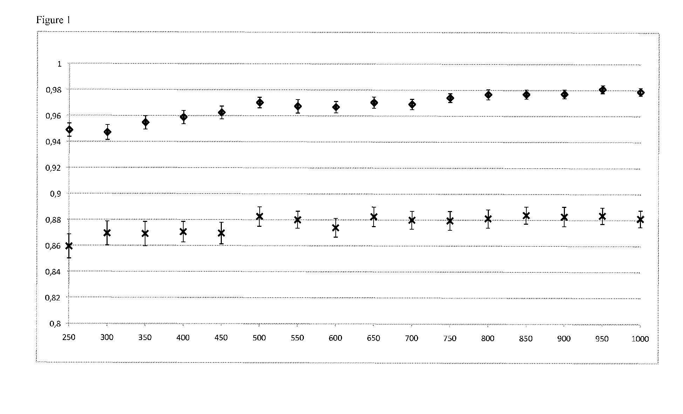

The present invention provides methods for detecting, diagnosing, or screening for colorectal cancer in a human subject with a sensitivity and specificity not previously described for a blood-based method (see Fig. 1). Specifically, the sensitivity of the methods provided herein is at least 75 %, at least 80 %, at least 85 %, or at least 90 %. The specificity of the methods is at least 85 %, at least 90 %, or at least 95%, for example, when determined with samples of at least 52 patients with CRC and adequate samples (e.g., at least 64) of normal individuals without CRC are tested.

Without wishing to be bound by any particular theory, the above finding may be due to the fact that an organism such as a human systemically reacts to the development of a colorectal tumor by altering the expression levels of genes in different pathways. The formation of cancerous tumor cells from a nonmalignant adenoma or nonmalignant polyps, the formation of high-grade intraepithelial neoplasias and the further growth and development of cancer of different stages may trigger differential expression of genes in white blood cells that are involved in both adaptive and innate immune responses, for example wound healing, inflammatory response and antibody production pathways. Although the change in expression (abundance) might be small for each gene in a particular signature, measuring a set of at least 250 genes, preferably even larger numbers such as 500, 800 or even more RNAs, for example at least 1000 RNAs at the same time allows for the detection of colorectal cancer in a human with high sensitivity and high specificity.

In this context, an RNA obtained from a subject's blood sample, i.e. an RNA biomarker, is an RNA molecule with a particular base sequence whose presence within a blood sample from a human subject can be quantitatively measured. The measurement can be based on a part of the RNA molecule, namely a part of the RNA molecule that has a certain base sequence, which allows for its detection and thereby allows for the measurement of its abundance in a sample. The measurement can be by methods known in the art, for example analysis on a solid phase device, or in solution (for example, by RT-PCR). Probes for the particular RNAs can either be bought commercially, or designed based on the respective RNA sequence.

In the method of the invention, the abundance of several RNA molecules (e.g. mRNA or prespliced RNA, intron-lariat RNA, micro RNA, small nuclear RNA, or fragments thereof) is determined in a relative or an absolute manner, wherein an absolute measurement of RNA abundance is preferred. The RNA abundance is, if applicable, compared with that of other individuals, or with multivariate quantitative thresholds.

The determination of the abundance of the RNAs described herein is performed from blood samples using quantitative methods. In particular, RNA is isolated from a blood sample obtained from a human subject that is to undergo CRC testing. Although the examples described herein use microarray-based methods, the invention is not limited thereto. For example, RNA abundance can be measured by in situ hybridization, amplification assays such as the polymerase chain reaction (PCR), sequencing, or microarray-based methods. Other methods that can be used include polymerase-based assays, such as RT-PCR (e.g., TAQMAN), hybridization-based assays, such as DNA microarray analysis, as well as direct mRNA capture with branched DNA (QUANTIGENE) or HYBRID CAPTURE (DIGENE).

In certain embodiments, the invention employs a microarray. A "micoroarray" includes a specific set of probes, such as oligonucleotides and/or cDNAs (*e.g*., expressed sequence tags, "ESTs") corresponding in whole or in part, and/or continuously or discontinuously, to regions of RNAs that can be extracted from a blood sample of a human subject. The probes are bound to a solid support. The support may be selected from beads (magnetic, paramagnetic, etc.), glass slides, and silicon waters. The probes can correspond in sequence to the RNAs of the invention such that hybridization between the RNA from the subject sample (or cDNA derived therefrom) and the probe occurs. In the microarray embodiments, the sample RNA can optionally be amplified before hybridization to the microarray. Prior to hybridization, the sample RNA is fluorescently labeled. Upon hybridization to the array and excitation at the appropriate wavelength, fluorescence emission is quantified. Fluorescence emission for each particular nucleic acid is directly correlate with the amount of the particular RNA in the sample. The signal can be detected and together with its location on the support can be used to determine which probe hybridized with RNA from the subject's blood sample.

Accordingly, in certain aspects, the invention is directed to a kit or microarray for detecting the level of expression or abundance of RNAs in the subject's blood sample, where this "profile" allows for the conclusion of whether the subject has colorectal cancer or not (at a level of accuracy described herein). In another aspect, the invention relates to a probe set that allows for the detection of the RNAs associated with CRC. If these particular RNAs are present in a sample, they (or corresponding cDNA) will hybridize with their respective probe (i.e, a complementary nucleic acid sequence), which will yield a detectable signal. Probes are designed to minimize cross reactivity and false positives. In one embodiment, the probes used are given e.g. in table 1 as so-called NimbleGen probe set ID numbers. A NimbleGen probe set ID number is an identifier that refers to a set of probes selected to represent expressed sequences on an array. A NimbleGen probe set ID number identifies each probe present on the array, as known to a person of skill in the art. From the sequence defined by an NimbleGen probe set ID number, the sequence of a nucleic acid, such as an RNA or a molecule derived therefrom (such as cDNA) hybridizing with the probe can be deduced.

Thus, the invention in certain aspects provides a microarray, which generally comprises a solid support and a set of oligonucleotide probes. The set of probes generally contains from 250 to about 3,000 probes, including at least 250 probes selected from table 1. In certain embodiments, the set contains 1200 probes or less, or 800 probes or less. In various embodiments, at least 250 probes are listed in table 1. The set of probes includes probes that hybridize to a combination of RNAs exemplified in any one of table 2, table 3, or table 4. The microarray may comprise, e.g., about 100.000 probes, some of which, usually about 10.000 to 100.000 probes, may be probes for providing reference data.

The conclusion whether the subject has colorectal cancer or not is preferably reached on the basis of a classification algorithm, which can be developed using e.g. a random forest method, a support vector machine (SVM), or a K-nearest neighbor method (K-NN), such as a 3-nearest neighbor method (3-NN), as known in the art.

From the cross-classification of the true disease state (Positive = patient with CRC and Negative = patient without CRC) as determined by a physician and the test result as determined by the classification algorithm, the following measures for binary tests can be derived (Sullivan MS. The Statistical Evaluation of Medical Tests for Classification and Prediction. Oxford University Press, 2003), see table E. An example is given in table F.

**Table E: Cross-Classification of True Disease State by Test Result**

| Test Result | True Disease State | | Total |
|---|---|---|---|
| | Negative | Positive | |
| Negative | n₁₁ | n₁₂ | n_{1∑} |
| Positive | n₂₁ | n₂₂ | n_{2∑} |
| Total | n_{∑1} | n_{∑2} | n_{ΣΣ} |

**Table F: Example of a Cross-Classification of True Disease State by Test Result**

| Test Result | True Disease State | | Total |
|---|---|---|---|
| | Negative | Positive | |
| Negative | 30 | 10 | 40 |
| Positive | 20 | 70 | 90 |
| Total | 50 | 80 | 130 |

"Sensitivity" (S⁺ or true positive fraction (TPF)) refers to the count of positive test results among all true positive disease states divided by the count of all true positive disease states; in terms of table E this reads: S⁺ = n₂₂ / n_{∑2}; the result form table F would read: S⁺ = 70 / 80 = 0.875. "Specificity" (S- or true negative fraction (TNF)) refers to the count of negative test results among all true negative disease states divided by the count of all true negative disease states; in terms of table E this reads: S⁺ = n₁₁ / n_{∑1}; the result form table F would read: S⁻ = 30 / 50 = 0.6. "Correct Classification Rate" (CCR or true fraction (TF)) refers to the sum of the count of positive test results among all true positive disease states and count of negative test results among all true negative disease states divided by all the sum of all cases; in terms of table E this reads: CCR = (n₁₁ + n₂₂) / n_{∑∑}; the result form table F would read: CCR = (30 + 70) / 130 ≈ 0.769230769. The measures S⁺, S⁻, and CCR address the question: To what degree does the test reflect the true disease state?

"Positive Predictive Value" (PV⁺ or PPV) refers to the count of true positive disease states among all positive test results dived by the count of all positive test results; in terms of table E this reads: PV⁺ = n₂₂ / n_{2∑}; the result form table F would read: PV⁺ = 70 / 90 ≈ 0.777777778. "Negative Predictive Value" (PV⁻ or NPV) refers to the count of true negative disease states among all negative test results dived by the count of all negative test results; in terms of table E this reads: PV⁻ = n₁₁ / n_{1∑∑}; the result form table F would read: PV⁻ = 30 / 40 = 0.75. The predictive values address the question: How likely is the disease given the test results?

Exact or asymptotic confidence limits (CI) for these rates or fractions can be computed using the commercially available software package SAS (SAS Institute Inc., Cary, NC, USA; www.sas.com) or the publicly available software package R (www.r-project.org) (for literature reference see: Agresti A, Caffo B. Simple and effective confidence intervals for proportions and differences of proportions from adding two successes and two failures. The American Statistician: 54: 280-288, 2000).

The preferred RNA molecules that can be used in combinations described herein for diagnosing and detecting colorectal cancer in a subject according to the invention can be found in table 1. The inventors have shown that the selection of at least 250 or more RNAs of the markers listed in table 1 can be used to diagnose or detect colorectal cancer in a subject using a blood sample from that subject. The RNA molecules that can be used for detecting, screening and diagnosing colorectal cancer are selected from the RNAs provided in table 2, 3, or 4. Also, the RNAs (e.g., at least 250, at least 500, at least 800, or more) can be selected from table 3.

Specifically, the method of the invention comprises at least the following steps: measuring the abundance of at least 250 RNAs (preferably 500 RNAs) in the sample, that are chosen from the RNAs listed in table 1, and concluding, based on the measured abundance, whether the subject has colorectal cancer or not. Measuring the abundance of RNAs may comprise isolating RNA from blood samples as described, and hybridizing the RNA or cDNA prepared therefrom to a microarray. Alternatively, other methods for determining RNA levels may be employed.

Examples for sets of 250 RNAs that are measured together, i.e. sequentially or preferably simultaneously, are shown in table 2. The sets of 250 RNAs of table 2 are defined by a common threshold of sensitivity of at least 84 % and specificity of at least 93 %.

In a preferred embodiment of the invention as mentioned herein, the abundance of at least 500 RNAs (preferably 800 RNAs) in the sample is measured, wherein the at least 500 RNAs are chosen from the RNAs listed in table 1. Examples for sets of 500 RNAs that can be measured together, i.e. sequentially or preferably simultaneously, to detect colorectal cancer in a human subject are shown in table 3. The sets of 500 RNAs of table 3 are defined by a common threshold of sensitivity of at least 86 % and specificity of at least 95 %.

Similarly, the abundance of at least 800 RNAs (preferably up to 1000 RNAs) that are chosen from the RNAs listed in table 1 can be measured in the method of the invention. The sets of 800 RNAs of table 4 are defined by a common threshold of sensitivity of at least 87 % and specificity of at least 97 %.

Tables 2 through 4 show examples of combinations from table 1, varying in size from 250 to 800 sequences. Examples 1,2, and 3 each consist of 250 sequences (table 2). Examples 4, 5, and 6 each consist of 500 sequences (table 3). Examples 7, 8, and 9 (table 4) each consist of 800 sequences.

The performance of the examples 1 to 9 was evaluated by applying them to the discovery set, split randomly into a learning and a test set, 1000 times. Each time, a random 85 % of the discovery set were used to train a classifier based on the expression profile of the example sequences. This classifier was then applied to the remaining 15 % as a test set. The results across all repetitions were averaged and are given in the following table:

| | Example 1 | Example 2 | Example 3 |
|---|---|---|---|
| Sensitivity | 87.0% | 86.9% | 84.6% |
| Specificity | 95.4% | 95.8% | 93.2% |

| | Example 4 | Example 5 | Example 6 |
|---|---|---|---|
| Sensitivity | 90.1% | 86.6% | 86.4% |
| Specificity | 96.0% | 95.5% | 95.3% |

| | Example 7 | Example 8 | Example 9 |
|---|---|---|---|
| Sensitivity | 88.2% | 88.2% | 87.7% |
| Specificity | 98.0% | 97.8% | 97.8% |

Examples 1, 2, 4, 5, 7, and 8 were chosen based on their performance in this retrospective evaluation. Examples 3, 6, and 9 were chosen during the discovery procedure as consensus over all outer loops. In that procedure, a prospective estimate for sequences of length 250 was observed of 79.3% sensitivity and 89.2% specificity; for length 500: 79.5% sensitivity and 90.6% specificity; for length 800: 79.1% sensitivity and 89.6% specificity.

When all 1000 sequences are evaluated in this retrospective fashion, a sensitivity of 88.2 % and a specificity of 97.7 % were seen. In the discovery procedure, prospective estimates of 78.8 % sensitivity and 90.2 % specificity were observed.

When the wording "at least a number of RNAs" is used, this refers to a minimum number of RNAs that are measured. It is possible to use up to 10,000 or 20,000 genes in the invention, a fraction of which can be RNAs listed in table 1. In preferred embodiments of the invention, abundance of up to 5,000, 2,500, 2,000, 1,000 RNAs of randomly chosen RNAs that are not listed in table 1 is measured in addition to RNAs of table 1 (or subsets thereof).

In a preferred embodiment, only RNAs that are mentioned in table 1 are measured.

The expression profile or abundance of RNA markers for colorectal cancer, for example the at least 250 RNAs described above, (or more RNAs as disclosed above and herein), is determined preferably by measuring the quantity of the transcribed RNA of the marker gene. This quantity of the mRNA of the marker gene can be determined for example through chip technology (microarray), (RT-) PCR (for example also on fixated material), Northern hybridization, dot-blotting, sequencing, or in situ hybridization.

The microarray technology, which is most preferred, allows for the simultaneous measurement of RNA abundance of up to many thousand RNAs and is therefore an important tool for determining differential expression (or differences in RNA abundance), in particular between two biological samples or groups of biological samples. In order to apply the microarray technology, the RNAs of the sample need to be amplified and labeled and the hybridization and detection procedure can be performed as known to a person of skill in the art.

As will be understood by those of ordinary skill in the art, the analysis can also be performed through single reverse transcriptase-PCR, competitive PCR, real time PCR, differential display RT-PCR, Northern blot analysis, sequencing, and other related methods. In general, the larger the number of markers is that are to be measured, the more preferred is the use of the microarray technology. However, multiplex PCR, for example, real time multiplex PCR is known in the art and is amenable for use with the present invention, in order to detect the presence of 2 or more genes or RNA simultaneously.

The RNA whose abundance is measured in the method of the invention can be mRNA, cDNA, unspliced RNA, or its fragments. Measurements can be performed using the complementary DNA (cDNA) or complementary RNA (cRNA), which is produced on the basis of the RNA to be analyzed, e.g. using microarrays. A great number of different arrays as well as their manufacture are known to a person of skill in the art and are described for example in the U.S. Patent Nos. 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,331; 5,554,501; 5,561,071; 5,571,639; 5,593,839; 5,599,695; 5,624,711; 5,658,734; and 5,700,637, each of which is hereby incorporated by reference in its entirety.

Preferably the decision whether the subject has colon cancer comprises the step of training a classification algorithm on an adequate training set of cases and controls and applying it to RNA abundance data that was experimentally determined based on the blood sample from the human subject to be diagnosed. The classification method can be a random forest method, a support vector machine (SVM), or a K-nearest neighbor method (K-NN), such as 3-NN.

For the development of a model that allows for the classification for a given set of biomarkers, such as RNAs, methods generally known to a person of skill in the art are sufficient, i.e., new algorithms need not be developed.

The major steps of such a model are:
1) condensation of the raw measurement data (for example combining probes of a microarray to probeset data, and/or normalizing measurement data against common controls);
2) training and applying a classifier (i.e. a mathematical model that generalizes properties of the different classes (carcinoma vs. healthy individual) from the training data and applies them to the test data resulting in a classification for each test sample.

For example, the raw data from microarray hybridizations can be.quantile-normalized after forming the median of multiple identical probes and subtracting the local background (see Bolstad,Irizarry,Anstrand, Speed: A comparison of normalization methods for high density oligonucleotide array data based on variance and bias. Bioinformatics, 19 (2):185-193). Alternative methods for condensation can be used, such as: FARMS as shown by Hochreiter (2006, Bioinformatics 22(8): 943-9), RMA, GC-RMA, see Irizarry et al (2003). Exploration, Normalization, and Summaries of High Density Oligonucleotide Array Probe Level Data. Biostatistics. 4, 249-264.). Similar to condensation, classification of the test data set through a support-vector-machine or other classification algorithms is known to a person of skill in the art, like for example classification and regression trees, penalized logistic regression, sparse linear discriminant analysis, Fisher linear discriminant analysis, K-nearest neighbors, shrunken centroids, and artificial neural networks (see Wladimir Wapnik: The Nature of Statistical Learning Theory, Springer Verlag, New York, NY, USA, 1995; Berhard Schölkopf, Alex Smola: Learning with Kernels: Support Vector Machines, Regularization, Optimization, and Beyond, MIT Press, Cambridge, MA, 2002; S. Kotsiantis, Supervised Machine Learning: A Review of Classification Techniques, Informatica Journal 31 (2007) 249-268).

The key component of these classifier training and classification techniques is the choice of RNA biomarkers that are used as input to the classification algorithm.

In a further aspect, the invention refers to the use of a method as described above and herein for the detection of colorectal cancer in a human subject, based on RNA from a blood sample.

In a further aspect, the invention also refers to the use of a microarray for the detection of colorectal cancer in a human subject based on RNA from a blood sample. According to the invention, such a use can comprise measuring the abundance of at least 250 RNAs (or more, as described above and herein) that are listed in table 1. Accordingly, the microarray comprises at least 250 probes for measuring the abundance of the at least at least 250 RNAs. It is preferred that the microarray has a set of 11 probes for each RNA, and 1 or 3 probes for each RNA are also preferred. Commercially available microarrays, such as from Roche NimbleGen, Inc., may be used. Alternatively, at most 250, at most 500, or at most 800 RNAs are measured in a sample, in order to detect or diagnose CRC.

In another embodiment, the abundance of the at least 250 RNAs is measured by multiplex RT-PCR. In a further embodiment, the RT-PCR includes real time detection, e.g., with fluorescent probes such as Molecular beacons or TaqMan^{®} probes.

In a preferred embodiment, the microarray comprises probes for measuring only RNAs that are listed in table 1 (or subsets thereof).

In yet a further aspect, the invention also refers to a kit for the detection of colorectal cancer in a human subject based on RNA obtained from a blood sample. Such a kit comprises a means for measuring the abundance of at least 250 RNAs that are chosen from the RNAs listed in table 1. In a further embodiment, the at least 250 RNAs are chosen from the RNAs listed in any of the tables provided herein, for example, the RNAs are chosen from tables 2 to 4. The means for measuring expression can be probes that allow for the detection of RNA in the sample or primers that allow for the amplification of RNA in the sample. Ways to devise probes and primers for such a kit are known to a person of skill in the art.

Further, the invention refers to the use of a kit as described above and herein for the detection of colorectal cancer in a human subject based on RNA from a blood sample comprising means for measuring the abundance of at least 250 RNAs that are chosen from the RNAs listed in table 1. Such a use may comprise the following steps: contacting at least one component of the kit with RNA from a blood sample from a human subject, measuring the abundance of at least 250 RNAs (or more as described above and herein) that are chosen from the RNAs listed in table 1 using the means for measuring the abundance of at least 250 RNAs, and concluding, based on the measured abundance, whether the subject has colorectal cancer.

In yet a further aspect, the invention also refers to a method for preparing an RNA expression profile that is indicative of the presence or absence of colorectal cancer, comprising: isolating RNA from a whole blood sample, and determining the level or abundance of from 250 to about 3000 RNAs, including at least 250 RNAs selected from table 1.

Preferably, the expression profile contains the level or abundance of 800 RNAs or less, of 500 RNAs or less. Further, it is preferred that at least 250 RNAs, at least 500 RNAs, at least 800 RNAs are listed in table 1 or in tables 2 to 4.

Another preferred embodiment of the method comprises determining the presence or absence of an RNA expression signature indicative of colorectal cancer.

In yet a further aspect, the invention also refers to a microarray, comprising a solid support and a set of oligonucleotide probes, the set containing from 250 to about 3,000 probes, and including at least 250 probes selected from table 1. Preferably, the set contains 2000 probes or less, 1000 probes or less. At least 250 probes can be those listed in table 1 or table 2. At least 500 probes can be those listed in table 1 or table 3. In another embodiment, at least 800 probes are listed in table 1 or table 4.

Features of the invention that were described herein in combination with a method, a microarray, a kit, or a use also refer, if applicable, to all other aspects of the invention.

### Tables and Figure

**sable 1** shows a list of 1000 RNAs that are differentially expressed in several human subjects with colorectal cancer in comparison to subject without colorectal cancer. Each marker is characterized by a SEQ ID NO., by a NimbleGen probe set ID, which is also the primary transcript. Seq ID
**Table 1A** (shows the changes of expression level (abundance) between cases and controls for all of 1000 RNAs listed in table 1. The data was derived from 116 samples, 52 CRC cases and 64 controls. In the second column, the log-2 fold change is shown, and in the third column, the non-log fold change is shown. The numbers in the second column represent the differences of cases (events) and control (non-envents) in log2 steps. For example, a value of 0.08 for the first probe set (SEQ ID NO. 1) means an expression increase by 2^0.08-fold for events vs. non-events. In other words, the events had a 106 % expression (see third column) with respect to non-events, i.e. 6 % more RNA (0.06 = 2^0.08-1, 1.08 = 2^0.08).
**Tables 2 to 4** show examples of combinations from table 1, varying in size from 250 to 800 sequences. Examples 1, 2, and 3 each consist of 250 sequences (table 2). Examples 4, 5, and 6 each consist of 500 sequences (table 3). Examples 7, 8, and 9 (table 4) each consist of 800 sequences.

**Figure 1** is a graph showing the performance of various RNA colorectal cancer signatures as a function of their length. The x-axis shows the number of RNAs in each particular signature subset. The subset length is varied along the x-axis from 250 to 1000 (the full set). The y-axis shows performance of the subset. Performance is shown in terms of sensitivity (percentage of real carcinomas that were properly classified; lower values (crosses)) and specificity (percentage of real healthy controls that were properly classified; upper values (diamonds)) scaled from 80 % to 100 %, with standard errors. These are retrospective examinations of the 1000 RNA full set. As the figure shows, reduced sets compared to the full set appear to have excellent performance. For each subset size, the performance was measured for 100 randomly chosen subsets based on leave-15 %-out runs: the subset is trained on 85 % and applied to the remaining 15 %. The average performance over all the 15 % tests is shown.

### Examples

### Materials and Methods

### Study Protocol

All results described herein are based on a prospective, clinical-diagnostic study protocol entitled Früherkennung kolorektaler Karzinome mit Hilfe von RNA-basierten Expression-Signaturen aus Blut - Eine multizentrische, diagnostische Studie zur Entwicklung und Validierung von Genexpressions-Signaturen zur Früherkennung kolorektaler Karzinome - Version CRC.SCR.2.BR.1 vom 06. Januar 2009" in accordance with the Guideline for Good Clinical Practice (Directive 75/318/EEC) July 1996, version July 2002 (http://www.emea.eu.int/pdfs/human/ich/013595en.pdf). This study protocol was reviewed and approved by the local ethics authority, the "Ethik-Kommission der Landesärztekammer Brandenburg" on January 14^{th} 2009. All persons entered into this study gave written informed consent that their blood samples and associated clinical data could be used for this research endeavor. Moreover, the persons gave written, informed consent that samples and clinical data could be audited by regulatory authorities if the research results would be used in a performance evaluation study (according to German law (Gesetz über Medizinprodukte)).

This study was designed as a cohort study. One cohort, the colonoscopy cohort, included persons undergoing colonoscopy. The second cohort, the surgery cohort, included patients scheduled for surgery for suspected colorectal carcinoma.

The inclusion criteria for the colonoscopy cohort were: 1) Willingness to undergo complete colonoscopy; 2) At least 55 years of age; 3) Ability to give written, informed consent; 4) Written informed consent after information about the study was given by a physician. The exclusion criteria for the colonoscopy cohort were: 1) Rectoscopy, sigmoidoscopy, or colonoscopy during the last five years prior to inclusion into the study; 2) Treatment of a malignant disease during the last five years prior to inclusion into the study, except for malignoma with low metastatic potential such as basalioma in situ of the skin.

The inclusion criteria for the surgery cohort were: 1) Age at initial diagnosis at least 18 years of age; 2) Ability to give written, informed consent; 3) (Suspected) Diagnosis of colorectal carcinoma UICC-stage I to IV; 4) Surgery is planned in such a fashion that staging according to UICC-criteria is feasible; 5) No treatment prior to surgery; 6) No treatment for a malignant disease during the last five years prior to inclusion into the study; 7) No other disease that lowers life expectancy below one year; 8) Regular follow-up examinations have to be possible; 9) Written informed consent after information about the study was given by a physician.

### Blood Drawing and Blood Sample Storage

In the colonoscopy cohort, blood was drawn after written, informed consent and prior to bowel cleaning and change of medication if necessary. In the surgery cohort, blood was drawn after written, informed consent and prior to removal of the resected tissue from the body of the patient by the surgeon. In both cohorts, colonoscopy cohort and surgery cohort, blood was drawn into PAXgene™ blood tubes (Becton Dickinson). The tubes were stored within four hours in a freezer at -20 °C until transport to the laboratory on dry ice, where the tubes were stored again in a freezer at -20 °C until RNA-extraction.

### Sample Sizes

The study protocol initially stipulated the use of 220 blood samples from the colonoscopy cohort and 220 blood samples from the surgery cohort for the discovery of the signatures. However, the study protocol was open to changes depending on the results of the discovery process. Additionally, the study protocol initially stipulated the use 220 blood samples from the colonoscopy cohort and 220 blood samples from the surgery cohort for prospective performance evaluation purposes. Again, these samples sizes were open and amenable to change.

### Qualify Control of Clinical Data

All clinical data of all included persons from both cohorts were checked directly from the patient's medical records and entered into study databases, as prescribed in the study protocol. Each item in the study databases were checked independently by two persons. Patients of both cohorts together with their blood samples were withdrawn if any violation of the inclusion of exclusion criteria cited above was detected. In particular for the colonoscopy cohort, all samples and their associated clinical data were excluded from all analyses if the colonoscopy was found to be not complete. Moreover, blood samples were destroyed and clinical data were deleted if the patient decided to withdraw his/her informed consent.

### Recruitment

499 persons scheduled for colonoscopy were recruited into the study; RNA blood samples were taken prior to colonoscopy. The recruitment period for the non-CRC patients lasted from February 9, 2009 until April 3, 2009. In parallel, RNA blood samples were taken from patients with (suspected) diagnosis of CRC prior or during surgery for CRC.

### Sample Selection for RNA-Extraction

The selection criteria of CRC-cases included availability of UICC stage information and the check that the patients did not fulfill any exclusion criteria for the MSKK-study. At this time clinical data of the non-CRC case were not available. Hence, the first 240 patients of the study were selected.

Altogether, 480 PAX-RNA samples, 240 from non-CRC persons and 240 from CRC patients, were randomized into the discovery set. Additionally, the order of processing by the laboratory was randomized. A drop-out rate of 10 %, or 20 cases in each group, was assumed.

### RNA extraction

Total RNA extraction from blood was performed using the QIAGEN PAXgene Blood miRNA Kit together with the QIAcube^{®} robot according to the manufacturer's instructions. The RNA obtained is therefore sometimes referred to as PAX-RNA.

Before starting RNA extraction, the buffers BM3 and BM4 were mixed with the specified amount of 96-100 % Ethanol. Furthermore, the DNAse I (1500 Kunitz units) was dissolved in 550 µl of RNAse free water.

After thawing, the PAX-blood tubes were turned several times to ensure proper mixing and left over night (or minimal two hours) at room temperature. Then, the tubes were centrifuged for 10 minutes at 4000 x g using a swing out bucket. Next, the supernatant was carefully removed from each tube, and the pellets were washed (vortexing until complete resuspension) with 4 ml RNAse free water. After centrifuging again for 10 minutes at 4000 x g, the pellets were resuspended in 350 µl buffer BR1 and transferred to 2 ml processing tubes. The opened tubes were then loaded into the rotor adapters of the centrifuge. Subsequently, all buffers were placed on the respective spots of the reagent bottle holder. After closing the lid, the PAXgene Blood miRNA Part A protocol was started. When it was finished, the instrument door was opened, the RNA containing tubes were closed and subsequently placed on the shaker adaptor. After closing the instrument door again, the PAXgene Blood miRNA Part B protocol was started. When the program was finished, concentration was determined by UV-absorption measurement and the samples were stored at -70°C until use.

For understanding of underlying principles of the automatic procedure the manual protocol is briefly described below. There is no difference between the two methods until the resuspension of the pellet.

To the resupended pellet 300 µl binding buffer (BR2) and 40 µl proteinase K solution was added and mixed by vortexing for 5 seconds. Incubation follows for 10 minutes at 55 °C using a shaker-incubator at 400-1400 rpm. After incubation, the temperature of the shaker-incubator is set to 65 °C. The lysate is pipetted directly into a PAXgene Shredder spin column (lilac) placed in a 2 ml processing tube and centrifuged for 3 minutes at maximum speed (don't not to exceed 20,000 x g). The entire supernatant of the flow-through fraction was carefully transferred the to a fresh 1.5 ml microcentrifuge tube without disturbing the pellet in the processing tube. 350 µl ethanol (96-100%, purity grade p.a.) was added and mixed by vortexing. The mixture is briefly (1-2 seconds at 500-1000 x g) centrifuged to remove drops from the inside of the tube Lid. 700 µl of the sample is pipetted into the PAXgene RNA spin column (red) placed in a 2 ml processing tube, and centrifuged for 1 minute at 8,000-20,000 x g. The spin column was placed in a new 2 ml processing tube (PT), and the old processing tube containing flow-through discarded. Subsequently, the remaining sample was pipetted into the PAXgene RNA spin column, and centrifuged for 1 minute at 8,000-20,000 x g. The spin column was placed in a new 2 ml processing tube and the old processing tube containing flow-through again discarded. Subsequently, 350 µl wash buffer 1 (BR3) was pipetted into the PAXgene RNA spin column. After centrifugation for 1 minute at 8000-20,000 x g the spin column was placed in a new 2 ml processing tube and the old processing tube containing the flow-through again discarded. 10 µl DNase I stock solution is added to 70 µl DNA digestion buffer in a 1.5 ml microcentrifuge tube and mixed by gently flicking the tube followed by a brief centrifugation to collect residual liquid from the sides of the tube. The DNase I incubation mix (80 µl) was pipetted directly onto the PAXgene RNA spin column membrane, and placed on the benchtop (20-30 °C) for 15 minutes. After Incubation, 350 µl wash buffer 1 (BR3) is pipetted into the PAXgene RNA spin column and centrifuged for 1 minute at 8000-20,000 x g. The spin column was placed in a new 2 ml processing tube and the old processing tube containing flow-through again discarded. 500 µl wash buffer 2 (BR4) was pipetted into the PAXgene RNA spin column and centrifuged for 1 minute at 8,000-20,000 x g. The spin column was placed in a new 2 ml processing tube and the old processing tube containing flow-through again discarded. Another 500 µl wash buffer 2 (BR4) was added to the PAXgene RNA spin column and centrifuged for 3 minutes at 8,000-20,000 x g. The spin column is placed in a new 2 ml processing tube and the old processing tube containing flow-through again discarded. The column is centrifuged for 1 minute at 8,000-20,000 x g. The processing tube is discarded and the column was placed on a 1.5 ml microcentrifuge tube. 40 µl elution buffer (BR5) directly pipetted onto the PAXgene RNA spin column membrane and subsequntly centrifuged for 1 minute at 8,000-20,000 x g to elute the RNA. The elution step is repeated using again 40 µl elution buffer (BR5) and the same 1.5 ml microcentrifuge tube. The RNA is denatured for 5 minutes at 65 °C in the shaker-incubator (see above) without shaking.

### Results of RNA-Extraction

The quality control is performed on the Agilent Bioanalyzer. From the RNA-extraction samples, 14 samples (2.92 %) showed RNA integrity numbers (RIN) lower than 3.5, which rendered these samples unfit for microarray hybridization.

### Selection of Samples for Microarray Hybridization

Based on the results of RNA extraction and clinical information about UICC-stages and complete colonoscopy in case of non-CRC cases, 117 samples were authorized for microarray hybridization. The vast majority of drop-outs were incomplete colonoscopies.

### Labeling and Chip Hybridization

Total RNA (100 ng) was amplified with the Ovation^{®} Whole Blood system (Nugen, San Carlos, USA):

### First Strand cDNA Synthesis

1. First Strand reagents A2 & A3 (stored at -20°C) and A1 (stored at -80°C) were obtained.
2. First Strand Enzyme Mix (blue: A3) was placed on ice.
3. First Strand Primer Mix (blue: A1) and First Strand Buffer Mix (blue: A2) were thawed at room temperature. Once thawed, reagents were placed on ice.
4. The contents of A1 were mixed by vortexing for two seconds, and then spun in a microcentrifuge for two seconds.
5. A 96 well PCR plate or 8-strip tubes were placed on ice.
6. The concentration of all RNA samples was adjusted so that the total amount in 5 µl was 50 ng. 5 µl of target RNA was aliquoted into each well of the 96 well plate (or 8-strip tubes) and kept on ice.
7. A1 was aliquoted into 8-strip tubes and kept on ice. See table below for volumes added to each tube of the 8-strip reservoir for the appropriate number of reactions.

| Reagents | 8 reactions = 1 strip | 16 reactions = 2 strips | 24 reactions = 3 strips | 48 reactions = 6 strips | 96 reactions =12 strips |
|---|---|---|---|---|---|
| Volume of A1 primer to add per tube of mini 8-strip reservoir (in mL) | 3 | 6 | 9 | 16 | 32 |

8. 2 µl of A1 was added per sample into the first column of the RNA reaction plate (or 8-strip tubes) using an 8-channel pipette. The reaction was mixed by pipetting up and down 3-4 times. The reaction mix was completely transferred from the pipette tips.
9. New tips were obtained, and the procedure was repeated for each column of the PCR plate (or 8-strip tubes) containing samples.
10. The PCR seal was placed on the plate in the correct orientation, then the plate (or 8-strip tubes) was spun briefly.
11. Plate was placed in a pre-warmed thermal cycler programmed to run:
12. The plate was incubated for five minutes at 65 °C.
13. While samples were incubating, the contents of A2 were mixed by vortexing for two seconds and then spun for two seconds and finally, placed on ice.
14. Shortly before the Primer Annealing step was complete, a Mastermix was prepared by combining A2 and A3 in an appropriately sized tube, using the volumes listed in the table below for various numbers of reactions. **Note:** The Mastermix was mixed by pipetting up and down 3-4 times to ensure the enzyme was mixed well.

**Mastermix Calculations for First Strand Mastermix (in ml)**

| Reagents | 1 reaction | 8 reactions = 1 strip | 16 reactions = 2 strips | 24 reactions = 3 strips | 48 reactions = 6 strips | 96 reactions = 12 strips |
|---|---|---|---|---|---|---|
| A2 (buffer) | 12 | 120 | 240 | 348 | 665 | 1311 |
| A3 (enzyme) | 1 | 10 | 20 | 29 | 55 | 109 |
| | | | | | | |
| Total Volume of Mastermix | 13 | 130 | 260 | 377 | 720 | 1420 |
| | | | | | | |
| Volume of Mastermix to add per tube of mini 8-strip reservoir | | 15.5 | 30 | 45 | 88 | 175 |

15. When the primer annealing was completed, the plate (or 8-strip tubes) was removed from the thermal cycler, then spun briefly and placed on ice.
16. #The thermal cycler was pre-warmed for the next incubation (see program 2 below).
17. 13 µl of the First Strand Mastermix was added per sample to the first column of the reaction plate, using an 8-channel pipette, mixed by pipetting up and down 3-4 times. The reaction mix was completely transferred from the pipette tips.
18. New tips were obtained, and the procedure was repeated for each column.
19. The PCR seal was placed on the plate in the correct orientation, then the plate (or 8-strip tubes) was spun briefly.
20. The plate (or 8-strip tubes) was placed in a pre-warmed thermal cycler programmed to run:
21. The plate (or 8-strip tubes) was incubated at 48 °C for 60 minutes.
22. The plate (or 8-strip tubes) was heated at 70 °C for 15 minutes.
23. Then the plate (or 8-strip tubes) was cooled to 4 °C.
24. While Program 2 was running, the Second Strand Buffer (yellow: B1) and Second Strand Enzyme Mix (yellow: B2) stored at -20°C was obtained. B2 was placed on ice, and B1 thaw was thawed at room temperature. (in preparation for Second Strand cDNA Synthesis, below)
25. Once the thermal cycler temperature had reached 4°C in Program 2, the plate (or 8-strip tubes) was removed from the thermal cycler, then spun briefly and placed on ice.
26. Second strand cDNA synthesis was immediately started.

### Second Strand cDNA Synthesis

1. The contents of B2 were mixed by flicking the tube 6-8 times. Then the contents were spun in a microcentrifuge for two seconds, and placed on ice.
2. The contents of B1 were mixed by vortexing for two seconds. Then the contents were spun for two seconds in a microcentrifuge and placed on ice.
3. A Mastermix was made by combining B1 and B2 in an appropriately sized tube using the volumes listed in table below for the various numbers of reactions. Note: The Mastermix was mixed by pipetting up and down several times to ensure the enzyme was mixed well.

**Mastermix Calculations for Second Strand Mastermix (in ml)**

| Reagents | 1 reaction | 8 reactions = 1 strip | 16 reactions = 2 strips | 24 reactions = 3 strips | 48 reactions = 6 strips | 96 reactions = 12 strips |
|---|---|---|---|---|---|---|
| B1 (buffer) | 18 | 180 | 360 | 522 | 972 | 1944 |
| B2 (enzyme) | 2 | 20 | 40 | 58 | 108 | 216 |
| | | | | | | |
| Total Volume of Mastermix | 20 | 200 | 400 | 580 | 1080 | 2160 |
| | | | | | | |
| Volume of Mastermix to add per tube of mini 8-strip reservoir | | 23 | 46 | 68 | 132 | 260 |

4. A strip of 8 tubes was filled with Second Strand Mastermix and kept on ice. See table above for volumes added to each tube of the 8-strip reservoir.
5. 20 µl of Second Strand master was added per sample to the first column of reaction plate (or 8-strip tubes), using an 8-channel pipette.
6. The reagents in that column were mixed by pipetting up and down 3-4 times, using the same pipette, volume setting, and tips.
7. New tips were obtained, and steps 5 & 6 were repeated for each column was spun briefly.
8. The PCR seal was placed on the plate, then the plate (or 8-strip tubes) briefly.
9. The plate was placed in a pre-warmed thermal cycler programmed to run:
10. The plate was incubated at 37°C for 30 minutes.
11. The plate was heated at 75°C for 15 minutes.
12. The plate was cooled to 4°C.
13. The SPIA Buffer Mix (red: C2), SPIA™ Enzyme Mix (red: C3), and water (green: D1) were obtained from the components stored at -20°C and the SPIA™ Primer Mix (red: C1) stored at -80°C. The enzyme mix was kept on ice. The remaining components were thawed at room temperature and then placed on ice. (in preparation for SPIA Amplification, below)
14. Once the temperature had reached 4°C, the plate was removed from the thermal cycler and placed on ice.
15. The SPIA™ amplification was immediately started.

### SPIA Amplification

1. The contents of C3 were mixed by inverting gently 5 times. The enzyme was well mixed without introducing bubbles, and placed on ice.
2. The contents of C1 were mixed by vortexing for two seconds, and then spun in a microcentrifuge for two seconds, and finally, placed on ice.
3. The contents of C2 were mixed by inverting gently 5 times and placed on ice.
4. ASPIA Mastermix was made by combining C2, C1, water, and C3 in an appropriately sized tube and kept on ice using the volumes listed in table below for the various numbers of reactions. Note: The Mastermix was mixed by pipetting up and down several times to ensure the enzyme is mixed well. The Mastermix was used immediately.

**Mastermix Calculations for SPIA Mastermix (in ml)**

| Reagents | 1 reaction | 8 reactions = 1 strip | 16 reactions = 2 strips | 24 reactions = 3 strips | 48 reactions = 6 strips | 96 reactions = 12 strips |
|---|---|---|---|---|---|---|
| C 1 (primer) | 2 | 18 | 36 | 54 | 106 | 201 |
| C2 (buffer) | 72 | 648 | 1296 | 1944 | 3816 | 7237 |
| D1 (water) | 4 | 36 | 72 | 108 | 212 | 402 |
| C3 (enzyme) | 40 | 360 | 720 | 1080 | 2120 | 4020 |
| | | | | | | |
| Total Volume of Mastermix | 118 | 1062 | 2124 | 3186 | 6254 | 11860 |
| | | | | | | |
| Volume of Mastermix to add per tube of large 8-strip reservoir | | 130 | 260 | 390 | 770 | 1475 |

5. A strip of 8 tubes was filled with SPIA Mastermix and kept on ice. See table above for volumes added to each tube of the 8-strip reservoir.
6. 118 µl of the SPIA^{™} Mastermix was added per sample to the first column of plate (or 8-strip tubes), using an 8-channel pipette.
7. The reagents in the column were mixed by pipetting up and down 3-4 times, using the same pipette, volume setting, and tips.
8. New tips were obtained, and the procedure repeated for each column.
9. At this stage, the SPIA reaction was divided into two plate wells (or 8-strip tubes) for incubation, and during this step the potential for sample cross contamination was high; therefore, care was taken in pipetting the correct columns.
10. 79 µl of the SPIA™ reaction mixture was transferred from each column of the reaction plate to the new plate wells (or 8-strip tubes), using an 8-channel pipette.
**Note:** This was done to accommodate the 100ul max volume recommendation for heating in the thermal cyclers.
11. Using fresh tips, this was repeated for each column.
12. A PCR seal was placed on the plate (or close caps to 8-strip tubes), then the plates (or 8-strip tubes) were spun for 2 seconds.
13. The plate was placed in a pre-warmed thermal cycler according to Program SPIA™ Amplification 1:

| | |
|---|---|
| Program SPIA™ Amplification 1 | 48°C for 30 minutes, then 4°C forever |

14. The plate was incubated at 48°C for 30 minutes.
15. Once the temperature had reached 4°C, the plate was removed from the thermal cycler and placed on ice.
16. 3 ul Whole Blood Reagent (C4) was added to each well. Each well was mixed by pipetting up and down 3-4 times with a large volume (>40 ul).
17. The plate (tubes) was resealed and returned to pre-warmed thermal cycler running Program SPIA™ Amplification 2:

| | |
|---|---|
| Program SPIA™ Amplification 2 | 48°C for 30 minutes, then 95°C for 5 minutes, then 4°C forever |

18. The plate (tubes) was incubated at 48°C for 30 minutes.
19. The samples were heated to 95°C for 5 minutes.
20. The samples were cooled to 4°C.
21. The plate (or 8-strip tubes) was removed from the thermal cycler and placed on ice for immediate use in purification or at -20°C (to store for purification at a later time).

After SPIA^{™} Amplification, the cDNA was purified with a QIAquick PCR purification spin column (QIAGEN, Hilden) using the Qiacube robot. One microgram of purified cDNA was then subjected to Cy3 labeling with the NimbleGen One-Color DNA Labeling Kits (Roche NimbleGen, Inc.; Madison, WI, USA.) following the manufacturers instructions:
1. The following solution was prepared:

| | |
|---|---|
| Random Primer Buffer (vial 2) | 1100 µl |
| β-Mercaptoethanol | 2 µl |

| | |
|---|---|
| (Fresh buffer was prepared each time the primers were resuspended.) | |

2. Cy3 Random Nonamers (vial 3) were briefly centrifuged because some of the product could have dislodged during shipping. The primer was diluted in 1,050 µl of Random Primer Buffer with β-Mercaptoethanol. 40 µl individual reaction volumes were aliquoted in 0.2 ml thin-walled PCR tubes and store at -15 to -25°C, protected from light.
3. The following components were assembled in separate 0.2 ml thin-walled PCR tubes:

| | |
|---|---|
| cDNA | 1 µg |
| Diluted Cy3 Random Nonamers from step 2 | 40 µl |
| PCR Grade Water (vial 1) fill to volume | 80 µl |

4. The samples were heat-denature in a thermocycler at +98°C for 10 minutes. Quick-chilled in an ice-water bath for 2 minutes.
5. The following dNTP/Klenow master mix was prepared for each sample prepared in step 4. All reagents and dNTP/Klenow master mix were kept on ice. After the addition of Klenow, the samples were no longer vortexed.

| | |
|---|---|
| dNTP Mix (10 mM each dNTP) (vial 5) | 10 µl |
| PCR Grade Water (vial 1) | 8 µl |
| Klenow Fragment (3'->5' exo-) 50U/µl (vial 4) | 2 µl |

6. 20 µl of the dNTP/Klenow master mix prepared in step 5 was added to each of the denatured samples prepared in step 4 and kept on ice. Total volume was now 100 µl
7. The samples were mixed well by pipetting up and down 10 times. No vortexing took place after the addition of Klenow.
8. The samples were spun quickly to collect contents in bottom of the tube.
9. The samples were incubated for 2 hours at +37°C in a thermocycler with a heated lid, and protected from light.
10. The reaction was stopped by adding 21,5µl of the Stop Solution (vial 6). Total volume was now 121,5 µl
11. The samples were vortexed briefly, spun, and the entire contents were transferred to a 1.5 ml tube containing 110 µl isopropanol. Total volume was now 231,5 µl
12. The samples were vortexed well and incubated for 10 minutes at +15 to +25°C, protected from light.
13. The samples were centrifuged at 12,000 x g for 10 minutes. The supernatant was removed with a pipette. The pellet was pink.
14. The pellet was rinsed with 500 µl 80% ice-cold ethanol. The pellet was dislodged from tube wall by pipetting a few times.
15. The pellet was centrifuged at 12,000 x g for 2 minutes. The supernatant was removed with a pipette.
16. The contents were dried in a DNA vacuum concentrator on low heat until dry (approximately 5 minutes), protected from light.
17. The tubes were spun briefly prior to opening. Each pellet was rehydrated in 25 µl PCR Grade Water (vial 1).
18. The pellet was vortexed for 30 seconds, quick-spun, and the contents were collected in the bottom of the tube. Either vortexing was continued or the samples were left at +15 to +25°C, protected from light, for approximately 5 minutes or until the pellet was completely rehydrated, then vortexed again and quick-spun.
19. Each sample was quantitated using the following formula:
Concentration (µg/ml) = A260 x 50 x Dilution Factor. Based on the concentration,
calculate the volume of Cy3-labeled cDNA sample required for each hybridization to obtain 4µg.

Four micrograms of the labeled cDNA was then hybridized onto NimbleGen Human Gene Expression 12x135K Arrays (Roche NimbleGen, Inc.; Madison, WI, USA) according to the manufacturer's instructions:

### Sample preparation

1. The Hybridization System was set to +42°C. With the cover closed, at least 3 hours were allowed for the temperature to stabilize.
2. The dried sample pellet was resuspended in 5µl water and 3.3 µl of the appropriate Sample Tracking Control. Each sample to be hybridized an array was resuspended in a unique STC. STCs used for each sample was recorded. If Sample Tracking Controls were not used, then the dried sample pellet was resuspended in 8.3 µl water.
3. The samples was vortexed well and spun to collect contents at bottom of the tube.
4. Using components from a NimbleGen Hybridization Kit, the hybridization solution
master mix was prepared.

| | |
|---|---|
| 2X Hybridization Buffer (vial 1) | 88.5 µl |
| Hybridization Component A (vial 2) | 35.4 µl |
| Alignment Oligo (vial 3) | 3.6 µl |

The amount was sufficient to hybridize all arrays on one slide. To hybridize multiple slides,
the amounts were adjusted accordingly.
5. 8,7 µl of hybridization solution was added to the resuspended pellet (3,3 µl) from step 2.
6. The sample was vortexed well (approximately 15 seconds) and spun to collect contents in bottom of the tube. The sample was incubated at +95°C for 5 minutes, protected from light.
7. The tubes were placed at +42°C (in the Hybridization System sample block or heat block) for at least 5 minutes or until ready for sample loading. The tubes were vortexed and spun prior to loading.

### The Preparation of mixers

1. The HX12 mixer was located and removed from its package. A compressed gas nozzle was used to gently blow compressed nitrogen or argon gas across the mixer and moved slowly to remove any dust or debris. Canned compressed air for this purpose was not used. Samples were loaded within 30 minutes of opening the vacuum-packaged mixer to prevent the formation of bubbles during loading and/or hybridization.
2. The Precision Mixer Alignment Tool (PMAT) was positioned with its hinge on the left. The PMAT was opened.
3. The mixer was snapped onto the two alignment pins on the lid of the PMAT with the tab end of the mixer toward the inside hinge and the mixer's adhesive gasket facing outward.
4. While pushing back the plastic spring with a thumb, the slide was placed in the base of the PMAT so that the barcode was on the right and the corner of the slide sat against the plastic spring. The NimbleGen logo and barcode number was readable. The thumb was removed when the spring had engaged the corner of the slide and the entire slide was registered to the edge of the PMAT to the rightmost. It was ensured that the slide was lying flat against the PMAT. Compressed nitrogen or argon gas was gently blown across the mixer and slide to remove dust.
5. Using forceps, the backing was removed from the adhesive gasket of the mixer, and the lid of the PMAT was closed so that the gasket makes contact with the slide.
6. The lid was lifted by grasping the long edges of the PMAT while simultaneously applying pressure with a finger through the window in the lid of the PMAT to free the mixer-slide assembly from the alignment pins.
7. The mixer-slide assembly from the PMAT was removed.
8. The mixer-slide assembly was placed on the back of a +42°C heating block for 5 minutes to facilitate complete adhesion of the mixer to the slide.
9. The Mixer Brayer rubbed over the mixer with moderate pressure to adhere the adhesive gasket and to remove any bubbles. A corner of the Mixer Brayer was used first to rub the borders between the arrays and then was used to rub around the outside of the arrays. The adhesive gasket became clear when they were fully adhered to both surfaces.
10. The mixer-slide assembly was placed in the slide bay of the Hybridization System.

### Loading & Hybridizing Samples

1. The manufacturers loading diagram was referred to when loading samples to localize the appropriate fill and vent ports.
2. The following was kept in mind when the samples were loaded:
   - When pipetting the sample before loading, residual volume was left in the sample tube to avoid introducing bubbles. The volumes needed (6 µl) allowed for a residual volume.
   - After aspirating the designated sample volume, the pipette tip was inspected for air bubbles. If bubbles existed, the pipette was dispensed and reloaded.
   The following was kept in mind when the samples were loaded:
   - The pipette tip was kept perpendicular to the slide to avoid possible leakage at the fill port.
   - Gentle pressure of the tip was applied into the port to ensure a tight seal while loading the sample.
3. The sample was loaded into a fill port. Due to the close proximity of the fill and vent ports, care was taken not to overfill the arrays. The sample was loaded until it entered the vent port channel. The sample was not allowed to come to the surface of the HX12 mixer. Any overflow from the fill and vent ports was dried with a cotton swab after loading the array. Small bubbles occasionally formed in the corners of the mixer-slide assembly during loading. These bubbles dissipated upon mixing and did not compromise the data.
4. One mixer port seal was used to cover both the fill and vent ports on the mixers, one chamber at a time was filled and sealed. The mixer port seal was pressed, using uniform pressure across the seal to adhere.
5. Forceps were used to press the mixer port seal around the fill and vent ports to ensure it had adhered in those areas
6. The bay clamp was closed.
7. The Mixing Panel was turned on in the Hybridization System, the mix mode was set to B, and the mix button was pressed to start mixing. That the Hybridization System recognizes the slide in each occupied bay (its indicator light becomes green) was confirmed.
8. Approximately 10 minutes after the Hybridization System was started:
   - The setting of the mix mode to B was verified.
   - The display of a green light for all occupied stations was verified.
9. The sample was hybridized at +42°C to the array(s) for 16 - 20 hours.

### Washing of Hybridized Arrays

1. The components of the NimbleGen Wash Buffer Kit and NimbleGen Array Processing Accessories were located. Prior to the first use of the Wash Buffer Kit, the DTT was reconstituted. In a fume hood, 1 M DTT solutions were prepared by adding 1.2 ml of water (vial 5) to each tube of dry DTT (vial 4). After reconstitution, the 1 M DTT solutions were stored at -15 to -25°C.
2. Before removing the mixer-slide assemblies from the Hybridization System, Washes I, II, and III were prepared by adding 10X Wash Buffer I, II, or III (vial 1, 2, or 3) in each case to 243 ml water and 27 ml DTT solution from step1. Two containers of Wash I were prepared.
3. To facilitate the removal of the mixer, a shallow dish containing Wash I to +42°C was heated. The temperature of Wash I was measured at every use. The remaining three wash solutions were kept at +15 to +25°C.
4. The Mixer Disassembly Tool was inserted into the shallow dish containing warm Wash I. When multiple slides were washed, a slide rack was inserted into the wash tank containing Wash I at +15 to +25°C.
5. The mixer-slide assembly was removed from the Hybridization System and was loaded into the Mixer Disassembly Tool, which was immersed in the shallow dish containing warm Wash 1.
6. The mixer-slide assembly was not allowed to cool before removing the mixer. Power was kept on to the Hybridization System's heat block and mixer system during mixer-slide disassembly, and each mixer-slide assembly was transferred one at a time to Wash I for immediate removal of the mixer.
7. With the mixer-slide assembly submerged, the mixer was carefully peeled off the slide. The Mixer Disassembly Tool was held flat while removing the mixer to avoid any horizontal movement or scraping with the mixer across the slide. Care was taken not to touch the array surface of the slide.
8. As the mixer is extremely flexible, care was taken to peel the mixer off slowly to avoid breaking the slide.
   The mixer was discarded, and the slide removed from the Mixer Disassembly Tool.
9. The slide was gently agitated for 10 - 15 seconds in the shallow dish containing warm Wash I to quickly remove the hybridization buffer. To achieve good array uniformity, the hybridization buffer was quickly and evenly washed off the slide surface as soon as the mixer was removed.
   When washing multiple slides, the slide was transferred with the barcode at the top into a slide rack in the wash tank that contained Wash I. When washing one slide, the slide was transferred into a slide container that contained Wash 1. It was agitated vigorously for 10 - 15 seconds.
   In order to ensure high quality data, the microarray area of the slide was kept wet at all times during all wash steps.
10. When a NimbleGen Microarray Dryer or other microarray dryer that dries multiple slides at a time was used, steps 4 - 9 were repeated until all the mixer was removed from all slides to wash. Each slide was loaded into the slide rack with the array facing the same direction.
11. The slides were washed for an additional 2 minutes in Wash I with vigorous, constant agitation. When multiple slides were washed, the rack was moved up and down with enough agitation to make foam appear. When one slide was washed, the slide container was shaken at least 20 times every 10 seconds.
   Several times during the wash, the wash tank was rocked so the wash solution covered and cleaned the top of the slides.
12. The rack was quickly blotted several times using paper towels to minimize buffer carryover. The slide(s) was transferred to Wash II and washed for 1 minute with vigorous, constant agitation. When multiple slides were washed, the wash tank was rocked so the wash solution covered and cleaned the tops of the slide(s).
   The slides were not allowed to dry between the wash steps.
13. The slide(s) was transferred to Wash III and washed for 15 seconds with vigorous, constant agitation. When multiple slides were washed, the wash tank was rocked so the wash solution covered and cleaned the tops of the slide(s).
14. The slide(s) was removed from Wash III. The slide(s) was spin-dried in a NimbleGen Microarray Dryer or other microarray dryer per the manufacturer's recommendation. For a NimbleGen Microarray Dryer, the drying time was 2 minutes (120 seconds).
15. The slide(s) was removed from the NimbleGen Microarray Dryer or other microarray dryer. The edges were blot-dried with lint-free paper to remove any residual moisture.
   The steps for scanning the array(s) were carried out immediately thereafter.
16. When the slides could not be scanned immediately after washing, they were kept in their original slide case in a dark desiccator until they were ready to be scanned.

The array was then scanned with the NimbleGen MS 200 Microarray Scanner and Hybridization signals were collected with the NimbleGen the MS 200 Data Collection Software, using the default settings. Text files were exported and imported into R (Bioconductor package) for further analysis.

### Microarray Quality Control

Chip images were visually inspected for surface errors. All chips passed this test. The microarray data were quality controlled using spearman correlation with a cutoff set to 0.85. One chip was excluded from further analysis.

### Discovery Procedure

In brief, from all raw measurement data from microarray hybridizations scheduled for the discovery (i.e., from samples selected for discovery, and measurements that passed quality control) the median value of the three probesets existing for each transcript was used and the logarithmic values were quantile normalized. The statistical software systems R (version 2.9.0) and Bioconductor (version 1.6) (for this software, see http://cran.r-project.org/, http://www.r-project.org/ and http://www.bioconductor.org, also Ross Ihaka and Robert Gentleman. R: A language for data analysis and graphics. Journal of Computational and Graphical Statistics, 5(3): 299-314, 1996; Gentleman et al 2005: Bioinformatics and Computational Biology Solutions Using R and Bioconductor, Springer, New York, NY) were used for anlysis.

The resulting normalized array data were then partitioned into groups of samples in a double nested bootstrap approach (Efron (1979) Bootstrap Methods--Another Look at the Jackknifing, Ann. Statist. 7, 1-6). In the outer loop of this bootstrap, the samples were partitioned into an outer test set and an outer training set. In the inner bootstrap loop, this outer training set was partitioned again into an inner training set and an inner test set.

On the inner training set, probeset relevance was estimated through a decision-tree-analysis. The influence of each feature was determined from its contribution to the classification error: In case the error of a probeset increases due to the permutation of the values of a probeset while the values of all other probesets remain unchanged, this probeset is weighted more strongly.

The relevance evaluations of the individual inner loops were combined for each external loop and the chosen probesets were used to train a support vector machine (Bernhard Schölkopf, Alex Smola: Learning with Kernels: Support Vector Machines, Regularization, Optimization, and Beyond, MIT Press, Cambridge, MA, 2002, also see citations above). This classifier was trained on the outer training set (which was already used for its feature selection) and applied to the outer test set of samples. For this classification, the same methods and parameters were use as for the intended application on external data (for example as part of validation): the details are described below as application for the RNAs of the invention. The whole external loop of the bootstrap procedure is a simulation of later classification of unknown data. Its average performance over all external loops gives a prospective estimate of the performance of the classification procedure (mainly based on the chosen biomarker set). The common mistake of overfitting, i.e., the overly optimistic evaluation of a classification method on its discovery set, is thereby avoided.

Finally, the results of all inner loops and of all external loops were combined to form a common biomarker set with prospective estimates of performance.

### Application of the Invention

A number of RNAs that result from the discovery above can be used with state of the art classification methods as described in the cited literature and as known by a person of skill in the art. As any classification, it will require a representative training set, which the inventors obtained through a clinical study fulfilling the requirements described above. Part of the training set is the necessary clinical information (carcinoma patient or healthy control, as defined by the clinical study). Similar to the clinical requirements, this description of the algorithmic part of the application of the invention also presupposes the described lab process and quality controls were applied.

The training set of microarray raw data was condensed by the same method as the discovery set in the section "Discovery Procedure" above: the median value of the three probesets existing for each transcript was used and the logarithmic values were quantile normalized. A standard implementation of support vector machines was used, such as the one contained in Bioconductor's (see references above) package for machine learning "e1071" with parameter "kernel" set to "linear" (preferential version for this package is 1.5-19).

It is important not to present the classification information on the training data as numeric data, but as categorical data. This can be ensured by passing the corresponding arguments as an R "factor" (for example as in "svm(..., as.factor(clinicalData),...)". Otherwise this svm algorithm will use the wrong type of classification.

To apply this svm, using the same software package, to any kind of new microarray data, a condensation step is necessary just as for the discovery data. It is possible to simply reapply the condensation method above to each individual new sample in combination with the whole discovery set. This first method approximates the preferential method well, which is to explicitly compute a condensation model or parameter based on the discovery data and apply it to the test data. This can be done with the software package on.farms, preferentially version 1.4.1, available from the author (see references above). The application of the described svm to the thus condensed test data produces the desired decision value.

The embodiments illustrated and discussed in this specification are intended only to teach those skilled in the art the one way to make and use the invention. Modifications and variation of the above-described embodiments of the invention are possible without departing from the invention, as appreciated by those skilled in the art in light of the above teachings. It is therefore understood that, within the scope of the claims and their equivalents, the invention may be practiced otherwise than as specifically described.

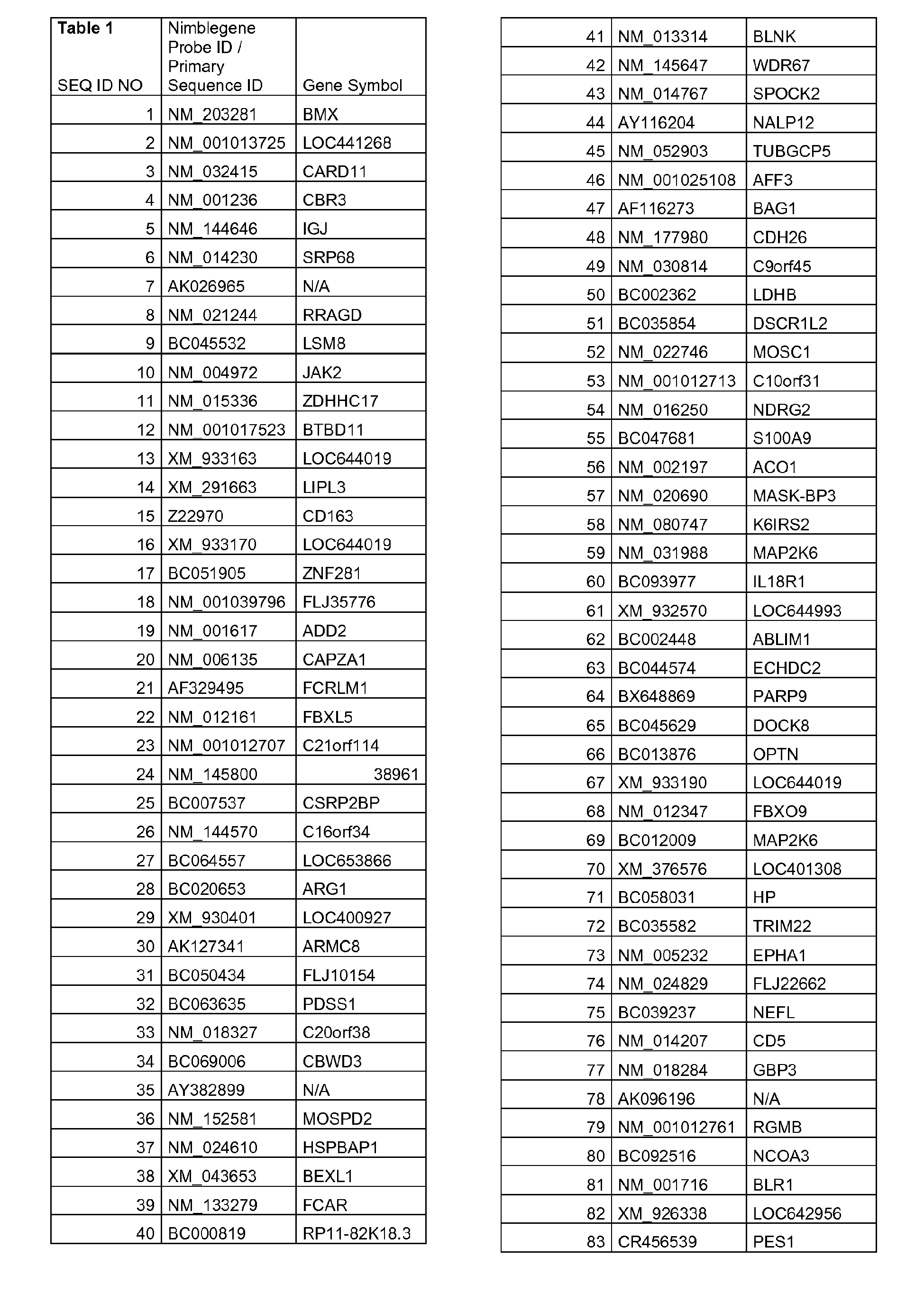

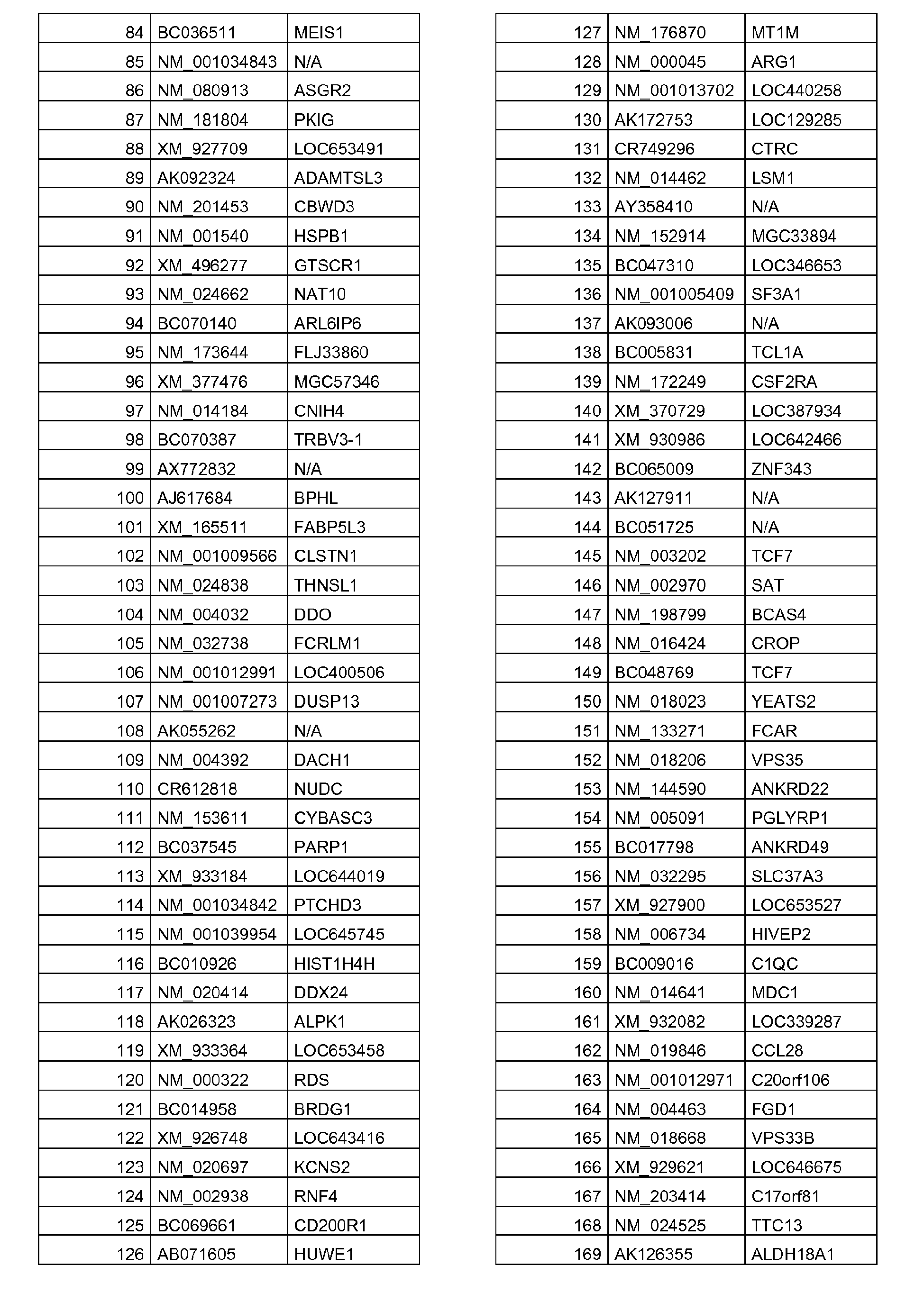

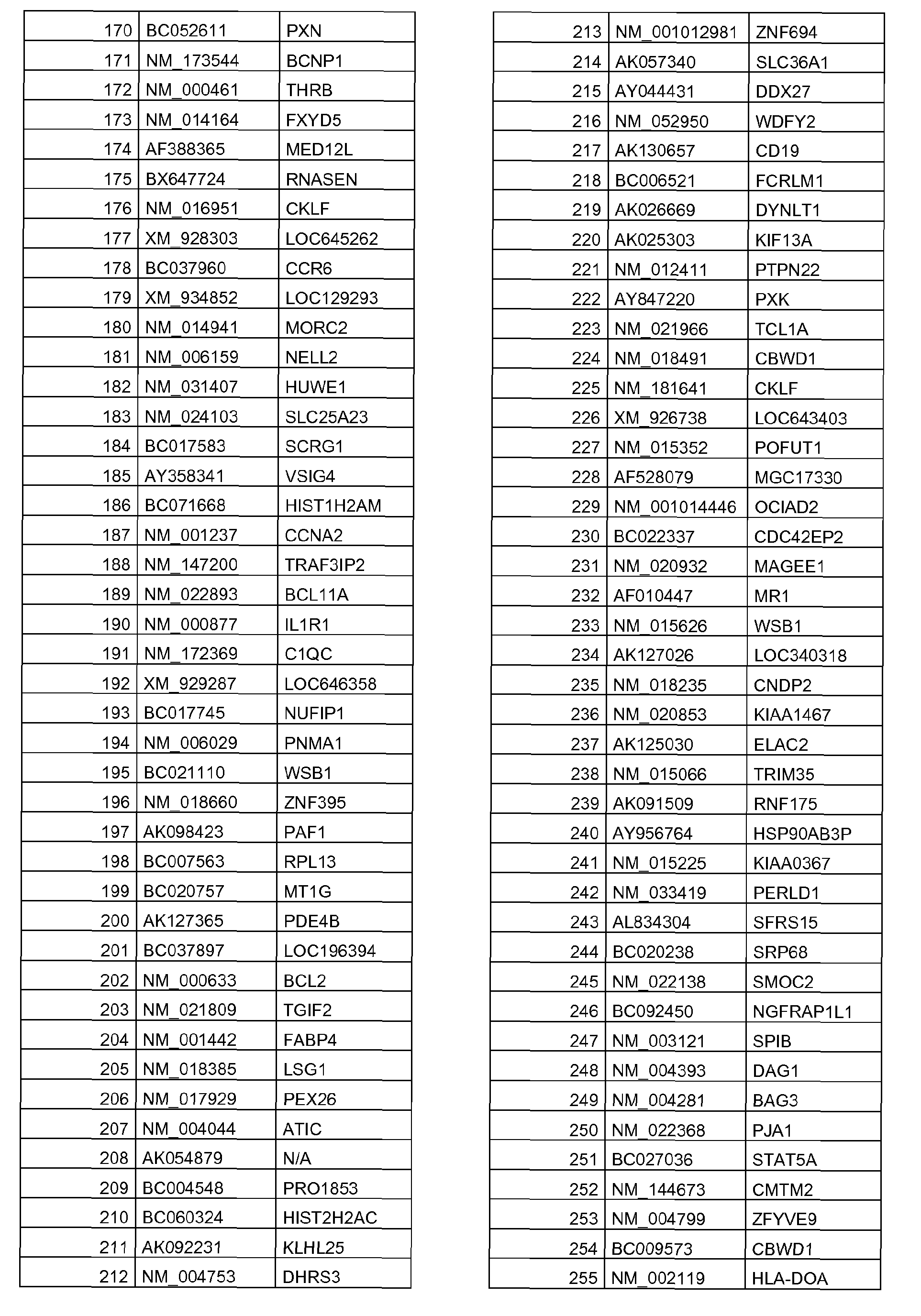

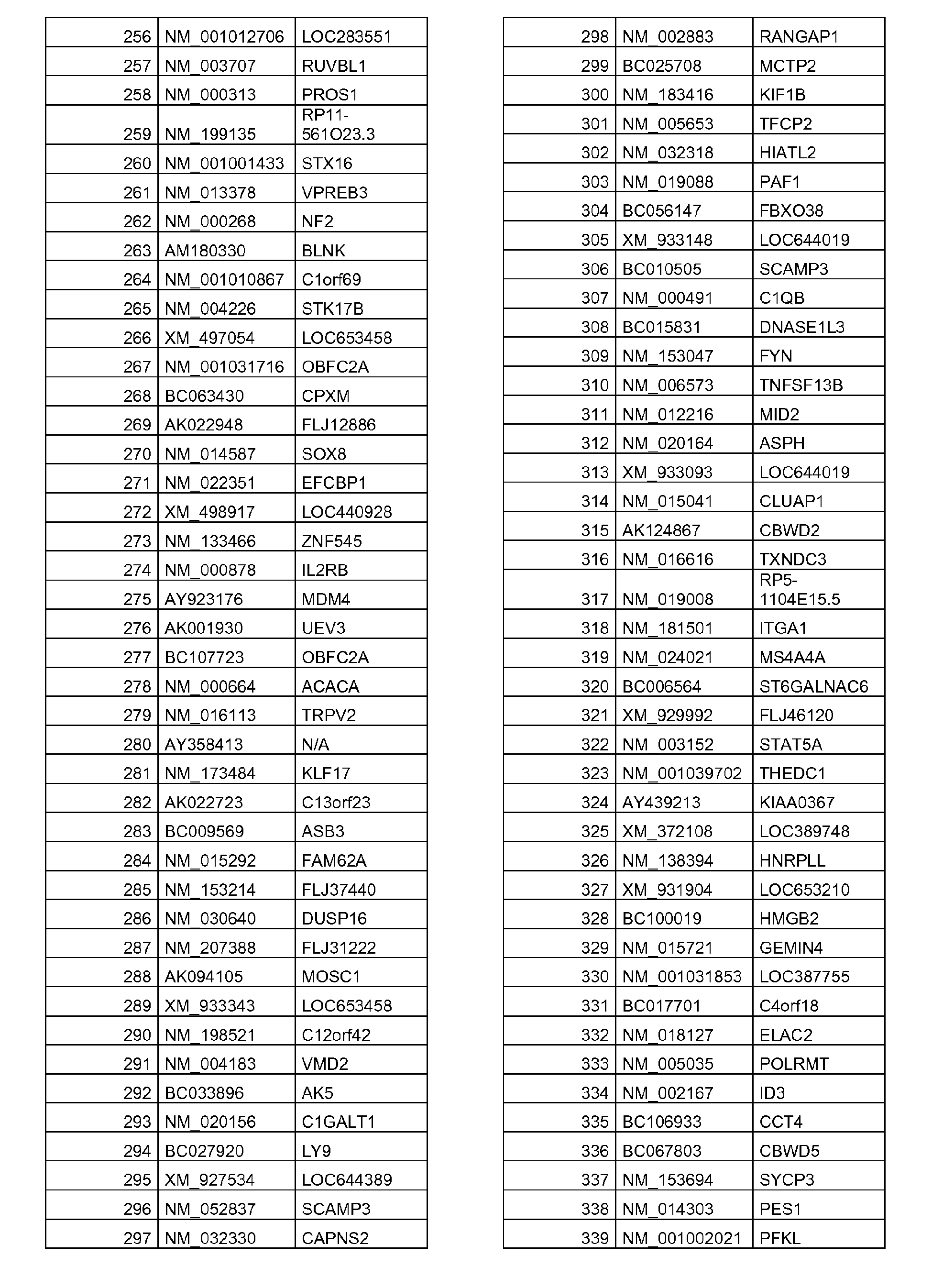

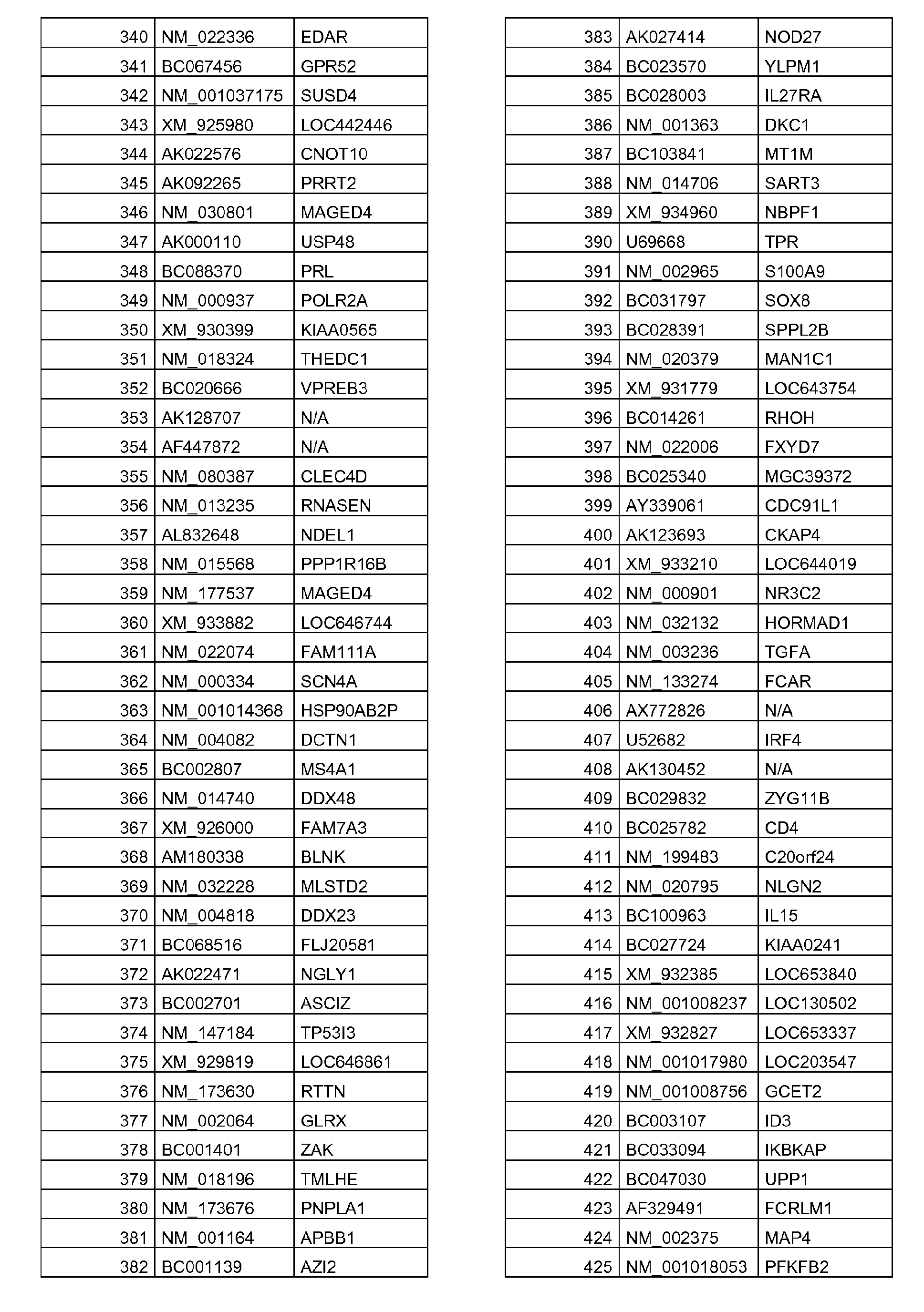

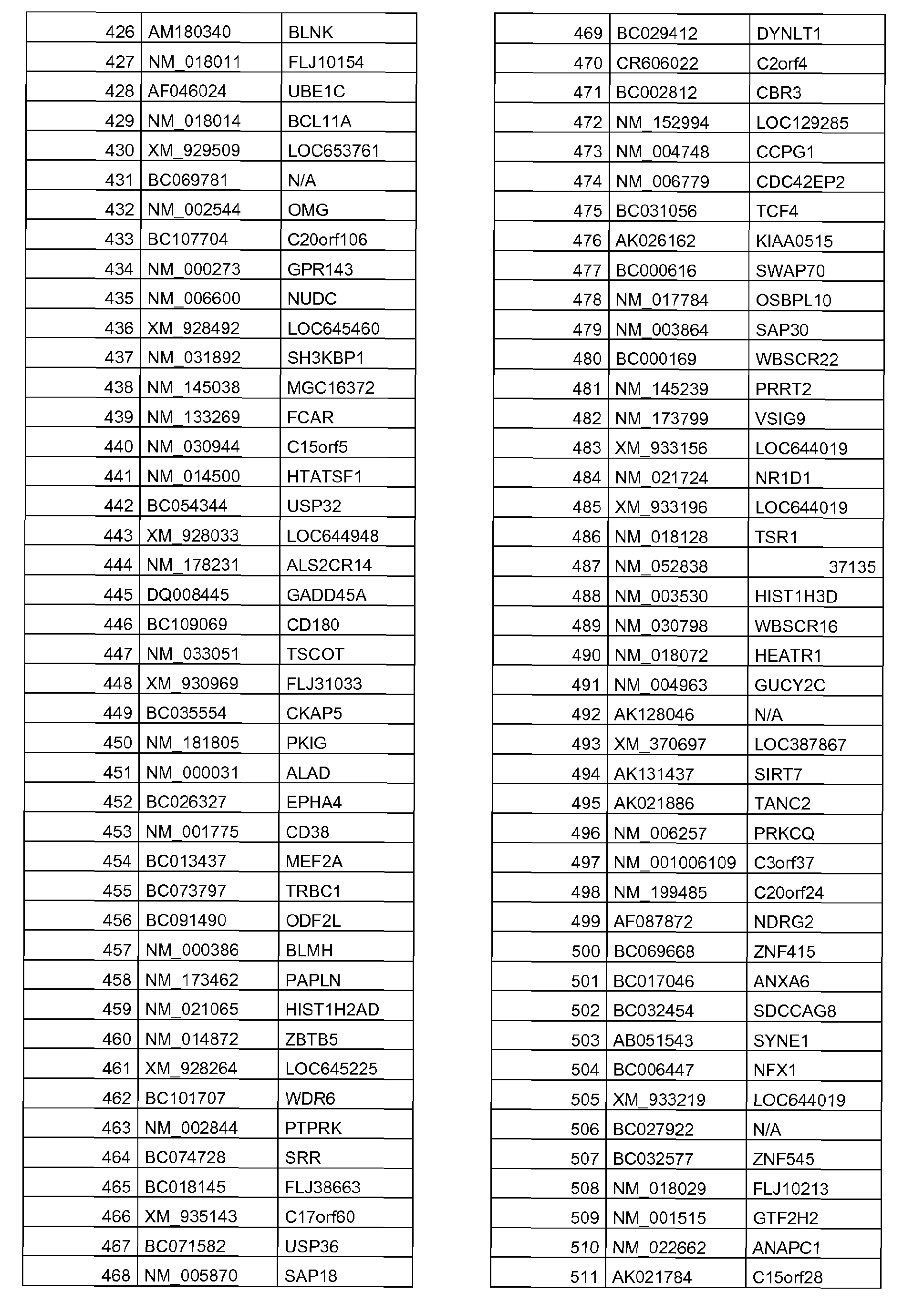

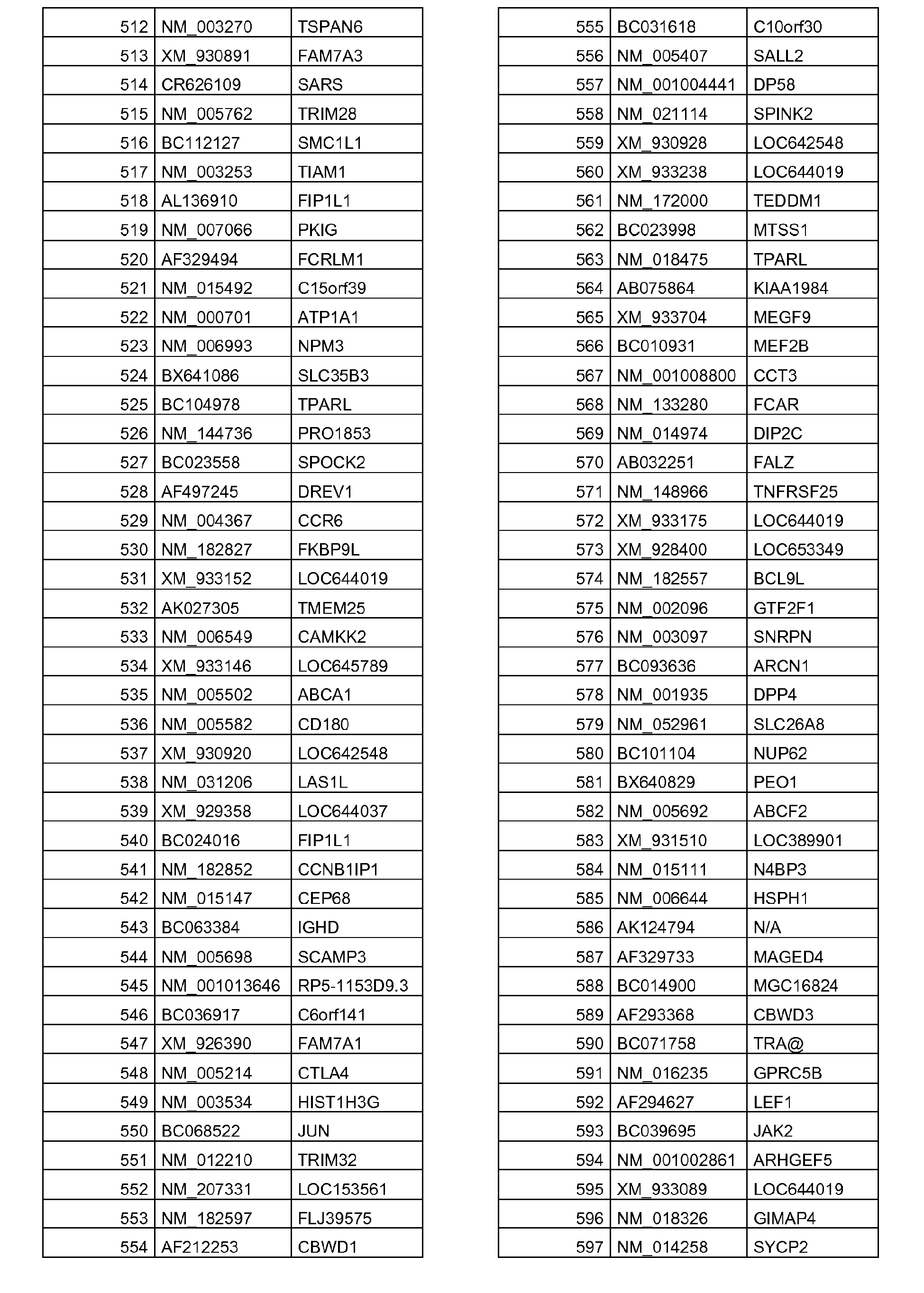

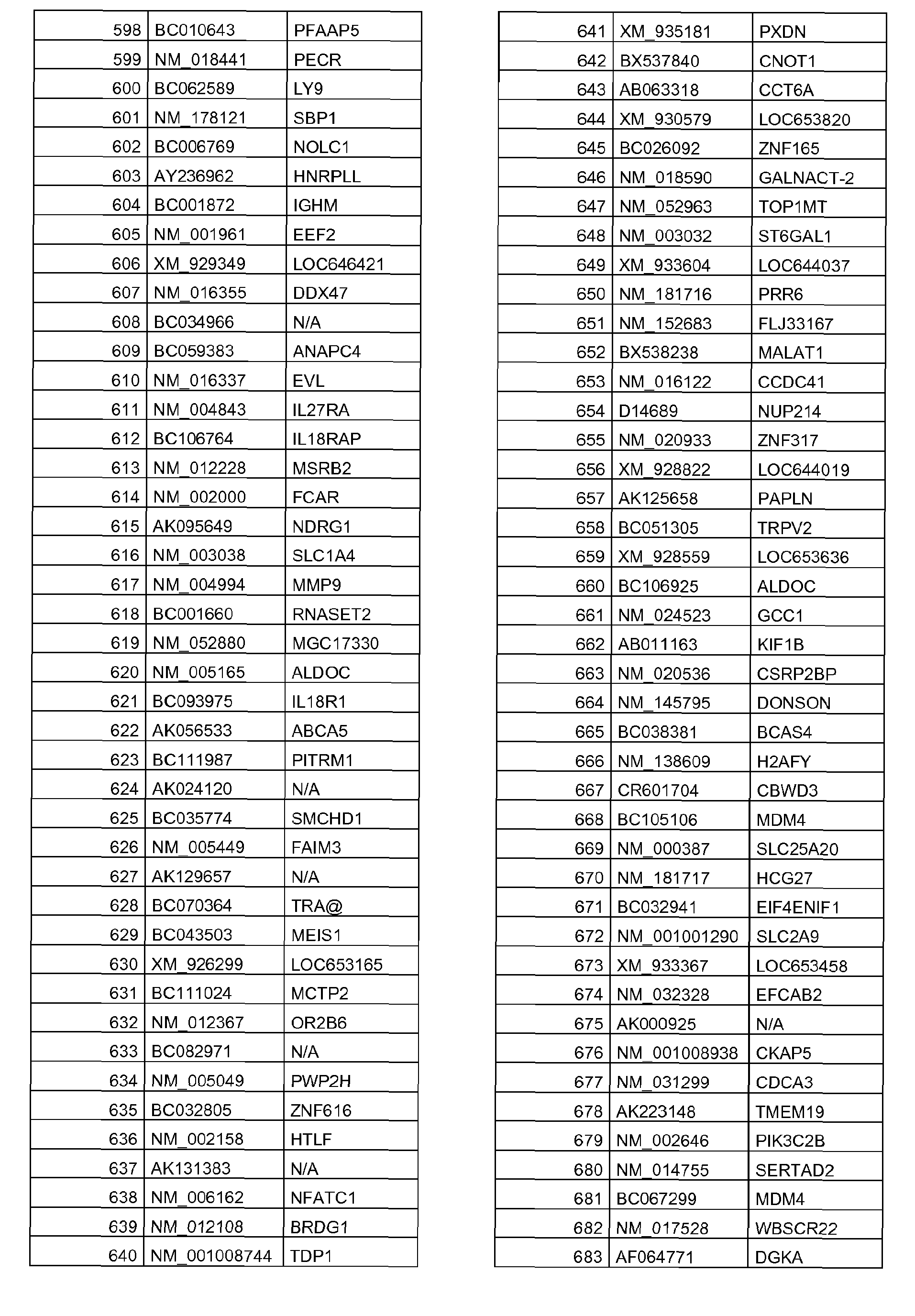

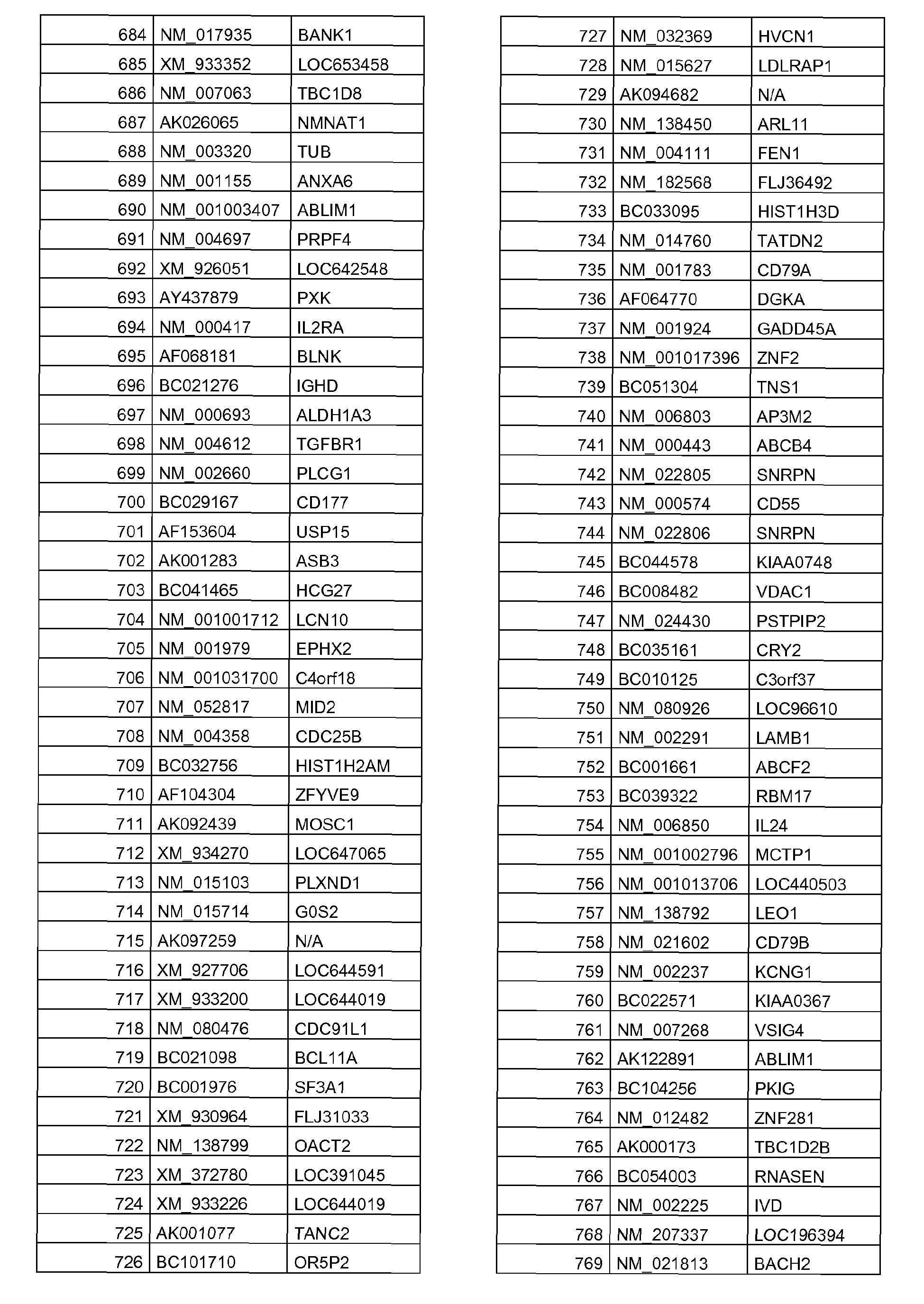

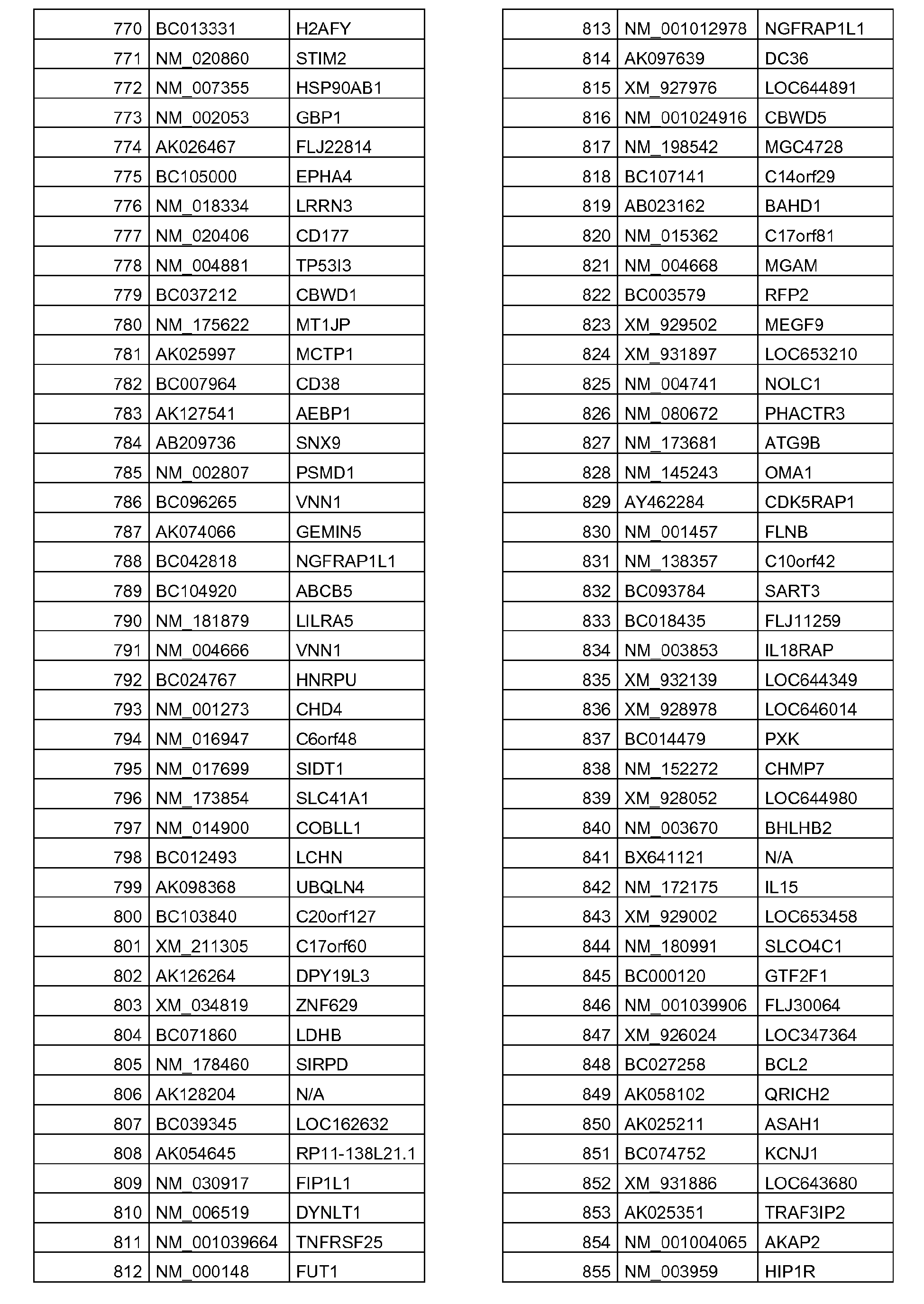

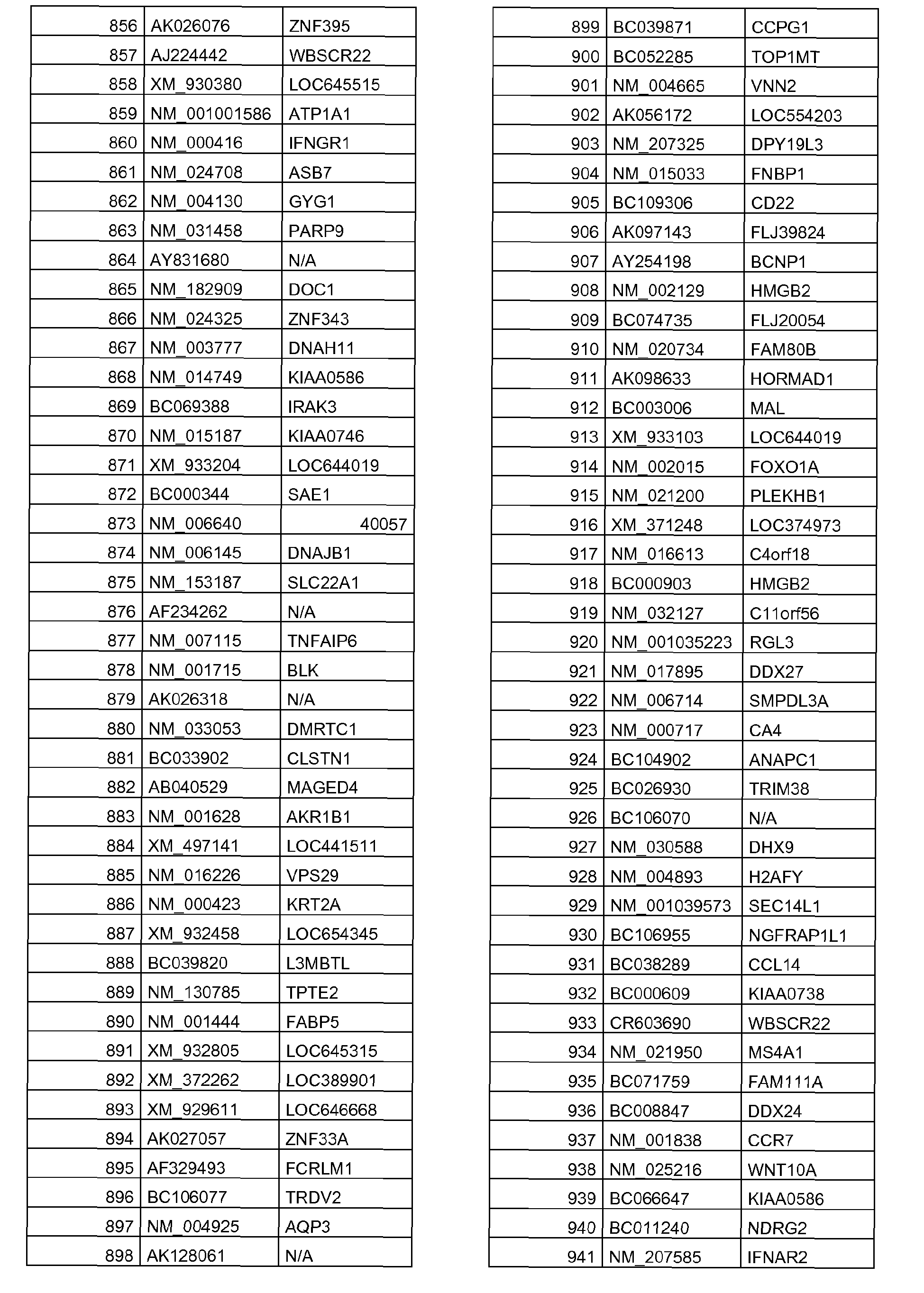

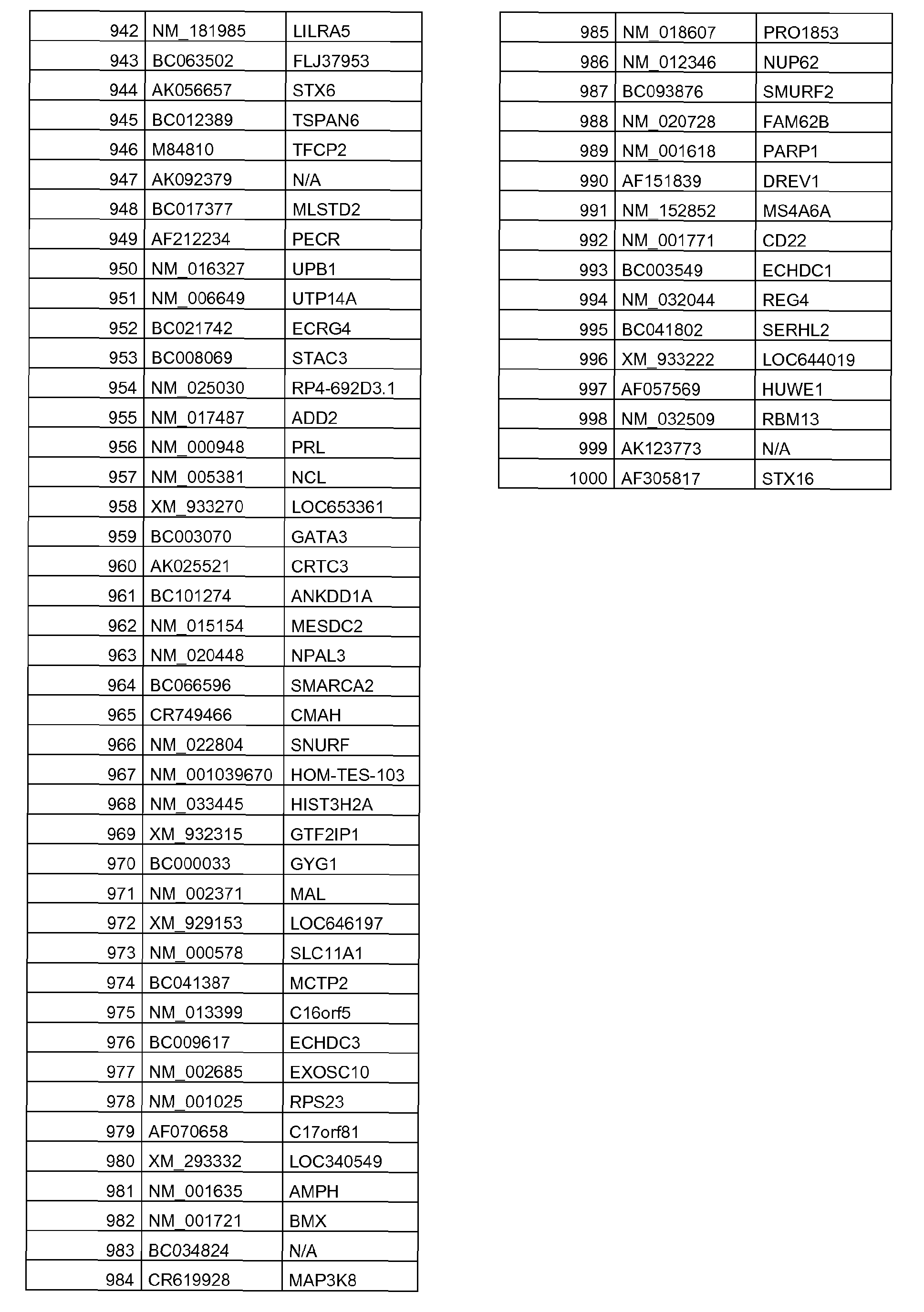

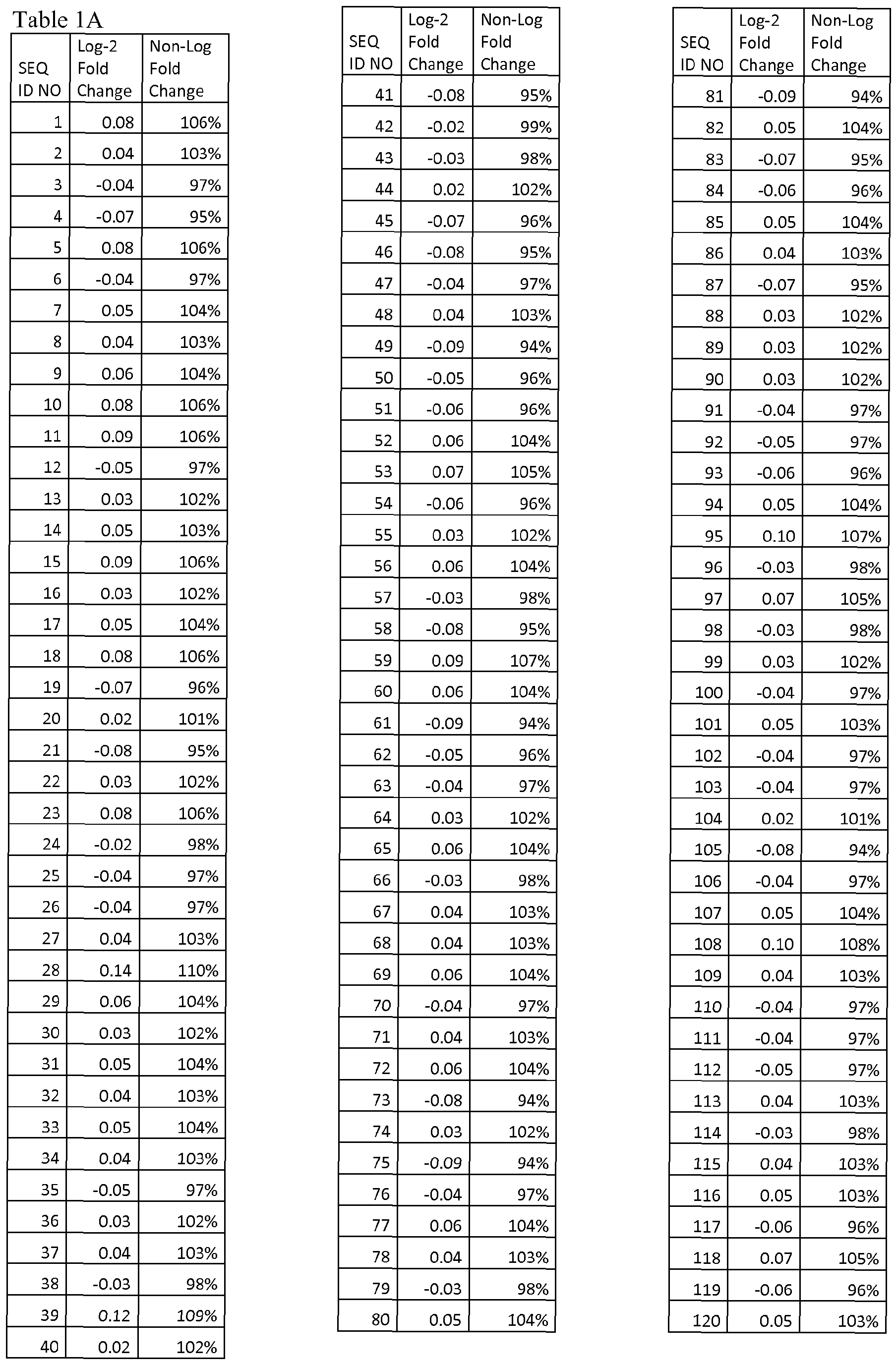

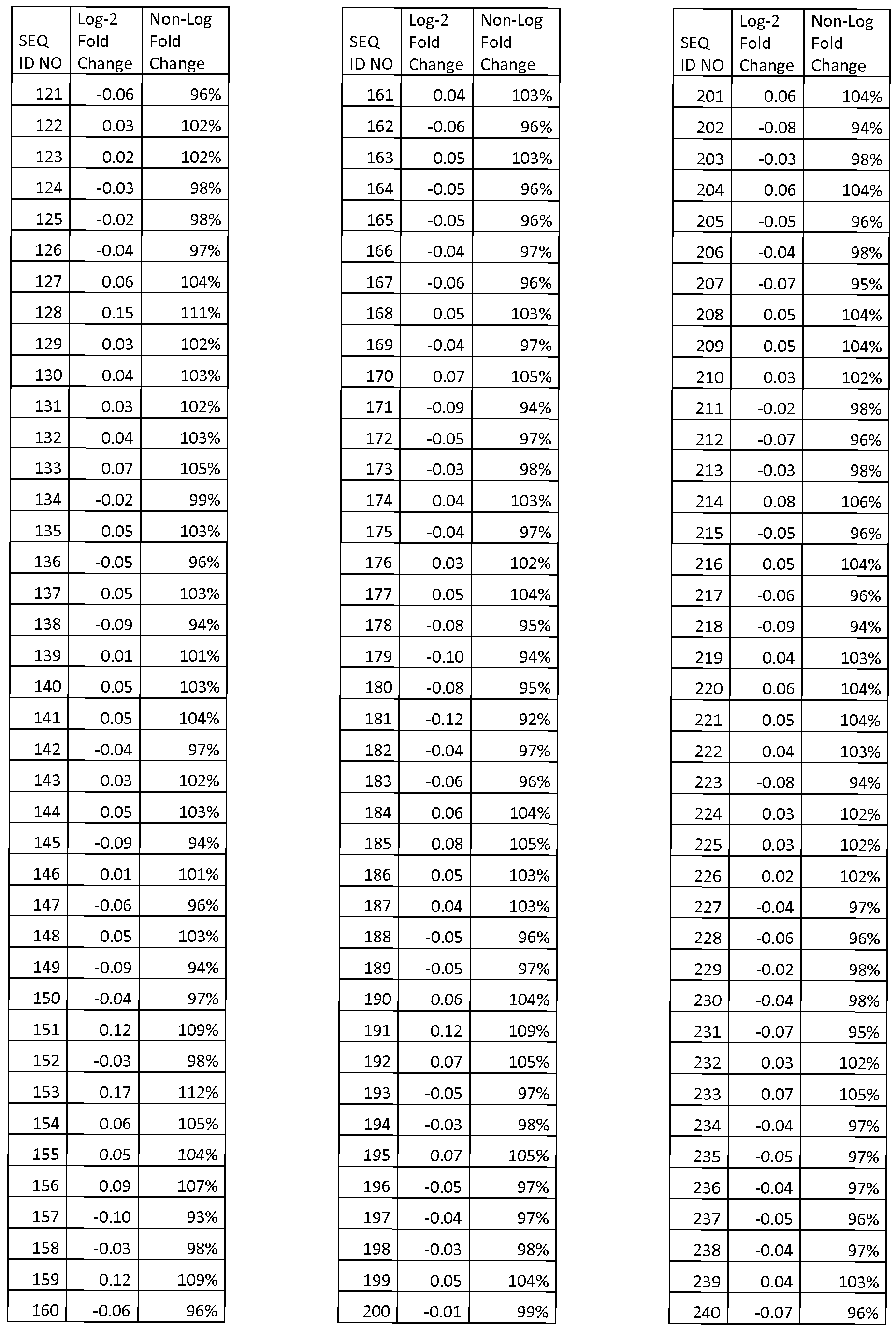

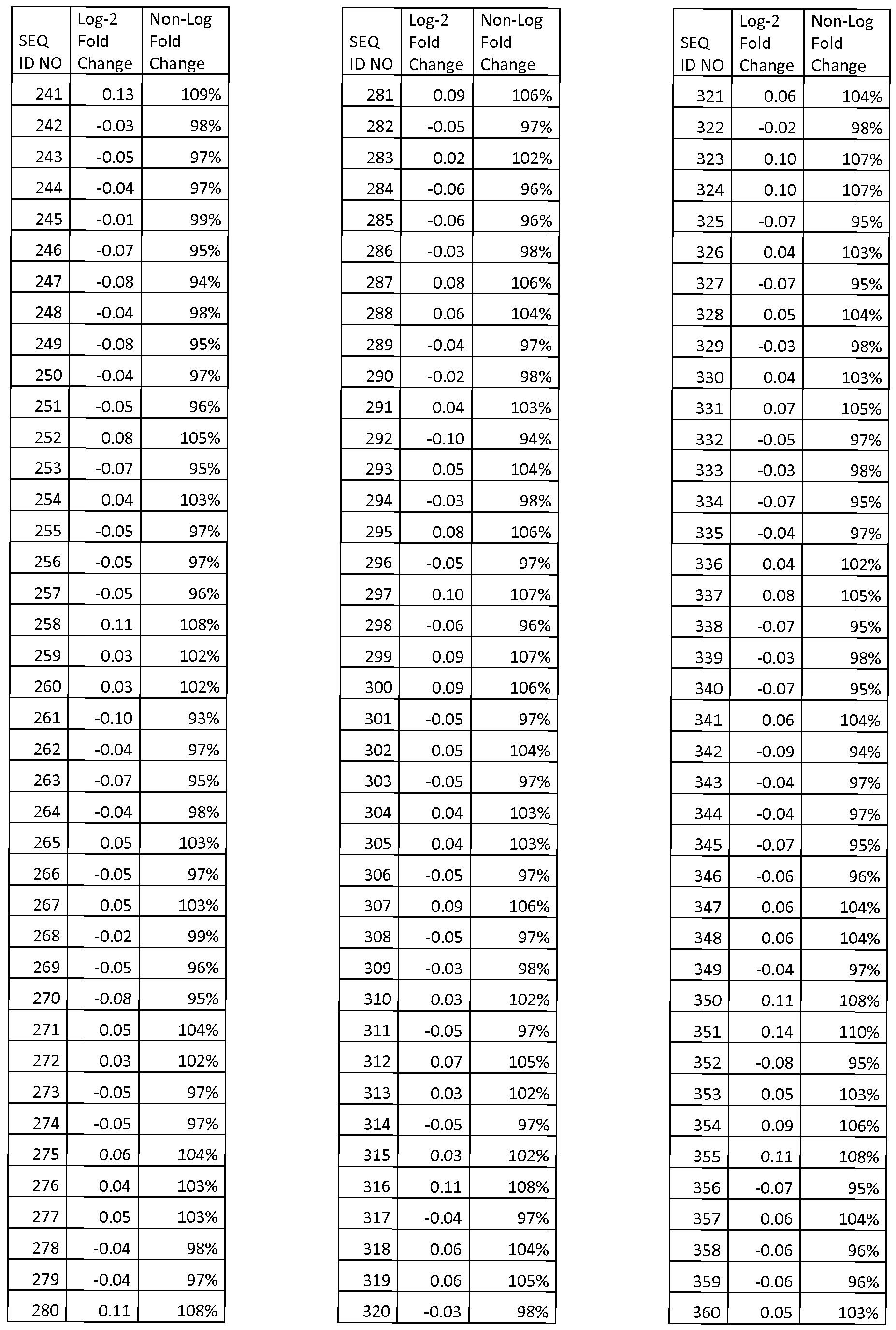

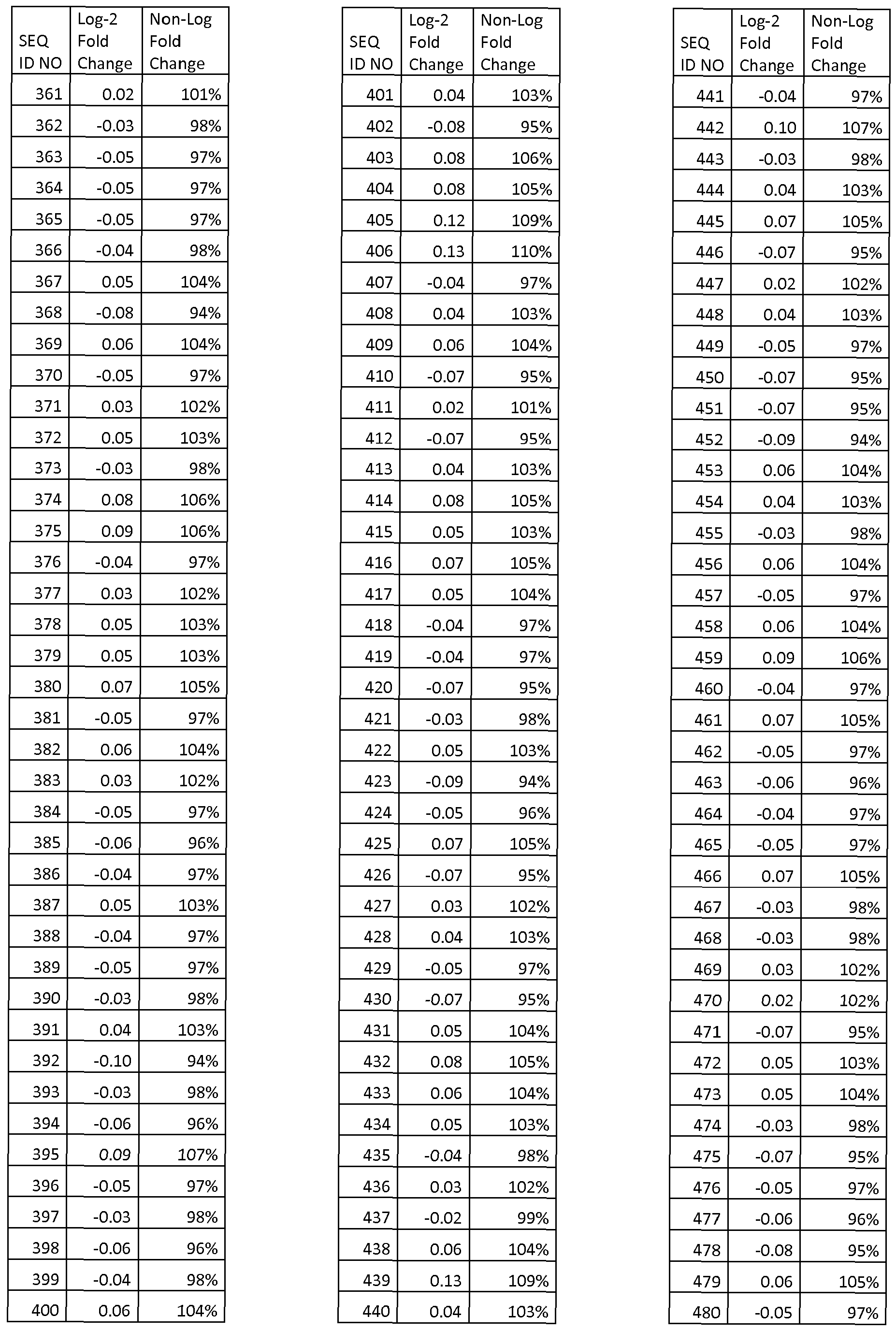

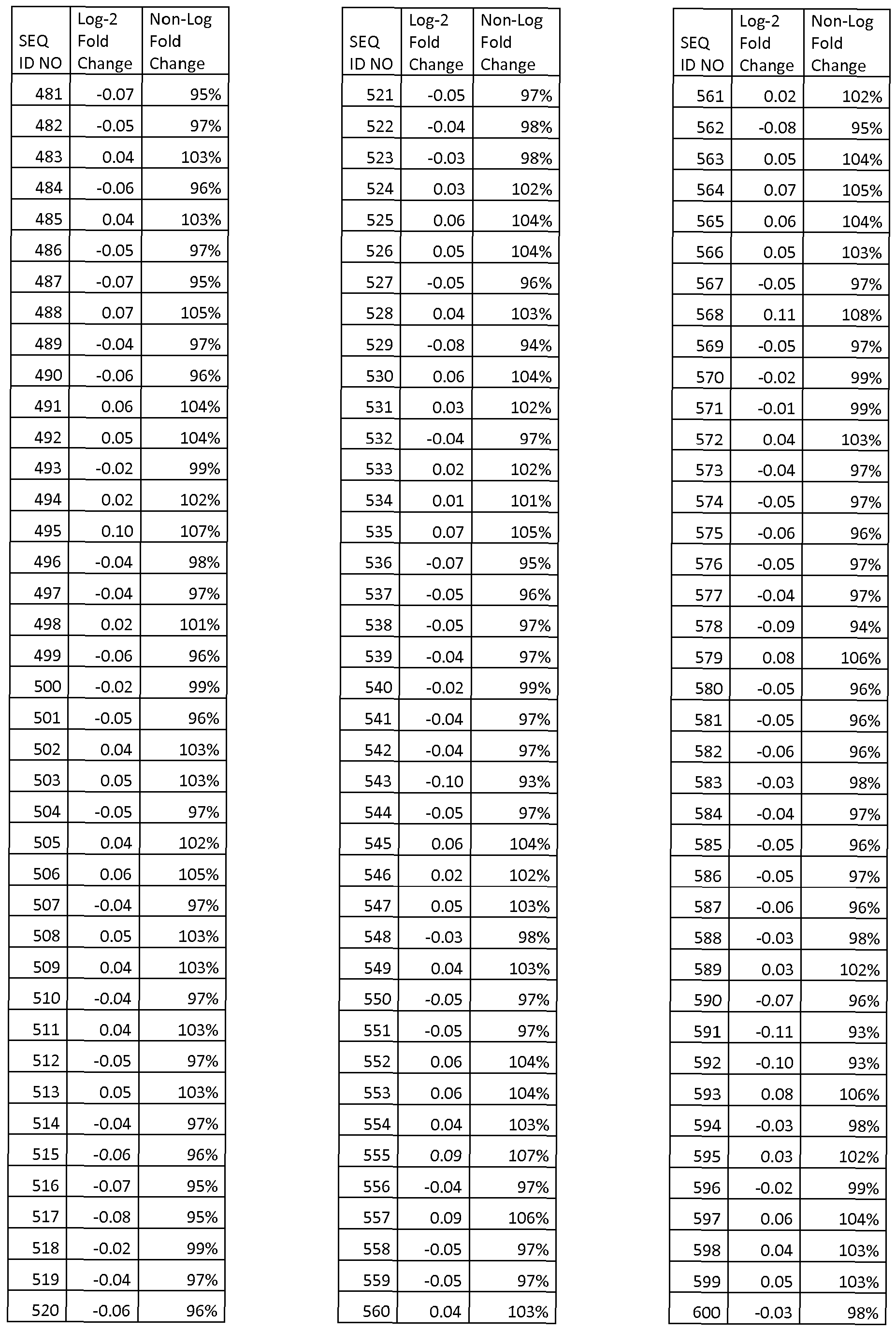

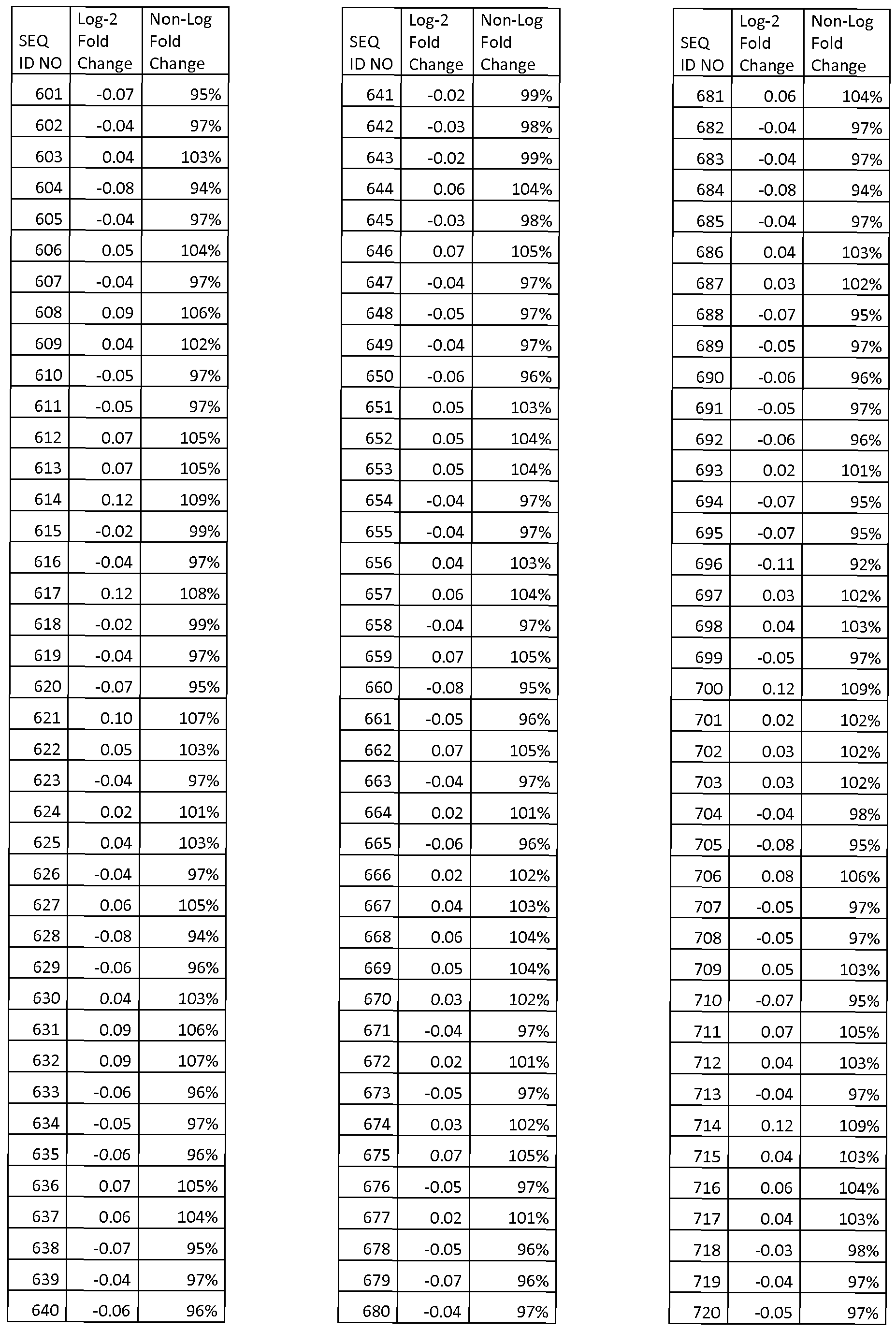

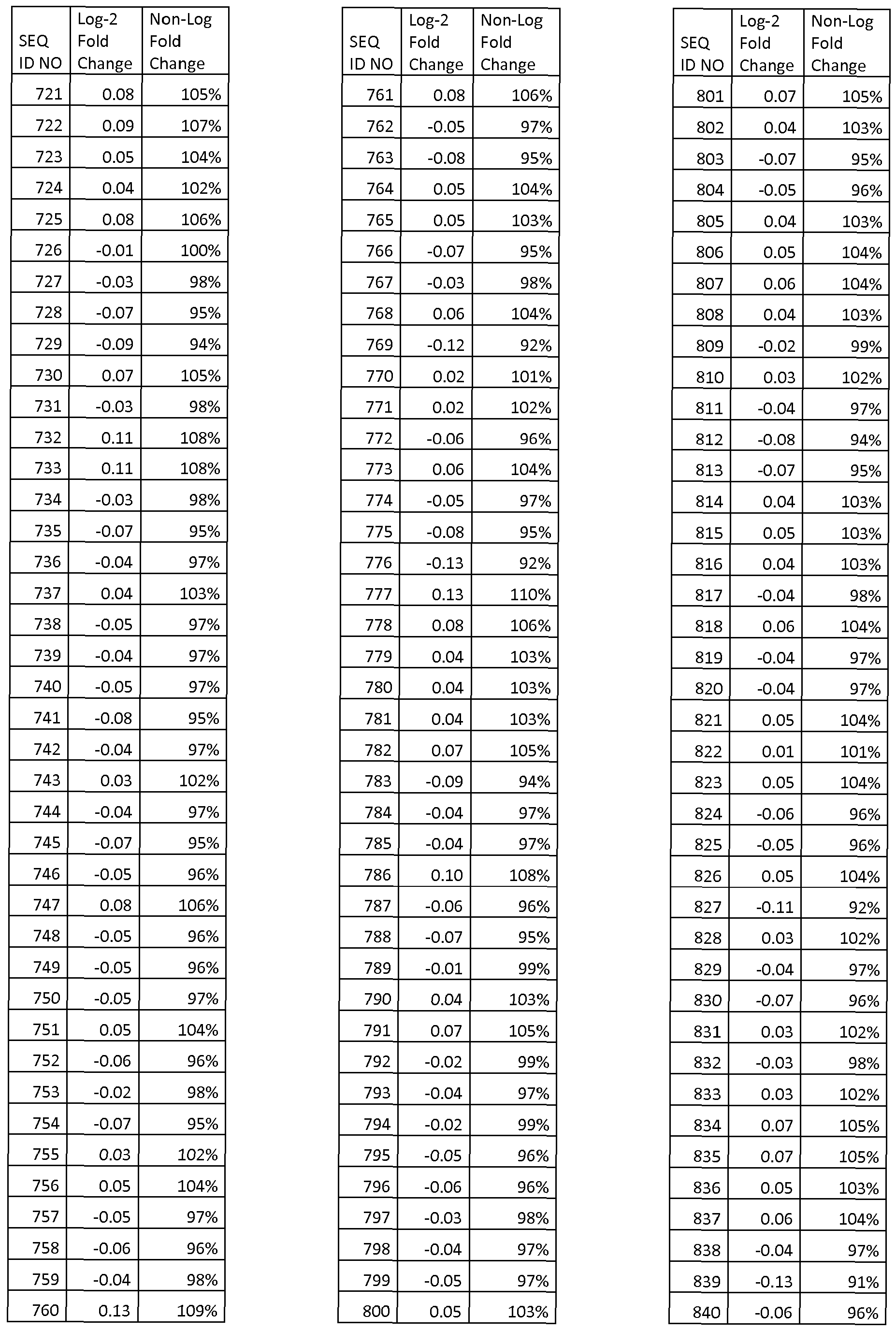

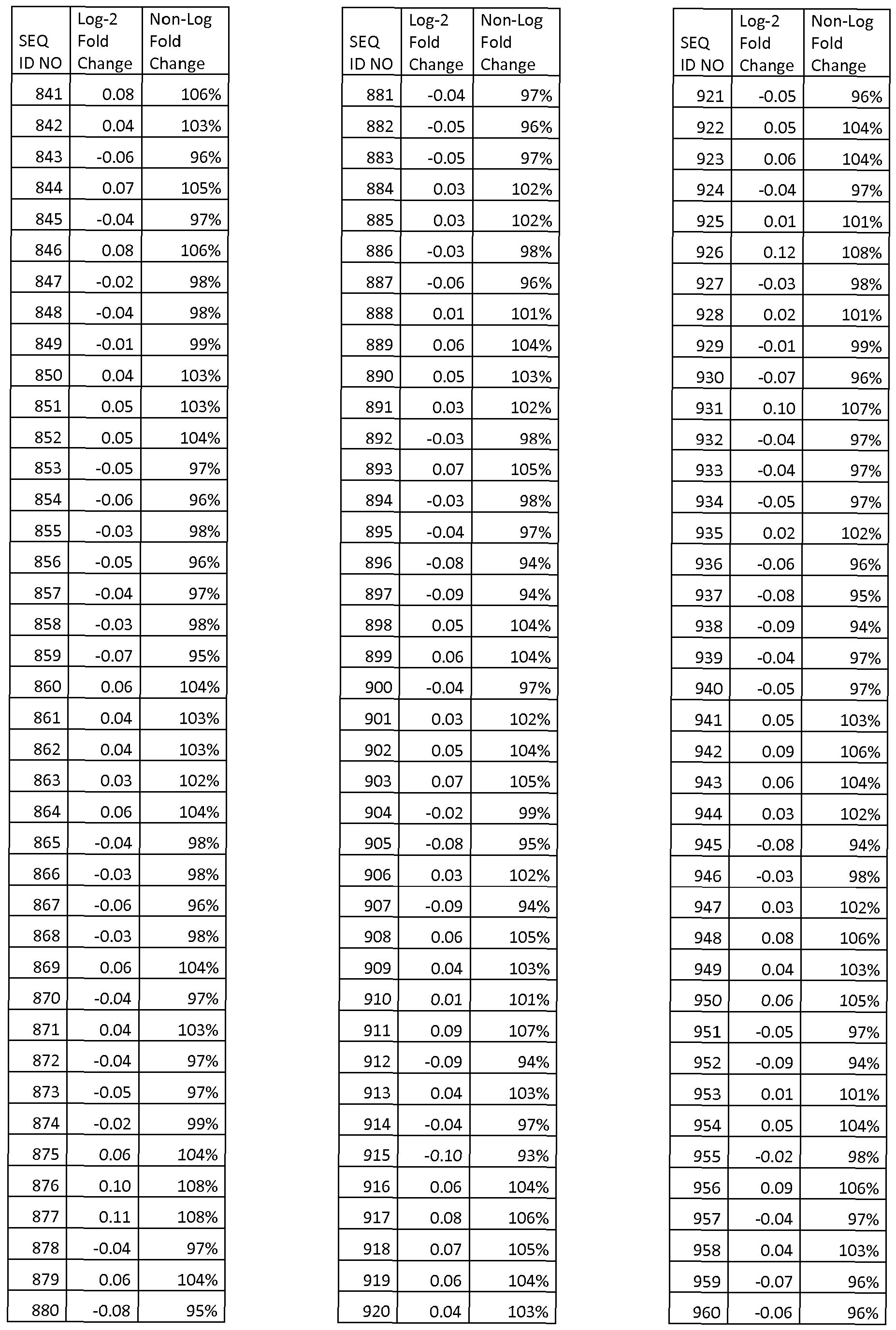

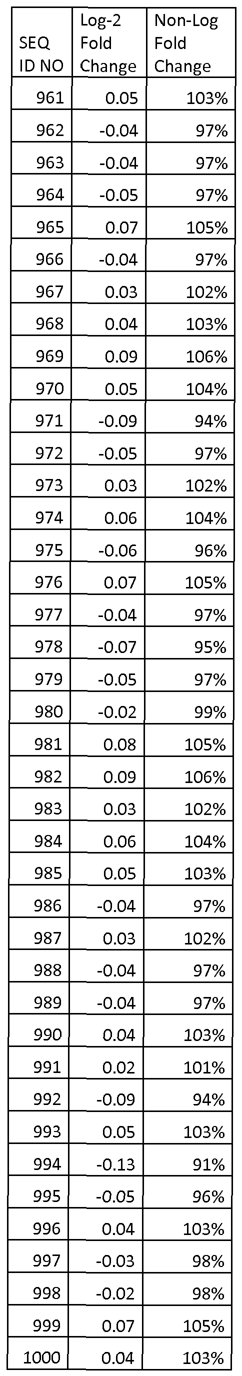

**Table 2**

| SEQ ID Nos for Examples 1-3 of length 250 | | | | SEQ ID Nos for Examples 1-3 of length 250 | | | | SEQ ID Nos for Examples 1-3 of length 250 | | |
|---|---|---|---|---|---|---|---|---|---|---|
| Example 1 | Example 2 | Example 3 | | Example 1 | Example 2 | Example 3 | | Example 1 | Example 2 | Example 3 |
| 1 | 1 | 4 | | 156 | 149 | 149 | | 353 | 313 | 302 |
| 3 | 2 | 5 | | 163 | 153 | 153 | | 356 | 319 | 305 |
| 5 | 8 | 8 | | 164 | 156 | 156 | | 357 | 329 | 306 |
| 10 | 12 | 10 | | 169 | 160 | 158 | | 363 | 330 | 313 |
| 12 | 17 | 12 | | 170 | 164 | 163 | | 365 | 334 | 318 |
| 20 | 26 | 13 | | 176 | 166 | 166 | | 367 | 338 | 319 |
| 25 | 28 | 15 | | 178 | 168 | 168 | | 374 | 350 | 325 |
| 29 | 30 | 17 | | 179 | 170 | 170 | | 375 | 351 | 334 |
| 33 | 34 | 18 | | 181 | 171 | 178 | | 385 | 354 | 337 |
| 35 | 46 | 25 | | 190 | 179 | 179 | | 387 | 357 | 338 |
| 42 | 48 | 26 | | 191 | 188 | 180 | | 390 | 361 | 350 |
| 46 | 52 | 28 | | 192 | 195 | 181 | | 391 | 365 | 351 |
| 51 | 56 | 29 | | 195 | 197 | 188 | | 392 | 371 | 354 |
| 52 | 58 | 30 | | 197 | 199 | 195 | | 394 | 372 | 363 |
| 54 | 60 | 34 | | 202 | 203 | 197 | | 396 | 373 | 365 |
| 59 | 62 | 35 | | 206 | 204 | 202 | | 401 | 375 | 366 |
| 67 | 67 | 46 | | 218 | 209 | 204 | | 403 | 385 | 374 |
| 75 | 70 | 51 | | 233 | 215 | 208 | | 404 | 386 | 375 |
| 78 | 72 | 52 | | 237 | 218 | 209 | | 405 | 387 | 379 |
| 80 | 78 | 59 | | 253 | 220 | 214 | | 406 | 391 | 385 |
| 81 | 80 | 62 | | 259 | 227 | 215 | | 409 | 392 | 390 |
| 87 | 81 | 67 | | 265 | 233 | 216 | | 410 | 394 | 391 |
| 92 | 82 | 78 | | 266 | 235 | 219 | | 415 | 400 | 392 |
| 93 | 85 | 80 | | 270 | 240 | 224 | | 420 | 401 | 394 |
| 104 | 88 | 81 | | 280 | 254 | 225 | | 422 | 403 | 401 |
| 107 | 89 | 82 | | 297 | 255 | 227 | | 424 | 404 | 403 |
| 111 | 93 | 87 | | 299 | 259 | 233 | | 426 | 406 | 406 |
| 113 | 102 | 93 | | 302 | 263 | 235 | | 432 | 420 | 420 |
| 117 | 113 | 97 | | 305 | 265 | 246 | | 438 | 421 | 424 |
| 119 | 117 | 102 | | 306 | 266 | 259 | | 445 | 422 | 430 |
| 126 | 118 | 113 | | 313 | 267 | 265 | | 449 | 423 | 432 |
| 132 | 119 | 117 | | 314 | 270 | 266 | | 452 | 424 | 433 |
| 133 | 122 | 119 | | 318 | 280 | 270 | | 454 | 426 | 442 |
| 136 | 126 | 126 | | 319 | 287 | 275 | | 461 | 427 | 445 |
| 137 | 128 | 127 | | 325 | 297 | 280 | | 463 | 432 | 448 |
| 140 | 131 | 136 | | 329 | 298 | 287 | | 466 | 447 | 449 |
| 142 | 134 | 137 | | 330 | 302 | 288 | | 467 | 449 | 452 |
| 145 | 136 | 141 | | 334 | 305 | 297 | | 469 | 467 | 456 |
| 149 | 137 | 143 | | 338 | 306 | 298 | | 473 | 469 | 458 |
| 153 | 138 | 145 | | 352 | 311 | 299 | | 479 | 473 | 461 |
| 481 | 477 | 468 | | 608 | 628 | 608 | | 806 | 791 | 807 |
| 484 | 484 | 469 | | 609 | 630 | 628 | | 807 | 799 | 810 |
| 485 | 486 | 477 | | 614 | 634 | 630 | | 810 | 801 | 813 |
| 488 | 499 | 483 | | 627 | 641 | 632 | | 813 | 804 | 814 |
| 489 | 511 | 484 | | 634 | 647 | 634 | | 815 | 806 | 816 |
| 499 | 512 | 488 | | 637 | 650 | 638 | | 816 | 807 | 823 |
| 501 | 515 | 499 | | 638 | 653 | 644 | | 823 | 810 | 827 |
| 508 | 516 | 501 | | 640 | 656 | 652 | | 825 | 811 | 830 |
| 510 | 517 | 505 | | 644 | 660 | 660 | | 826 | 813 | 834 |
| 515 | 522 | 512 | | 652 | 666 | 666 | | 830 | 816 | 839 |
| 516 | 526 | 515 | | 656 | 667 | 667 | | 834 | 824 | 841 |
| 518 | 527 | 516 | | 660 | 668 | 668 | | 839 | 827 | 846 |
| 526 | 528 | 522 | | 665 | 675 | 676 | | 841 | 830 | 850 |
| 530 | 529 | 526 | | 667 | 676 | 679 | | 846 | 839 | 856 |
| 544 | 541 | 527 | | 668 | 678 | 681 | | 847 | 844 | 859 |
| 546 | 542 | 529 | | 675 | 682 | 689 | | 849 | 845 | 861 |
| 547 | 544 | 536 | | 678 | 689 | 690 | | 850 | 861 | 862 |
| 549 | 546 | 537 | | 679 | 690 | 699 | | 851 | 862 | 864 |
| 550 | 547 | 538 | | 681 | 692 | 704 | | 859 | 867 | 871 |
| 554 | 549 | 550 | | 682 | 694 | 706 | | 862 | 871 | 874 |
| 555 | 554 | 552 | | 684 | 699 | 707 | | 863 | 874 | 876 |
| 557 | 558 | 554 | | 689 | 707 | 711 | | 871 | 877 | 877 |
| 559 | 559 | 555 | | 699 | 724 | 732 | | 874 | 881 | 883 |
| 560 | 562 | 557 | | 704 | 730 | 733 | | 876 | 886 | 887 |
| 561 | 564 | 559 | | 707 | 732 | 739 | | 877 | 889 | 889 |
| 563 | 568 | 560 | | 719 | 733 | 743 | | 883 | 893 | 890 |
| 570 | 574 | 564 | | 722 | 743 | 745 | | 886 | 897 | 893 |
| 571 | 575 | 565 | | 732 | 744 | 749 | | 888 | 902 | 897 |
| 575 | 576 | 570 | | 739 | 749 | 750 | | 889 | 907 | 902 |
| 576 | 585 | 571 | | 741 | 751 | 754 | | 893 | 908 | 911 |
| 580 | 592 | 572 | | 744 | 756 | 762 | | 894 | 911 | 915 |
| 582 | 593 | 574 | | 745 | 760 | 764 | | 897 | 915 | 917 |
| 585 | 601 | 575 | | 748 | 762 | 765 | | 906 | 917 | 921 |
| 588 | 602 | 576 | | 749 | 764 | 769 | | 931 | 921 | 926 |
| 590 | 608 | 582 | | 772 | 765 | 772 | | 936 | 931 | 928 |
| 592 | 611 | 584 | | 774 | 772 | 775 | | 937 | 936 | 931 |
| 593 | 614 | 592 | | 775 | 774 | 778 | | 940 | 940 | 936 |
| 599 | 620 | 593 | | 778 | 775 | 779 | | 947 | 945 | 937 |
| 600 | 623 | 601 | | 788 | 779 | 783 | | 948 | 954 | 940 |
| 602 | 625 | 602 | | 798 | 788 | 796 | | 950 | 956 | 947 |
| 954 | 958 | 948 | | 976 | 972 | 982 | | 990 | 995 | 993 |
| 956 | 969 | 957 | | 981 | 982 | 985 | | 994 | 996 | 996 |
| 963 | 971 | 958 | | 982 | 989 | 990 | | 996 | 998 | 999 |

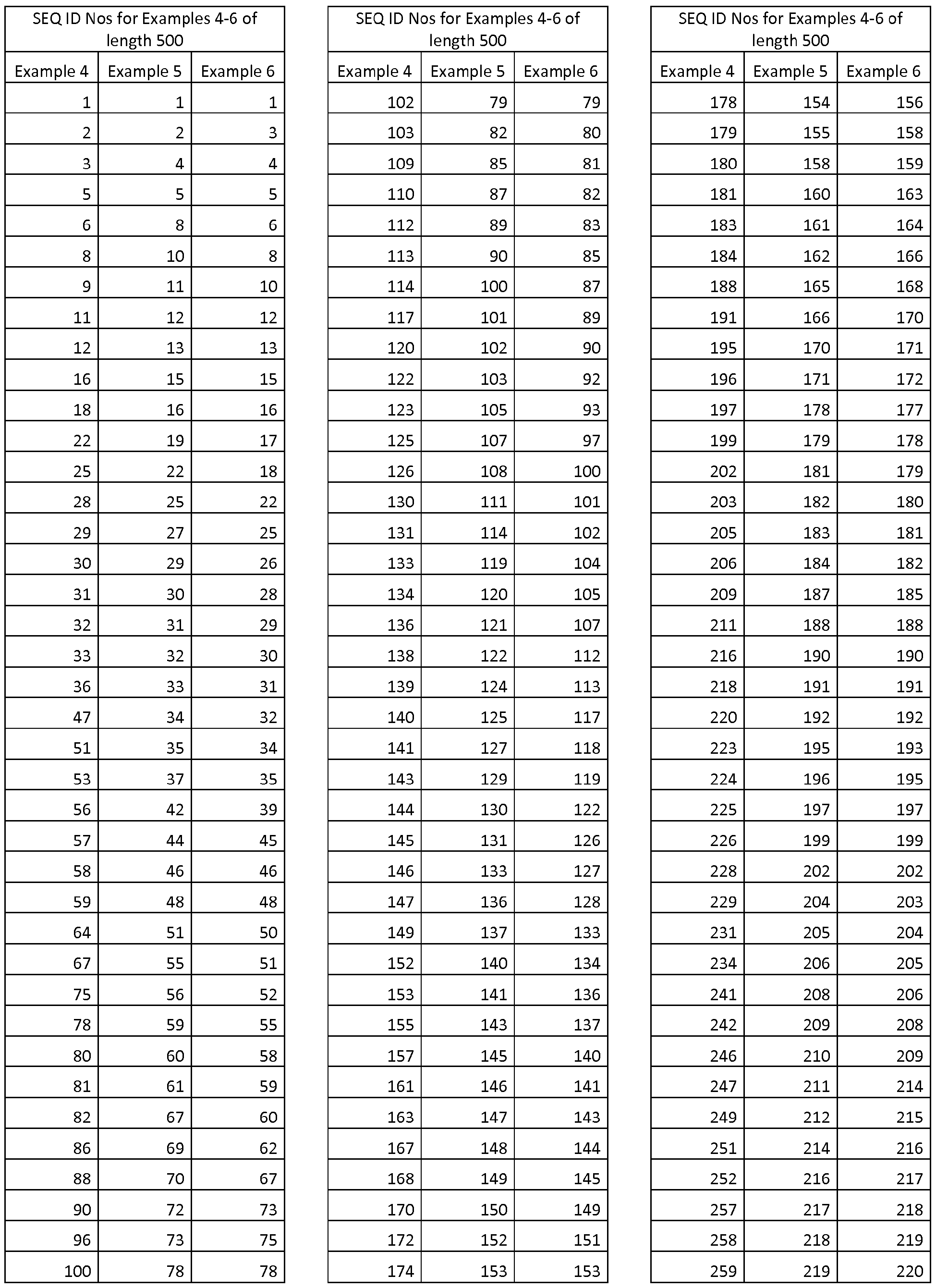

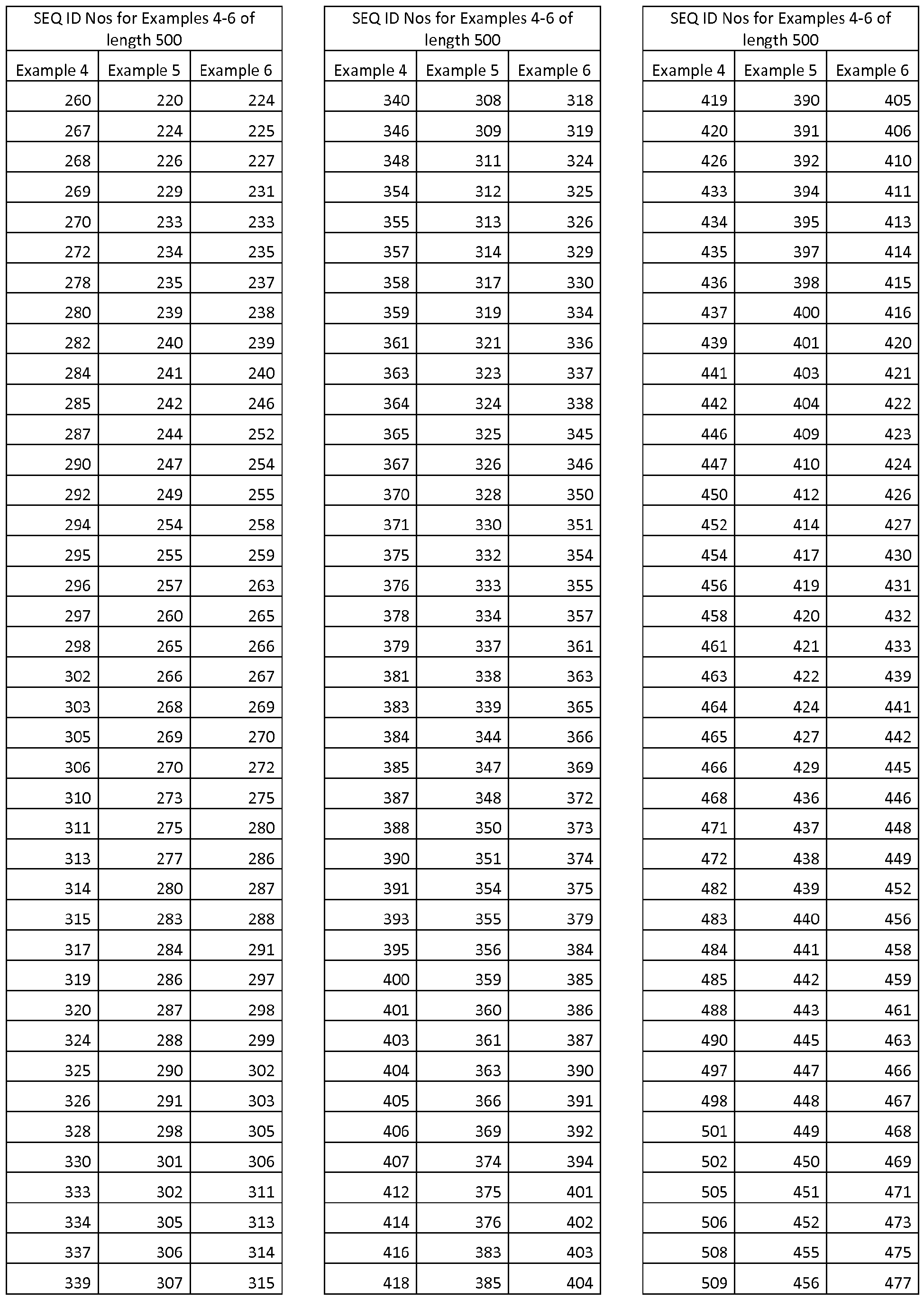

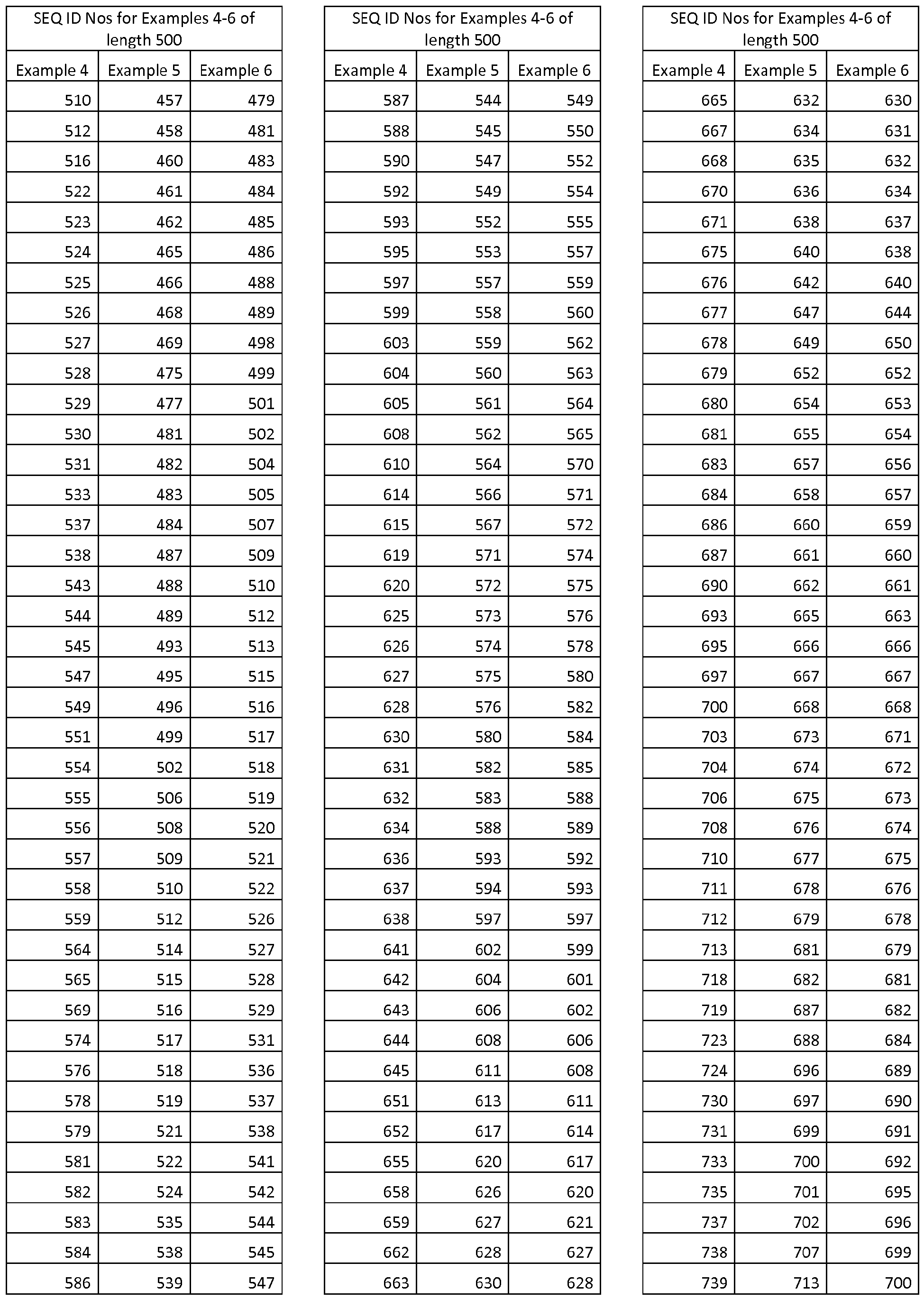

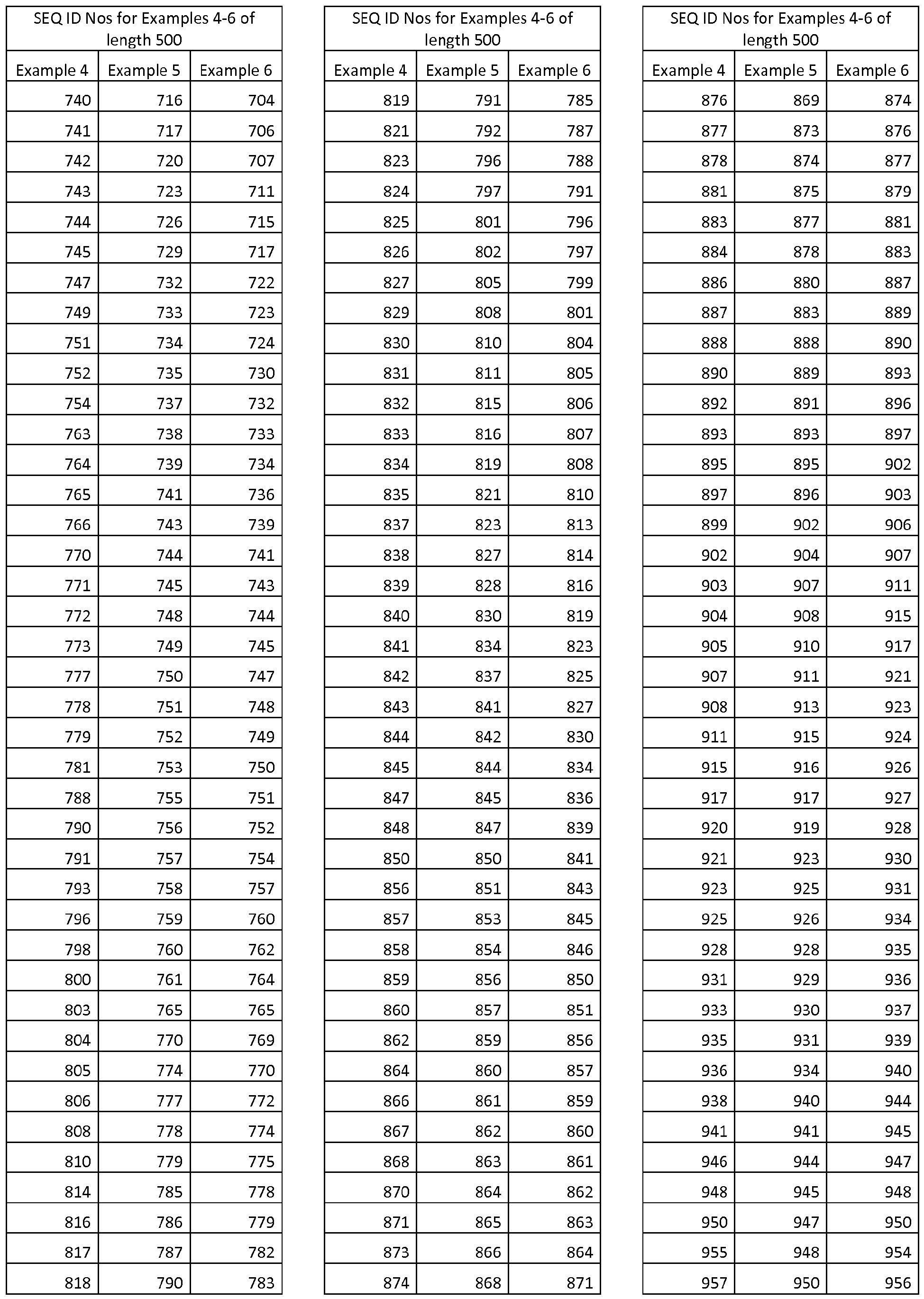

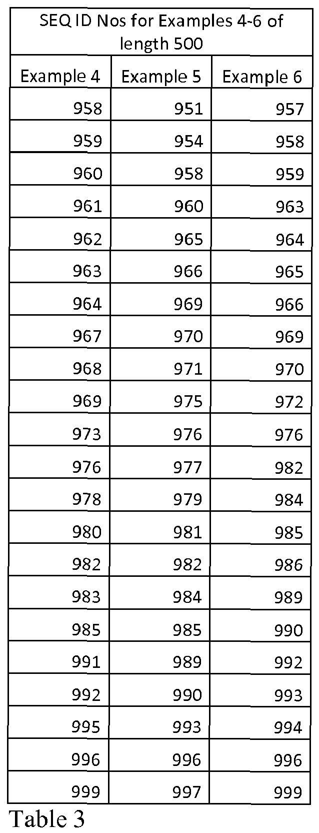

| SEQ ID Nos for Examples 7-9 of length 800 | | | | SEQ ID Nos for Examples 7-9 of length 800 | | | | SEQ ID Nos for Examples 7-9 of length 800 | | |
|---|---|---|---|---|---|---|---|---|---|---|
| Example 7 | Example 8 | Example 9 | | Example 7 | Example 8 | Example 9 | | Example 7 | Example 8 | Example 9 |
| 1 | 1 | 1 | | 48 | 46 | 45 | | 101 | 93 | 92 |
| 2 | 2 | 2 | | 49 | 47 | 46 | | 102 | 94 | 93 |
| 4 | 4 | 3 | | 50 | 48 | 48 | | 103 | 95 | 94 |
| 5 | 5 | 4 | | 51 | 49 | 49 | | 104 | 98 | 96 |
| 6 | 6 | 5 | | 52 | 50 | 50 | | 105 | 100 | 97 |
| 7 | 8 | 6 | | 53 | 51 | 51 | | 107 | 102 | 98 |
| 8 | 9 | 8 | | 54 | 52 | 52 | | 108 | 103 | 99 |
| 9 | 10 | 9 | | 55 | 53 | 53 | | 110 | 105 | 100 |
| 10 | 11 | 10 | | 57 | 54 | 54 | | 111 | 107 | 101 |
| 11 | 13 | 11 | | 58 | 55 | 55 | | 112 | 108 | 102 |
| 12 | 14 | 12 | | 59 | 58 | 56 | | 113 | 109 | 104 |
| 13 | 15 | 13 | | 62 | 59 | 58 | | 114 | 110 | 105 |
| 14 | 16 | 14 | | 63 | 61 | 59 | | 115 | 111 | 107 |
| 15 | 17 | 15 | | 65 | 62 | 60 | | 116 | 112 | 111 |
| 17 | 18 | 16 | | 66 | 63 | 62 | | 117 | 113 | 112 |
| 18 | 19 | 17 | | 67 | 64 | 63 | | 118 | 114 | 113 |
| 20 | 20 | 18 | | 71 | 66 | 65 | | 119 | 115 | 116 |
| 21 | 21 | 20 | | 72 | 68 | 67 | | 121 | 117 | 117 |
| 22 | 22 | 21 | | 73 | 69 | 68 | | 122 | 118 | 118 |
| 24 | 23 | 22 | | 74 | 70 | 69 | | 123 | 119 | 119 |
| 25 | 24 | 23 | | 76 | 71 | 70 | | 125 | 121 | 121 |
| 26 | 25 | 25 | | 78 | 72 | 72 | | 126 | 123 | 122 |
| 27 | 26 | 26 | | 79 | 74 | 73 | | 127 | 125 | 126 |
| 28 | 27 | 27 | | 80 | 75 | 74 | | 128 | 127 | 127 |
| 29 | 28 | 28 | | 81 | 76 | 75 | | 129 | 128 | 128 |
| 30 | 29 | 29 | | 85 | 77 | 77 | | 130 | 130 | 130 |
| 31 | 30 | 30 | | 87 | 78 | 78 | | 131 | 131 | 131 |
| 33 | 31 | 31 | | 88 | 79 | 79 | | 132 | 132 | 132 |
| 34 | 32 | 32 | | 89 | 80 | 80 | | 133 | 133 | 133 |
| 35 | 33 | 33 | | 90 | 81 | 81 | | 134 | 136 | 134 |
| 36 | 34 | 34 | | 91 | 84 | 82 | | 135 | 137 | 136 |
| 38 | 35 | 35 | | 93 | 85 | 83 | | 137 | 140 | 137 |
| 39 | 36 | 37 | | 94 | 86 | 85 | | 139 | 141 | 138 |
| 40 | 38 | 38 | | 95 | 87 | 86 | | 140 | 142 | 140 |
| 42 | 39 | 39 | | 96 | 88 | 87 | | 141 | 143 | 141 |
| 44 | 41 | 41 | | 97 | 89 | 88 | | 142 | 144 | 142 |
| 45 | 43 | 42 | | 98 | 90 | 89 | | 143 | 145 | 143 |
| 46 | 44 | 43 | | 99 | 91 | 90 | | 144 | 147 | 144 |
| 47 | 45 | 44 | | 100 | 92 | 91 | | 145 | 148 | 145 |

| SEQ ID Nos for Examples 7-9 of length 800 | | | | SEQ ID Nos for Examples 7-9 of length 800 | | | | SEQ ID Nos for Examples 7-9 of length 800 | | |
|---|---|---|---|---|---|---|---|---|---|---|
| Example 7 | Example 8 | Example 9 | | Example 7 | Example 8 | Example 9 | | Example 7 | Example 8 | Example 9 |
| 146 | 150 | 146 | | 192 | 195 | 197 | | 241 | 248 | 252 |
| 147 | 151 | 147 | | 193 | 196 | 198 | | 243 | 249 | 253 |
| 148 | 153 | 148 | | 194 | 197 | 199 | | 244 | 250 | 254 |
| 149 | 154 | 149 | | 195 | 198 | 202 | | 245 | 252 | 255 |
| 151 | 156 | 151 | | 196 | 201 | 203 | | 247 | 253 | 257 |
| 152 | 157 | 152 | | 197 | 202 | 204 | | 248 | 254 | 258 |
| 153 | 158 | 153 | | 199 | 203 | 205 | | 250 | 255 | 259 |
| 154 | 159 | 154 | | 200 | 204 | 206 | | 251 | 256 | 261 |
| 155 | 160 | 156 | | 201 | 205 | 208 | | 252 | 257 | 263 |
| 156 | 161 | 158 | | 202 | 206 | 209 | | 253 | 258 | 264 |
| 158 | 162 | 159 | | 203 | 207 | 210 | | 254 | 259 | 265 |
| 159 | 163 | 160 | | 204 | 208 | 211 | | 255 | 261 | 266 |
| 161 | 164 | 163 | | 206 | 209 | 214 | | 256 | 263 | 267 |
| 162 | 165 | 164 | | 207 | 213 | 215 | | 257 | 264 | 268 |
| 163 | 166 | 165 | | 208 | 214 | 216 | | 258 | 265 | 269 |
| 164 | 167 | 166 | | 209 | 215 | 217 | | 259 | 266 | 270 |
| 165 | 168 | 168 | | 210 | 216 | 218 | | 260 | 267 | 272 |
| 166 | 169 | 169 | | 211 | 217 | 219 | | 262 | 268 | 274 |
| 167 | 170 | 170 | | 214 | 218 | 220 | | 263 | 269 | 275 |
| 168 | 171 | 171 | | 215 | 219 | 221 | | 264 | 270 | 277 |
| 169 | 172 | 172 | | 216 | 221 | 223 | | 265 | 271 | 279 |
| 170 | 173 | 176 | | 217 | 224 | 224 | | 266 | 272 | 280 |
| 172 | 174 | 177 | | 218 | 225 | 225 | | 267 | 274 | 282 |
| 173 | 175 | 178 | | 219 | 226 | 227 | | 268 | 275 | 283 |
| 174 | 176 | 179 | | 220 | 227 | 228 | | 271 | 276 | 285 |
| 175 | 177 | 180 | | 221 | 228 | 230 | | 272 | 277 | 286 |
| 177 | 178 | 181 | | 223 | 229 | 231 | | 273 | 279 | 287 |
| 178 | 179 | 182 | | 225 | 231 | 232 | | 274 | 280 | 288 |
| 179 | 180 | 183 | | 226 | 232 | 233 | | 275 | 281 | 291 |
| 180 | 181 | 184 | | 227 | 233 | 235 | | 277 | 282 | 292 |
| 182 | 182 | 185 | | 228 | 235 | 236 | | 278 | 283 | 294 |
| 183 | 183 | 187 | | 229 | 236 | 237 | | 279 | 284 | 296 |
| 184 | 184 | 188 | | 230 | 237 | 238 | | 280 | 285 | 297 |
| 185 | 185 | 189 | | 231 | 238 | 239 | | 282 | 286 | 298 |
| 186 | 188 | 190 | | 232 | 239 | 240 | | 283 | 287 | 299 |
| 187 | 189 | 191 | | 236 | 241 | 241 | | 285 | 288 | 300 |
| 188 | 190 | 192 | | 237 | 242 | 246 | | 286 | 290 | 301 |
| 189 | 192 | 193 | | 238 | 243 | 248 | | 287 | 291 | 302 |
| 190 | 193 | 195 | | 239 | 244 | 249 | | 289 | 292 | 303 |
| 191 | 194 | 196 | | 240 | 246 | 250 | | 290 | 293 | 305 |

| SEQ ID Nos for Examples 7-9 of length 800 | | | | SEQ ID Nos for Examples 7-9 of length 800 | | | | SEQ ID Nos for Examples 7-9 of length 800 | | |
|---|---|---|---|---|---|---|---|---|---|---|
| Example 7 | Example 8 | Example 9 | | Example 7 | Example 8 | Example 9 | | Example 7 | Example 8 | Example 9 |
| 291 | 295 | 306 | | 343 | 346 | 356 | | 397 | 399 | 402 |
| 292 | 297 | 307 | | 348 | 347 | 357 | | 398 | 400 | 403 |
| 293 | 298 | 310 | | 350 | 348 | 358 | | 399 | 401 | 404 |
| 294 | 299 | 311 | | 351 | 351 | 359 | | 400 | 403 | 405 |
| 296 | 300 | 312 | | 352 | 352 | 360 | | 401 | 404 | 406 |
| 298 | 302 | 313 | | 353 | 353 | 361 | | 402 | 406 | 407 |
| 300 | 304 | 314 | | 354 | 354 | 362 | | 403 | 407 | 408 |
| 301 | 305 | 315 | | 355 | 355 | 363 | | 404 | 409 | 409 |
| 302 | 309 | 318 | | 356 | 358 | 364 | | 405 | 410 | 410 |
| 303 | 310 | 319 | | 357 | 359 | 365 | | 406 | 411 | 411 |
| 304 | 311 | 320 | | 358 | 360 | 366 | | 407 | 412 | 413 |
| 306 | 312 | 321 | | 359 | 361 | 367 | | 410 | 413 | 414 |
| 307 | 314 | 322 | | 361 | 362 | 368 | | 411 | 414 | 415 |
| 308 | 315 | 323 | | 362 | 364 | 369 | | 412 | 415 | 416 |
| 309 | 317 | 324 | | 363 | 365 | 370 | | 413 | 416 | 417 |
| 310 | 318 | 325 | | 364 | 366 | 371 | | 414 | 418 | 420 |
| 311 | 319 | 326 | | 365 | 367 | 372 | | 415 | 419 | 421 |
| 312 | 320 | 327 | | 366 | 368 | 373 | | 416 | 420 | 422 |
| 314 | 321 | 328 | | 367 | 369 | 374 | | 417 | 421 | 423 |
| 315 | 322 | 329 | | 368 | 370 | 375 | | 418 | 422 | 424 |
| 316 | 323 | 330 | | 369 | 371 | 376 | | 419 | 424 | 426 |
| 317 | 324 | 331 | | 371 | 372 | 379 | | 420 | 425 | 427 |
| 319 | 325 | 334 | | 372 | 373 | 380 | | 422 | 426 | 428 |
| 320 | 326 | 336 | | 374 | 374 | 382 | | 423 | 427 | 430 |
| 321 | 327 | 337 | | 375 | 375 | 383 | | 424 | 428 | 431 |
| 323 | 328 | 338 | | 377 | 376 | 384 | | 426 | 429 | 432 |
| 325 | 329 | 340 | | 379 | 379 | 385 | | 427 | 430 | 433 |
| 326 | 330 | 341 | | 380 | 380 | 386 | | 428 | 431 | 435 |
| 327 | 332 | 343 | | 381 | 381 | 387 | | 429 | 432 | 436 |
| 328 | 333 | 344 | | 382 | 382 | 388 | | 430 | 433 | 437 |
| 329 | 334 | 345 | | 385 | 385 | 389 | | 431 | 435 | 438 |
| 330 | 335 | 346 | | 387 | 386 | 390 | | 432 | 436 | 439 |
| 331 | 336 | 347 | | 388 | 387 | 391 | | 433 | 437 | 441 |
| 332 | 337 | 349 | | 389 | 389 | 392 | | 434 | 438 | 442 |
| 335 | 338 | 350 | | 390 | 390 | 393 | | 435 | 439 | 444 |
| 338 | 339 | 351 | | 391 | 391 | 394 | | 437 | 441 | 445 |
| 339 | 340 | 352 | | 392 | 392 | 396 | | 438 | 442 | 446 |
| 340 | 341 | 353 | | 394 | 394 | 399 | | 440 | 443 | 447 |
| 341 | 344 | 354 | | 395 | 395 | 400 | | 442 | 445 | 448 |
| 342 | 345 | 355 | | 396 | 396 | 401 | | 443 | 446 | 449 |

| SEQ ID Nos for Examples 7-9 of length 800 | | | | SEQ ID Nos for Examples 7-9 of length 800 | | | | SEQ ID Nos for Examples 7-9 of length 800 | | |
|---|---|---|---|---|---|---|---|---|---|---|
| Example 7 | Example 8 | Example 9 | | Example 7 | Example 8 | Example 9 | | Example 7 | Example 8 | Example 9 |
| 445 | 448 | 450 | | 493 | 499 | 499 | | 543 | 552 | 546 |
| 446 | 449 | 452 | | 494 | 500 | 501 | | 545 | 553 | 547 |
| 447 | 450 | 453 | | 495 | 501 | 502 | | 546 | 554 | 549 |
| 448 | 451 | 454 | | 496 | 502 | 503 | | 547 | 557 | 550 |
| 449 | 452 | 456 | | 499 | 503 | 504 | | 549 | 558 | 551 |
| 450 | 455 | 457 | | 500 | 504 | 505 | | 550 | 559 | 552 |
| 451 | 456 | 458 | | 501 | 505 | 506 | | 551 | 560 | 553 |
| 452 | 457 | 459 | | 502 | 508 | 507 | | 552 | 561 | 554 |
| 453 | 458 | 460 | | 504 | 510 | 508 | | 553 | 562 | 555 |
| 454 | 461 | 461 | | 505 | 511 | 509 | | 554 | 563 | 557 |
| 456 | 462 | 462 | | 507 | 512 | 510 | | 555 | 564 | 558 |
| 457 | 464 | 463 | | 508 | 513 | 511 | | 556 | 565 | 559 |
| 458 | 466 | 464 | | 509 | 514 | 512 | | 557 | 566 | 560 |
| 459 | 467 | 466 | | 510 | 515 | 513 | | 559 | 568 | 561 |
| 461 | 468 | 467 | | 511 | 516 | 515 | | 560 | 569 | 562 |
| 462 | 469 | 468 | | 513 | 517 | 516 | | 561 | 570 | 563 |
| 463 | 470 | 469 | | 514 | 518 | 517 | | 562 | 571 | 564 |
| 465 | 472 | 470 | | 515 | 519 | 518 | | 564 | 572 | 565 |
| 466 | 473 | 471 | | 516 | 520 | 519 | | 566 | 574 | 567 |
| 468 | 474 | 472 | | 517 | 522 | 520 | | 568 | 575 | 568 |
| 469 | 475 | 473 | | 519 | 524 | 521 | | 569 | 576 | 570 |
| 470 | 476 | 474 | | 520 | 525 | 522 | | 570 | 577 | 571 |
| 471 | 477 | 475 | | 521 | 526 | 525 | | 571 | 578 | 572 |
| 472 | 479 | 476 | | 522 | 527 | 526 | | 572 | 579 | 574 |
| 473 | 480 | 477 | | 524 | 528 | 527 | | 573 | 580 | 575 |
| 475 | 481 | 478 | | 525 | 529 | 528 | | 574 | 581 | 576 |
| 476 | 482 | 479 | | 526 | 530 | 529 | | 575 | 582 | 577 |
| 477 | 483 | 481 | | 527 | 534 | 530 | | 576 | 583 | 578 |
| 478 | 484 | 483 | | 528 | 536 | 531 | | 577 | 584 | 580 |
| 479 | 486 | 484 | | 529 | 537 | 533 | | 578 | 585 | 582 |
| 480 | 488 | 485 | | 530 | 538 | 536 | | 582 | 586 | 584 |
| 481 | 489 | 486 | | 532 | 539 | 537 | | 584 | 588 | 585 |
| 482 | 490 | 487 | | 533 | 540 | 538 | | 585 | 590 | 586 |
| 483 | 491 | 488 | | 536 | 542 | 539 | | 586 | 591 | 587 |
| 484 | 492 | 489 | | 537 | 544 | 540 | | 587 | 592 | 588 |
| 485 | 493 | 491 | | 538 | 545 | 541 | | 588 | 593 | 589 |
| 488 | 494 | 492 | | 539 | 546 | 542 | | 589 | 594 | 590 |
| 489 | 495 | 493 | | 540 | 547 | 543 | | 590 | 596 | 591 |
| 490 | 497 | 495 | | 541 | 549 | 544 | | 591 | 597 | 592 |
| 492 | 498 | 498 | | 542 | 551 | 545 | | 592 | 598 | 593 |

| SEQ ID Nos for Examples 7-9 of length 800 | | | | SEQ ID Nos for Examples 7-9 of length 800 | | | | SEQ ID Nos for Examples 7-9 of length 800 | | |
|---|---|---|---|---|---|---|---|---|---|---|
| Example 7 | Example 8 | Example 9 | | Example 7 | Example 8 | Example 9 | | Example 7 | Example 8 | Example 9 |
| 593 | 599 | 595 | | 644 | 654 | 647 | | 699 | 707 | 692 |
| 594 | 601 | 597 | | 645 | 655 | 649 | | 701 | 708 | 694 |
| 595 | 602 | 599 | | 647 | 656 | 650 | | 702 | 709 | 695 |
| 597 | 604 | 600 | | 648 | 658 | 652 | | 704 | 710 | 696 |
| 598 | 606 | 601 | | 649 | 659 | 653 | | 705 | 711 | 699 |
| 599 | 607 | 602 | | 650 | 660 | 654 | | 706 | 712 | 700 |
| 600 | 608 | 603 | | 652 | 661 | 655 | | 707 | 713 | 703 |
| 601 | 610 | 605 | | 656 | 663 | 656 | | 708 | 714 | 704 |
| 602 | 611 | 606 | | 657 | 664 | 657 | | 709 | 715 | 706 |
| 603 | 612 | 607 | | 658 | 665 | 659 | | 710 | 717 | 707 |
| 604 | 613 | 608 | | 660 | 666 | 660 | | 711 | 718 | 708 |
| 605 | 614 | 609 | | 661 | 667 | 661 | | 712 | 719 | 709 |
| 606 | 617 | 610 | | 663 | 668 | 662 | | 713 | 720 | 710 |
| 608 | 618 | 611 | | 664 | 671 | 663 | | 714 | 721 | 711 |
| 610 | 620 | 612 | | 665 | 672 | 664 | | 715 | 722 | 712 |
| 611 | 623 | 613 | | 666 | 673 | 665 | | 716 | 724 | 714 |
| 612 | 624 | 614 | | 667 | 674 | 666 | | 717 | 727 | 715 |
| 613 | 625 | 615 | | 668 | 675 | 667 | | 718 | 728 | 717 |
| 614 | 627 | 617 | | 669 | 676 | 668 | | 719 | 729 | 719 |
| 616 | 628 | 619 | | 670 | 677 | 670 | | 720 | 730 | 720 |
| 617 | 629 | 620 | | 671 | 678 | 671 | | 721 | 731 | 721 |
| 618 | 630 | 621 | | 672 | 679 | 672 | | 722 | 732 | 722 |
| 619 | 631 | 623 | | 673 | 682 | 673 | | 724 | 733 | 723 |
| 620 | 632 | 625 | | 675 | 683 | 674 | | 725 | 734 | 724 |
| 621 | 633 | 627 | | 676 | 684 | 675 | | 726 | 735 | 725 |
| 623 | 634 | 628 | | 677 | 685 | 676 | | 727 | 737 | 726 |
| 624 | 635 | 630 | | 678 | 686 | 677 | | 728 | 738 | 729 |
| 626 | 636 | 631 | | 679 | 688 | 678 | | 729 | 740 | 730 |
| 627 | 637 | 632 | | 680 | 690 | 679 | | 730 | 742 | 732 |
| 628 | 638 | 633 | | 681 | 691 | 680 | | 731 | 744 | 733 |
| 629 | 641 | 634 | | 683 | 693 | 681 | | 732 | 745 | 734 |
| 630 | 642 | 635 | | 684 | 694 | 682 | | 733 | 748 | 735 |
| 631 | 644 | 637 | | 687 | 696 | 683 | | 735 | 749 | 736 |
| 633 | 646 | 638 | | 688 | 698 | 684 | | 737 | 750 | 737 |
| 634 | 647 | 639 | | 689 | 699 | 685 | | 738 | 751 | 738 |
| 636 | 648 | 640 | | 690 | 700 | 686 | | 739 | 752 | 739 |
| 637 | 649 | 641 | | 693 | 701 | 688 | | 740 | 753 | 740 |
| 638 | 650 | 643 | | 695 | 703 | 689 | | 741 | 754 | 741 |
| 641 | 652 | 644 | | 697 | 705 | 690 | | 742 | 755 | 742 |
| 643 | 653 | 645 | | 698 | 706 | 691 | | 743 | 756 | 743 |

| SEQ ID Nos for Examples 7-9 of length 800 | | | | SEQ ID Nos for Examples 7-9 of length 800 | | | | SEQ ID Nos for Examples 7-9 of length 800 | | |
|---|---|---|---|---|---|---|---|---|---|---|
| Example 7 | Example 8 | Example 9 | | Example 7 | Example 8 | Example 9 | | Example 7 | Example 8 | Example 9 |
| 744 | 757 | 744 | | 793 | 806 | 792 | | 846 | 853 | 842 |
| 745 | 759 | 745 | | 795 | 807 | 796 | | 847 | 855 | 843 |
| 746 | 760 | 746 | | 796 | 808 | 797 | | 848 | 856 | 844 |
| 747 | 761 | 747 | | 797 | 809 | 798 | | 849 | 857 | 845 |
| 748 | 762 | 748 | | 798 | 810 | 799 | | 850 | 859 | 846 |
| 749 | 763 | 749 | | 799 | 811 | 801 | | 853 | 860 | 847 |
| 750 | 764 | 750 | | 801 | 812 | 804 | | 855 | 862 | 848 |
| 751 | 765 | 751 | | 802 | 813 | 805 | | 856 | 863 | 849 |
| 752 | 769 | 752 | | 803 | 814 | 806 | | 857 | 864 | 850 |
| 756 | 770 | 753 | | 804 | 815 | 807 | | 859 | 865 | 851 |
| 757 | 772 | 754 | | 805 | 816 | 808 | | 860 | 867 | 853 |
| 758 | 773 | 756 | | 806 | 818 | 809 | | 861 | 868 | 856 |
| 759 | 774 | 757 | | 807 | 821 | 810 | | 862 | 869 | 857 |
| 763 | 775 | 760 | | 808 | 823 | 811 | | 863 | 870 | 859 |
| 764 | 776 | 761 | | 810 | 824 | 812 | | 864 | 871 | 860 |
| 765 | 777 | 762 | | 812 | 825 | 813 | | 865 | 872 | 861 |
| 767 | 779 | 763 | | 813 | 826 | 814 | | 866 | 874 | 862 |
| 768 | 781 | 764 | | 815 | 827 | 815 | | 867 | 875 | 863 |
| 769 | 782 | 765 | | 817 | 828 | 816 | | 868 | 876 | 864 |
| 770 | 783 | 767 | | 818 | 829 | 817 | | 869 | 877 | 867 |
| 771 | 784 | 769 | | 819 | 830 | 819 | | 870 | 880 | 869 |
| 772 | 785 | 770 | | 820 | 832 | 820 | | 871 | 881 | 871 |
| 773 | 786 | 771 | | 821 | 833 | 821 | | 872 | 883 | 873 |
| 774 | 787 | 772 | | 823 | 834 | 823 | | 873 | 885 | 874 |
| 775 | 788 | 773 | | 824 | 835 | 824 | | 874 | 886 | 875 |
| 776 | 789 | 774 | | 825 | 836 | 825 | | 875 | 887 | 876 |
| 777 | 790 | 775 | | 827 | 837 | 826 | | 876 | 888 | 877 |
| 778 | 791 | 776 | | 828 | 838 | 827 | | 877 | 889 | 879 |
| 779 | 792 | 777 | | 829 | 840 | 829 | | 879 | 890 | 880 |
| 781 | 793 | 778 | | 831 | 841 | 830 | | 880 | 891 | 881 |
| 782 | 794 | 779 | | 832 | 842 | 831 | | 881 | 892 | 883 |
| 783 | 795 | 780 | | 833 | 843 | 833 | | 882 | 893 | 885 |
| 784 | 796 | 781 | | 834 | 844 | 834 | | 883 | 895 | 886 |
| 785 | 797 | 782 | | 836 | 846 | 835 | | 884 | 896 | 887 |
| 787 | 798 | 783 | | 837 | 847 | 836 | | 886 | 897 | 888 |
| 788 | 799 | 785 | | 838 | 848 | 837 | | 887 | 898 | 889 |
| 789 | 801 | 786 | | 839 | 849 | 838 | | 888 | 899 | 890 |
| 790 | 802 | 787 | | 840 | 850 | 839 | | 889 | 900 | 891 |
| 791 | 803 | 788 | | 842 | 851 | 840 | | 890 | 901 | 892 |
| 792 | 804 | 791 | | 843 | 852 | 841 | | 894 | 902 | 893 |

**Table 4**

| SEQ ID Nos for Examples 7-9 of length 800 | | | | SEQ ID Nos for Examples 7-9 of length 800 | | | | SEQ ID Nos for Examples 7-9 of length 800 | | |
|---|---|---|---|---|---|---|---|---|---|---|
| Example 7 | Example 8 | Example 9 | | Example 7 | Example 8 | Example 9 | | Example 7 | Example 8 | Example 9 |
| 896 | 904 | 894 | | 946 | 950 | 944 | | 999 | 999 | 998 |
| 897 | 906 | 895 | | 947 | 951 | 945 | | 1000 | 1000 | 999 |
| 899 | 907 | 896 | | 948 | 952 | 946 | | | | |
| 900 | 908 | 897 | | 949 | 954 | 947 | | | | |
| 901 | 909 | 899 | | 950 | 956 | 948 | | | | |
| 902 | 910 | 902 | | 951 | 958 | 949 | | | | |
| 903 | 911 | 903 | | 953 | 959 | 950 | | | | |
| 904 | 912 | 906 | | 954 | 960 | 951 | | | | |
| 906 | 913 | 907 | | 955 | 961 | 952 | | | | |
| 907 | 914 | 908 | | 956 | 962 | 954 | | | | |
| 908 | 915 | 909 | | 957 | 965 | 956 | | | | |
| 909 | 916 | 910 | | 959 | 966 | 957 | | | | |
| 910 | 917 | 911 | | 960 | 967 | 958 | | | | |
| 911 | 918 | 913 | | 961 | 968 | 959 | | | | |
| 912 | 919 | 914 | | 962 | 969 | 961 | | | | |
| 915 | 920 | 915 | | 964 | 970 | 963 | | | | |
| 916 | 921 | 916 | | 965 | 971 | 964 | | | | |
| 917 | 922 | 917 | | 966 | 972 | 965 | | | | |
| 918 | 923 | 918 | | 968 | 973 | 966 | | | | |
| 919 | 924 | 919 | | 969 | 974 | 967 | | | | |
| 920 | 925 | 920 | | 971 | 976 | 969 | | | | |
| 923 | 927 | 921 | | 972 | 977 | 970 | | | | |
| 924 | 928 | 923 | | 973 | 978 | 971 | | | | |
| 925 | 929 | 924 | | 974 | 979 | 972 | | | | |
| 926 | 930 | 925 | | 975 | 980 | 974 | | | | |
| 927 | 931 | 926 | | 976 | 981 | 976 | | | | |
| 928 | 934 | 927 | | 979 | 982 | 978 | | | | |
| 929 | 935 | 928 | | 982 | 983 | 981 | | | | |
| 930 | 936 | 930 | | 983 | 984 | 982 | | | | |
| 932 | 937 | 931 | | 984 | 985 | 984 | | | | |
| 933 | 939 | 933 | | 985 | 987 | 985 | | | | |
| 934 | 940 | 934 | | 986 | 988 | 986 | | | | |
| 935 | 941 | 935 | | 987 | 989 | 989 | | | | |
| 936 | 942 | 936 | | 991 | 991 | 990 | | | | |
| 937 | 943 | 937 | | 992 | 992 | 991 | | | | |
| 939 | 944 | 938 | | 994 | 993 | 992 | | | | |
| 940 | 945 | 939 | | 995 | 994 | 993 | | | | |
| 942 | 946 | 940 | | 996 | 995 | 994 | | | | |
| 943 | 947 | 941 | | 997 | 996 | 995 | | | | |
| 945 | 948 | 942 | | 998 | 997 | 996 | | | | |

## Claims

1. A method for the detection of colorectal cancer in a human subject based on RNA from a blood sample obtained from said subject, comprising:
- measuring the abundance of at least 250 RNAs in the sample, that are chosen from the RNAs listed in table 1, and
- concluding based on the measured abundance whether the subject has colorectal cancer.

2. The method of claim 1, wherein the abundance of at least 500 RNAs, of at least 800 RNAs, of at least 1000 RNAs that are chosen from the RNAs listed in table 1 is measured.

3. The method of claim 1, wherein the abundance of 250 RNAs as listed in table 2 is measured.

4. The method of claim 2, wherein the abundance of 500 RNAs as listed in table 3 is measured.

5. The method of claim 2, wherein the abundance of 800 RNAs as listed in table 4 is measured.

6. The method of claim 2, wherein the abundance of 1000 RNAs as listed in table 1 is measured.

7. The method of claims 1 to 6, wherein the measuring of RNA abundance is performed using a microarray, a real-time polymerase chain reaction or sequencing.

8. The method of claims 1 to 7, wherein the decision whether the subject has colorectal cancer comprises the step of training a classification algorithm on a training set of cases and controls, and applying it to measured RNA abundance.

9. The method of claims 1 to 8, wherein the classification method is a random forest method, a support vector machine (SVM), or a K-nearest neighbor method (K-NN), such as a 3-nearest neighbor method (3-NN).

10. The method of claims 1 to 9, wherein the RNA is mRNA, cDNA, micro RNA, small nuclear RNA, unspliced RNA, or its fragments.

11. Use of a method of claims 1 to 10 for detection of colorectal cancer in a human subject based on RNA from a blood sample.

12. A microarray, comprising a solid support and a set of oligonucleotide probes, the set containing from 250 to about 100,000 probes, and including at least 250 probes selected from table 1.

13. Use of a microarray for detection of colorectal cancer in a human subject based on RNA from a blood sample, comprising measuring the abundance of at least 250 RNAs listed in table 1, wherein the microarray comprises at least 1 probe for measuring the abundance of each of at least 250 RNAs.

14. A kit for the detection of colorectal cancer in a human subject based on RNA obtained from a blood sample, comprising means for measuring the abundance of at least 250 RNAs that are chosen from the RNAs listed in table 1, preferably comprising means for exclusively measuring the abundance of RNAs that are chosen from table 1.

15. Use of a kit of claim 14 for the detection of colorectal cancer in a human subject based on RNA from a blood sample, comprising means for measuring the abundance of at least 250 RNAs that are chosen from the RNAs listed in table 1, comprising
- measuring the abundance of at least 250 RNAs in a blood sample from a human subject, wherein the at least 250 RNAs are chosen from the RNAs listed in table 1, and
- concluding based on the measured abundance whether the subject has colorectal cancer.

16. A method for preparing an RNA expression profile that is indicative of the presence or absence of colorectal cancer in a subject, comprising:
- isolating RNA from a blood sample obtained from the subject, and
- determining the abundance of from 250 to about 1000 RNAs, including at least 250 RNAs selected from table 1.
